# EUROPEAN PATENT APPLICATION

(11) **EP 4 159 766 A1**
(43) Date of publication of application: **05.04.2023**
(21) Application number: 21812563.1
(22) Date of filing: 24.05.2021
(51) Int. Cl.: C07K 17/00, C12N 15/10, C12Q 1/68

(54) **CLEAVABLE DNA-ENCODED LIBRARY**

(30) Priority: 25.05.2020 JP 2020090304
(71) Applicant: Nissan Chemical Corporation, Tokyo 103-6119 (JP)
(72) Inventor: NIWA Masatoshi, Funabashi-shi, Chiba 274-8507 (JP); TOKUGAWA Munefumi, Funabashi-shi, Chiba 274-8507 (JP); HAYASHIDA Jun, Shiraoka-shi, Saitama 349-0294 (JP); FUKANO Hajime, Tokyo 134-8630 (JP)
(74) Representative: dompatent von Kreisler Selting Werner - Partnerschaft von Patent- und Rechtsanwälten mbB
(86) International application number: PCT/JP2021/019685
(87) International publication number: WO 2021/241528

(57) **Abstract**

The present invention relates to a utilizing method of a nucleic acid compound containing a selectively cleavable site. Also, the present invention relates to a DNA-encoded library containing the selectively cleavable site, a composition for synthesis therefor and a method of use thereof.

## Description

### TECHNICAL FIELD

The present invention relates to a DNA-encoded library containing a cleavable site in a DNA chain.

### BACKGROUND ART

A compound library is a group of compound derivatives in which compounds having a possibility to have a specific activity, such as a drug candidate compound, etc., are systematically collected. This compound library is synthesized in many cases based on the synthetic techniques and methodologies of combinatorial chemistry.

Combinatorial chemistry is an experimental method for efficiently conducting a wide variety synthesis of a series of compound libraries which are enumerated and designed based on combinatorics by a systematic synthetic route, and a research field related to it.

DNA-encoded library is one kind of compound library based on combinatorial chemistry. Hereinafter, the DNA-encoded library is appropriately abbreviated to as DEL. In DEL, a DNA tag is added to each compound in the library. The sequence of the DNA tag is designed so that each structure of each compound can be identified and functions as a label of the compound (Patent Documents 1 to 3).

DNA strand structure of the conventionally known DEL is representatively two strands, a double-strand and a hairpin strand.

Hereinafter, the outline of the double-stranded DEL and the hairpin strand DEL and the merit and the demerit thereof are described.

### (1) Hairpin-stranded DEL

A DEL using a hairpin-stranded DNA has a single-stranded structure in which two complementary DNA strands are linked and synthesized by using a hairpin type DNA having functional groups for introducing various building blocks as a raw material (head piece) (Patent Document 3 and Non-Patent Document 1 and 2).

### (A) Merits

### (a) Short DNA tags can be used.

In this method, in many cases, a relatively short double-stranded DNA tag of about 9 to 13-mer having a sticky terminal of 2-mer is used and the double-stranded DNA tag is introduced by a ligation reaction with DNA ligase. Use of such a short DNA tag becomes possible because the hairpin-stranded DNA strongly forms a duplex in the molecule and the DNA site other than the sticky terminal does not interfere with the DNA tag. Use of a short double-stranded DNA tag has some merits in DEL synthesis. One of the merits may be mentioned the cost of synthesizing the DNA tag is low. Also, as another merit, there may be mentioned that use of shorter DNA tag can suppress the overall length of the DEL in short length when the same number of reaction cycles are encoded. That is, even if a larger number of cycles is encoded, the overall length of the DEL can be suppressed to a range in which the DNA sequence can be efficiently read by the next-generation sequencer. In fact, in Non-Patent Document 3, by using a hairpin-stranded DNA, construction of the DEL using a hairpin-stranded DNA encoding the reaction of 6 cycles has been achieved.

### (b) Chemical stability is high

Different from the double strand, in the hairpin strand, even when a duplex structure is melted during the reaction under heating, the duplex in the original molecule is reformed without generating strand exchange under the subsequent reannealing conditions. Accordingly, DEL using a hairpin-stranded DNA has a merit that it can be used under a wider range of chemical conditions (Non-Patent Document 2). Also, in general, as for nucleic acid strands, if the chain length is the same, the hairpin strand forms a stronger duplex than the double strand (Tm value is high). Accordingly, under various chemical conditions at the time of introducing the building blocks, each chemical structure of the hairpin-stranded DNA, particularly the structure of the base portion, should resist the structural conversion as compared with the double strand.

### (B) Demerit

The hairpin-stranded DNA has a problem that it is difficult to melt the duplex and bind the primer oligonucleotide to initiate the polymerase reaction due to its strong duplex-forming ability, so that PCR efficiency is low (Patent Document 4).

### (2) Double-stranded DEL

A DEL using a double-stranded DNA is synthesized using a single-stranded DNA (single-stranded DNA that is not a hairpin strand) or a double-stranded DNA having a functional group(s) for introducing various building blocks as a raw material (head piece).

### (A) Demerit

Contrary to the DEL that uses the hairpin-stranded DNA, in many cases, relatively long single-stranded or double-stranded DNA tags of about 20 to 30-mer having 4 to 10-mer sticky terminal have been used (Patent Document 2, Non-Patent Document 4) and a DEL encoding a reaction of about 3 cycles is common.

### (B) Merit

DEL using a double-stranded DNA does not have the problem like the hairpin-stranded DNA from the viewpoint of the PCR efficiency. Further, different from the hairpin-stranded DNA, it is possible to convert the double-stranded DNA to a single-stranded DNA by denaturation, or to carry out a strand exchange reaction, so that it has the merit that it can adapt to a wider evaluation means by converting into a DNA structure suitable for various purposes. For example, an evaluation method having a high sensitivity ratio utilizing the double-strand forming ability of DNA has been developed (Non-Patent Documents 5 and 6).

Like this, although the hairpin-stranded DNA and the double-stranded DNA each have merits at the time of synthesis of DEL and evaluation, no technique that can achieve both merits has been known.

### PRIOR ART DOCUMENTS

### PATENT DOCUMENTS

Patent Document 1: WO 93/20243
Patent Document 2: WO 2004/039825
Patent Document 3: WO 2005/058479
Patent Document 4: WO 2010/094036

### NON-PATENT DOCUMENTS

Non-Patent Document 1: Nature Chemical Biology, 2009, vol. 5, pp. 647-654
Non-Patent Document 2: A Handbook for DNA -Encoded Chemistry, Edited by Robert A. Goodnow, Jr., John Wiley & Sons, Inc.
Non-Patent Document 3: ACS Chemical Biology, 2018, vol. 13, pp. 53-59
Non-Patent Document 4: Nature Chemistry, 2018, vol. 10, pp.441-448
Non-Patent Document 5: Annual Review of Biochemistry, 2018, vol. 87, pp. 479-502
Non-Patent Document 6: ACS Combinatorial Science, 2020, vol. 22, pp. 204-212

### SUMMARY OF THE INVENTION

### PROBLEMS TO BE SOLVED BY THE INVENTION

The present invention is to provide a DEL containing a cleavable site in a DNA strand and a method for producing the DEL.

### MEANS TO SOLVE THE PROBLEMS

As one of nucleic acid chemistry such as DNA, etc., there is cleavage technology of a nucleic acid. For example, when deoxyuridine is introduced into a DNA strand, it can be selectively cleaved by a USER (Registered trademark) enzyme.

The present inventor has found that, as a result of earnest studies, for example, both the merits of hairpin-stranded DNA and double-stranded DNA can be obtained by introducing a cleavable site such as deoxyuridine into the DNA strand, whereby completed the present invention.

Accordingly, the present invention is as follows.
[1] A compound represented by the formula (I) 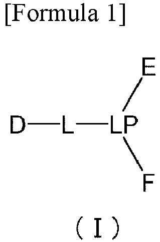 (wherein
   E and F are each independently
   an oligomer constituted by nucleotides or nucleic acid analogues,
   provided that E and F contain base sequences, which are complementary to each other and form a duplex oligonucleotide,
   LP is a loop site,
   L is a linker and
   D is a reactive functional group.),
   and has at least one selectively cleavable site at any of at least one site of E, F and LP
[2] A composition using for preparation of a head piece of a compound library wherein the composition comprises the compound described in [1].
[3] A composition using for preparation of a head piece of a DNA encoding library which comprises the compound described in [1].
[4] A compound used as a head piece of a compound library, which is represented by the formula (I) 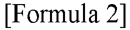 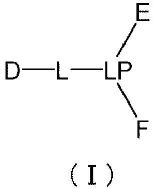 (wherein
   E and F are each independently
   an oligomer constituted by nucleotides or nucleic acid analogues,
   provided that E and F contain base sequences, which are complementary to each other and form a duplex oligonucleotide,
   LP is a loop site,
   L is a linker and
   D is a reactive functional group.) and
   has at least one selectively cleavable site at any of at least one site of E, F and LP.
[5] A compound used as a head piece of a DNA-encoded library, which is represented by the formula (I) 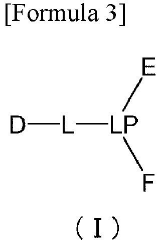 (wherein
   E and F are each independently
   an oligomer constituted by nucleotides or nucleic acid analogues,
   provided that E and F contain base sequences, which are complementary to each other and form a duplex oligonucleotide,
   LP is a loop site,
   L is a linker and
   D is a reactive functional group.) and
   has at least one selectively cleavable site at any of at least one site of E, F and LP.
[6] A head piece of a compound library, which is a compound represented by the formula (I) 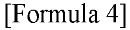 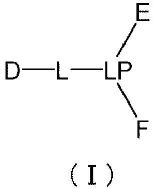 (wherein
   E and F are each independently
   an oligomer constituted by nucleotides or nucleic acid analogues,
   provided that E and F contain base sequences, which are complementary to each other and form a duplex oligonucleotide,
   LP is a loop site,
   L is a linker and
   D is a reactive functional group.) and
   has at least one selectively cleavable site at any of at least one site of E, F and LP.
[7] A head piece of a DNA-encoded library, which is a compound represented by the formula (I) 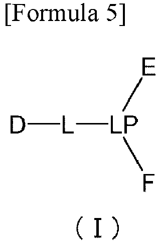 (wherein
   E and F are each independently
   an oligomer constituted by nucleotides or nucleic acid analogues,
   provided that E and F contain base sequences, which are complementary to each other and form a duplex oligonucleotide,
   LP is a loop site,
   L is a linker and
   D is a reactive functional group.) and
   has at least one selectively cleavable site at any of at least one site of E, F and LP.
[8] A compound represented by the formula (II) 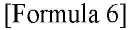 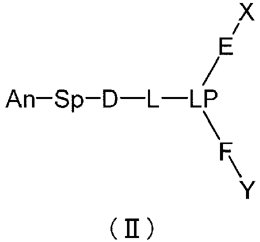 (wherein
   X and Y are oligonucleotide chains,
   E and F are each independently
   an oligomer constituted by nucleotides or nucleic acid analogues,
   provided that E and F contain base sequences, which are complementary to each other and form a duplex oligonucleotide,
   LP is a loop site,
   L is a linker and
   D is a divalent group derived from a reactive functional group,
   Sp is a bonding or a bifunctional spacer and
   An is a partial structure constituted by at least one building block.),
   X and Y have a sequence capable of forming a duplex at least a part thereof,
   X binds to E at the 5' terminal end,
   Y binds to F at the 3' terminal end and
   has at least one selectively cleavable site at any of at least one site of E, F and LP.
[9] The compound described in [8], which is represented by the formula (III)

   An-Sp-C-Bn (III)

   (wherein
   An and Sp represent the same meanings as defined in [8],
   Bn represents a double-stranded oligonucletide tag formed by an oligonucleotide chain X and an oligonucleotide chain Y,
   C is represented by the formula (I) 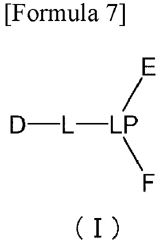
   (wherein E, LP, L, D and F represent the same meanings as defined in [8], provided that D binds to Sp and E and F each bind to corresponding terminal side of the double-stranded oligonucletide tag Bn.).
[10] The compound according to [8] or [9], wherein An is the same as defined in [8] and is a partial structure constructed by n building blocks α1 to αn (n is an integer of 1 to 10.) and
   Bn is a double-stranded oligonucletide tag formed by an oligonucleotide chain X and an oligonucleotide chain Y and is a partial structure containing an oligonucleotide which contains a base sequence capable of identifying the structure of An.
[11] The compound according to any of [1], [4], [5] and [8] to [10], wherein LP is a loop site represented by (LP1)p-LS-(LP2)q and
   LS is a partial structure selected from a compound group according to the following (A) to (C),
      (A) a nucleotide
      (B) a nucleic acid analogue
      (C) a C1 to 14 trivalent group which may have a substituent(s)
   LP1 is each a partial structure selected independently or differently with a number of p from a compound group according to the following (1) and (2),
      (1) a nucleotide
      (2) a nucleic acid analogue
   LP2 is each a partial structure selected independently or differently with a number of q from a compound group according to the following (1) and (2),
      (1) a nucleotide
      (2) a nucleic acid analogue
   and a total number of p and q is 0 to 40.
[12] The compound according to [11], wherein the total number of p and q is 2 to 20.
[13] The compound according to [11], wherein the total number of p and q is 2 to 10.
[14] The compound according to [11], wherein the total number of p and q is 2 to 7.
[15] The compound according to [11], wherein the total number of p and q is 0.
[16] The compound according to any of [11] to [15], wherein LP1, LP2 and LS are each a structure independently or differently selected from the following structures:
   (A) a nucleotide
      or
   (B) a nucleic acid analogue which requires the following (B11) to (B15)
      (B11) it has phosphoric acid (or a corresponding site) and a hydroxyl group (or its corresponding site),
      (B12) it is constituted by carbon, hydrogen, oxygen, nitrogen, phosphorus or sulfur,
      (B13) a molecular weight is from 142 to 1,500,
      (B14) a number of atoms between residues is 3 to 30 and
      (B15) a bonding mode of the atoms between the residues is either all single bonds or containing one to two double bonds and the remaining are single bonds.
[17] The compound according to any of [11] to [16], wherein LP1, LP2 and LS are each a structure independently or differently selected from the following structures:
   (A) a nucleotide
      or
   (B) a nucleic acid analogue which requires the following (B21) to (B25)
      (B21) it has phosphoric acid and a hydroxyl group,
      (B22) it is constituted by carbon, hydrogen, oxygen, nitrogen or phosphorus,
      (B23) a molecular weight is from 142 to 1,000,
      (B24) a number of atoms between residues is 3 to 15 and
      (B25) a bonding mode of the atoms between the residues is all single bonds.
[18] The compound according to any of [11] to [17], wherein LP1, LP2 and LS are each a structure independently or differently selected from the following structures:
   (A) a nucleotide
      or
   (B) a nucleic acid analogue which requires the following (B31) to (B35)
      (B31) it has phosphoric acid and a hydroxyl group,
      (B32) it is constituted by carbon, hydrogen, oxygen, nitrogen or phosphorus,
      (B33) a molecular weight is from 142 to 700,
      (B34) a number of atoms between residues is 4 to 7 and
      (B35) a bonding mode of the atoms between the residues is all single bonds.
[19] The compound according to any of [11] to [18], wherein LP1 and LP2 are each any of the following:
   (B41) a d-Spacer and
   (B5) a polyalkylene glycol phosphoric acid ester.
[20] The compound according to any of [11] to [19], wherein LP1 and LP2 are each diethylene glycol phosphoric acid ester or triethylene glycol phosphoric acid ester.
[21] The compound according to any of [11] to [20], wherein LP1 and LP2 are each triethylene glycol phosphoric acid ester.
[22] The compound according to any of [11] to [19], wherein LP1 and LP2 are each d-Spacer.
[23] The compound according to any of [11] to [18], wherein
   LP1 and LP2 are each nucleotide.
[24] The compound according to any of [11] to [23], wherein LS is any of the formula (a) to the formula (g): 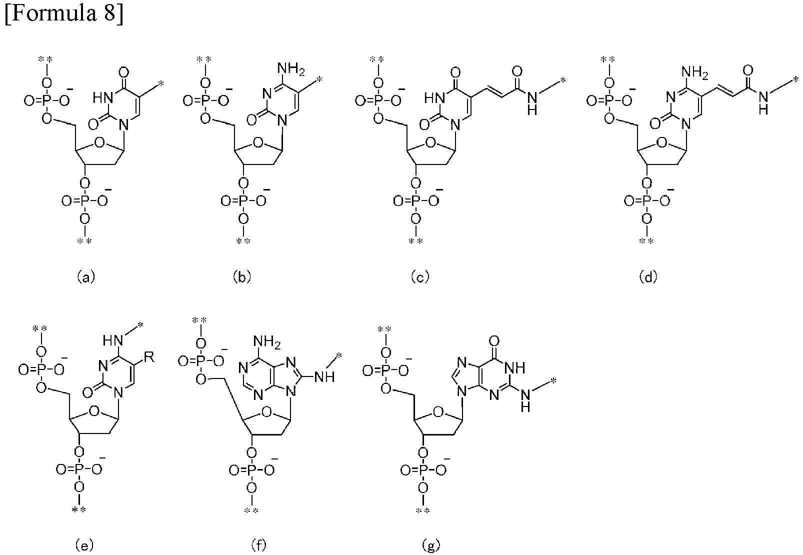 (wherein * means a binding site with the linker, ** means a binding site with LP1 or LP2 and R is a hydrogen atom or a methyl group.).
[25] The compound according to any of [11] to [23], wherein LS is the formula (h): 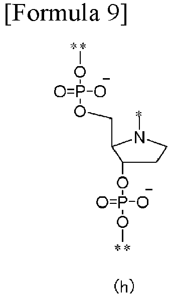 (wherein * means a binding site with the linker and ** means a binding site with LP1 or LP2).
[26] The compound according to any of [11] to [23], wherein LS is a polyalkylene glycol phosphoric acid ester.
[27] The compound according to any of [11] to [23], wherein LS is any of the formula (i) to the formula (k): 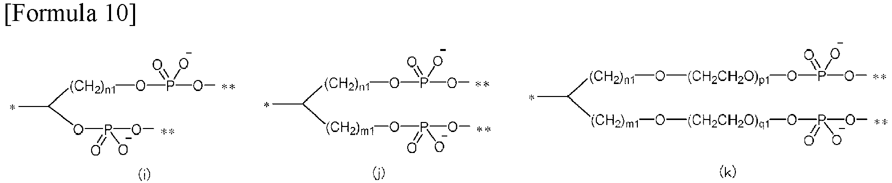 (wherein n1, m1, p1 and q1 are each independently an integer of 1 to 20, * means a binding site with the linker and ** means a binding site with LP1 or LP2).
[28] The compound according to any of [11] to [23], wherein LS is the formula (l): 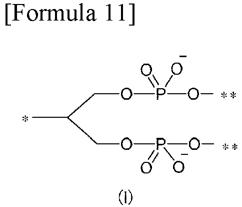 (wherein * means a binding site with the linker and ** means a binding site with LP1 or LP2).
[29] The compound according to any of [11] to [23], wherein LS is any of (B42), (B43) or (B44):
   (B42) Amino C6 dT
   (B43) mdC(TEG-Amino)
   (B44) Uni-Link (trademark registration) Amino Modifier.
[30] The compound according to any of [11] to [23], wherein LS is a nucleotide.
[31] The compound according to any of [11] to [15] and [19] to [23], wherein LS is (C) a C1 to 14 trivalent group which may have a substituent(s) and (C) is any of the following structures:
   (1) a C1 to 10 aliphatic hydrocarbon which may have a substituent(s) and may be replaced with 1 to 3 hetero atoms,
   (2) a C6 to 14 aromatic hydrocarbon which may have a substituent(s),
   (3) a C2 to 9 aromatic heterocyclic ring which may have a substituent(s), or
   (4) a C2 to 9 non-aromatic heterocyclic ring which may have a substituent(s).
[32] The compound according to any of [11] to [15] and [19] to [23], wherein LS is (C) a C1 to 14 trivalent group which may have a substituent(s) and (C) is any of the following structures:
   (1) a C1 to 6 aliphatic hydrocarbon which may have a substituent(s),
   (2) a C6 to 10 aromatic hydrocarbon which may have a substituent(s), or
   (3) a C2 to 5 aromatic heterocyclic ring which may have a substituent(s).
[33] The compound according to any of [11] to [15] and [19] to [23], wherein LS is (C) a C1 to 14 trivalent group which may have a substituent(s) and (C) is any of the following structures:
   (1) a C1 to 6 aliphatic hydrocarbon,
   (2) benzene, or
   (3) a C2 to 5 nitrogen-containing aromatic heterocyclic ring
   here, the above (1) to (3) are unsubstituted, or may be substituted by 1 to 3 substituents independently or differently selected from a substituent group ST1, the substituent group ST1 is a group constituted by a C1 to 6 alkyl group, a C1 to 6 alkoxy group, a fluorine atom and a chlorine atom, provided that when the substituent group ST1 is substituted with the aliphatic hydrocarbon, an alkyl group is not selected from the substituent group ST1.
[34] The compound according to any of [11] to [15] and [19] to [23], wherein LS is (C) a C1 to 14 trivalent group which may have a substituent(s) and (C) is any of the following structures:
   (1) a C1 to 6 alkyl group, or
   (2) benzene which is unsubstituted or substituted by one or two C1 to 3 alkyl group(s) or C1 to 3 alkoxy group(s).
[35] The compound according to any of [11] to [15] and [19] to [23], wherein LS is (C) a C1 to 14 trivalent group which may have a substituent(s) and (C) is the following structure:
   (1) a C1 to 6 alkyl group.
[36] The compound according to any of [1], [4], [5] and [8] to [35], wherein E and F are each independently an oligomer constituted by nucleotides or nucleic acid analogues and
   a chain length of E and F is each 3 to 40.
[37] The compound according to any of [1], [4], [5] and [8] to [36], wherein E and F are each independently an oligomer constituted by nucleotides or nucleic acid analogues and
   a chain length of E and F is each 4 to 30.
[38] The compound according to any of [1], [4], [5] and [8] to [37], wherein E and F are each independently an oligomer constituted by nucleotides or nucleic acid analogues and
   a chain length of E and F is each 6 to 25.
[39] The compound according to any of [1], [4], [5] and [8] to [38], wherein E and F are each independently an oligomer constituted by nucleotides or nucleic acid analogues,
   E and F contain base sequences, which are complementary to each other and form a duplex oligonucleotide, and
   the duplex oligonucleotide of E and F is a sticky end.
[40] The compound according to [39], wherein a protruded portion of the sticky end has a length of 2 bases or more.
[41] The compound according to any of [1], [4], [5] and [8] to [38], wherein E and F are each independently an oligomer constituted by nucleotides or nucleic acid analogues,
   E and F contain base sequences, which are complementary to each other and form a duplex oligonucleotide, and
   the duplex oligonucleotide of E and F is a blunt end.
[42] The compound according to any of [1], [4], [5] and [8] to [41], wherein chain lengths of the base sequences, which are complementary to each other contained in E and F are each 3 bases or more.
[43] The compound according to any of [1], [4], [5] and [8] to [42], wherein chain lengths of the base sequences, which are complementary to each other contained in E and F are each 4 bases or more.
[44] The compound according to any of [1], [4], [5] and [8] to [43], wherein chain lengths of the base sequences, which are complementary to each other contained in E and F are each 6 bases or more.
[45] The compound according to any of [1], [4], [5] and [8] to [44], wherein E and F are each independently an oligomer constituted by a nucleotide.
[46] The compound according to any of [1], [4], [5] and [8] to [45], wherein the nucleotide is a ribonucleotide or a deoxyribonucleotide.
[47] The compound according to any of [1], [4], [5] and [8] to [46], wherein the nucleotide is a deoxyribonucleotide.
[48] The compound according to any of [1], [4], [5] and [8] to [47], wherein the nucleotide is deoxyadenosine, deoxyguanosine, thymidine, or deoxycytidine.
[49] The compound according to any of [1], [4], [5] and [8] to [44], wherein E and F are each independently an oligomer constituted by nucleic acid analogues.
[50] The compound according to any of [1], [4], [5] and [8] to [49], wherein L is
   (1) a C1 to 20 aliphatic hydrocarbon which may have a substituent(s) and may be replaced with 1 to 3 hetero atoms,
      or
   (2) a C6 to 14 aromatic hydrocarbon which may have a substituent(s).
[51] The compound according to any of [1], [4], [5] and [8] to [50], wherein L is a C1 to 6 aliphatic hydrocarbon which may have a substituent(s), a C1 to 6 aliphatic hydrocarbon which may be replaced with one or two oxygen atoms, or a C6 to 10 aromatic hydrocarbon which may have a substituent(s).
[52] The compound according to any of [1], [4], [5] and [8] to [51], wherein L is a C1 to 6 aliphatic hydrocarbon substitutable with the substituent group ST1 or benzene substitutable with the substituent group ST1, here, the substituent group ST1 is a group constituted by a C1 to 6 alkyl group, a C1 to 6 alkoxy group, a fluorine atom and a chlorine atom (provided that when the substituent group ST1 is substituted with the aliphatic hydrocarbon, an alkyl group is not selected from the substituent group ST1.).
[53] The compound according to any of [1], [4], [5] and [8] to [52], wherein L is a C1 to 6 alkyl group, or a benzene which is unsubstituted or substituted by one or two C1 to 3 alkyl group(s) or C1 to 3 alkoxy group(s).
[54] The compound according to any of [1], [4], [5] and [8] to [53], wherein L is a C1 to 6 alkyl group.
[55] The compound according to any of [1], [4], [5] and [8] to [54], wherein the reactive functional group of D is a reactive functional group which can constitute a C-C, amino, ether, carbonyl, amide, ester, urea, sulfide, disulfide, sulfoxide, sulfonamide or sulfonyl bond.
[56] The compound according to any of [1], [4], [5] and [8] to [55], wherein the reactive functional group of D is a C1 hydrocarbon having a leaving group, an amino group, a hydroxyl group, a precursor of a carbonyl group, a thiol group or an aldehyde group.
[57] The compound according to any of [1], [4], [5] and [8] to [56], wherein the reactive functional group of D is a C1 hydrocarbon having a halogen atom(s), a C1 hydrocarbon having a sulfonic acid-based leaving group, an amino group, a hydroxyl group, a carboxyl group, a halogenated carboxyl group, a thiol group or an aldehyde group.
[58] The compound according to any of [1], [4], [5] and [8] to [57], wherein the reactive functional group of D is -CH₂Cl, -CH₂Br, -CH₂OSO₂CH₃, -CH₂OSO₂CF₃, an amino group, a hydroxyl group or a carboxy group.
[59] The compound according to any of [1], [4], [5] and [8] to [58], wherein the reactive functional group of D is a primary amino group.
[60] The compound according to any of [1], [4], [5] and [8] to [59], wherein the selectively cleavable site is deoxyribonucleoside which is neither of deoxyadenosine, deoxyguanosine, thymidine nor deoxycytidine.
[61] The compound according to any of [1], [4], [5] and [8] to [60], wherein the selectively cleavable site is deoxyuridine, bromodeoxyuridine, deoxyinosine, 8-hydroxydeoxyguanosine, 3-methyl-2'-deoxyadenosine, N6-etheno-2'-deoxyadenosine, 7-methyl-2'-deoxyguanosine, 2'-deoxyxanthosine or 5,6-dihydroxy-5,6 dihydrodeoxy-thymidine.
[62] The compound according to any of [1], [4], [5] and [8] to [61], wherein the selectively cleavable site is deoxyuridine or deoxyinosine.
[63] The compound according to any of [1], [4], [5] and [8] to [62], wherein the selectively cleavable site is deoxyuridine
[64] The compound according to any of [1], [4], [5] and [8] to [62], wherein the selectively cleavable site is deoxyinosine.
[65] The compound according to any of [1], [4], [5] and [8] to [59], wherein the selectively cleavable site is a phosphodiester bond at the second in a 3' direction from deoxyinosine.
[66] The compound according to any of [1], [4], [5] and [8] to [59], wherein the selectively cleavable site is ribonucleoside.
[67] The compound according to any of [1], [4], [5] and [8] to [66], wherein the selectively cleavable site is one.
[68] The compound according to any of [1], [4], [5] and [8] to [66], wherein at least one cleavable site is contained in E or (LP1)p and at least one cleavable site is contained in F or (LP2)q.
[69] The compound according to [68], wherein the cleavable site contained in E or (LP1)p and the cleavable site contained in F or (LP2)q can be cleaved under different conditions.
[70] The compound according to any of [8] to [69], wherein An is a partial structure constructed by n building blocks α1 to αn (n is an integer of 1 to 10.).
[71] The compound according to any of [8] to [70], wherein An is a low molecular weight organic compound.
[72] The compound according to any of [8] to [71], wherein the building block of An is a compound having a molecular weight of 500 or less.
[73] The compound according to any of [8] to [72], wherein the building block of An is a compound having a molecular weight of 300 or less.
[74] The compound according to any of [8] to [73], wherein the building block of An is a compound having a molecular weight of 150 or less.
[75] The compound according to any of [8] to [74], wherein An is an organic compound constituted by an element selected alone or differently from the element group consisting of H, B, C, N, O, Si, P, S, F, Cl, Br and I.
[76] The compound according to any of [8] to [75], wherein An is a low molecular weight organic compound having a substituent selected alone or differently from a substituent group consisting of an aryl group, a non-aromatic cyclyl group, a heteroaryl group and a non-aromatic heterocyclyl group.
[77] The compound according to any of [8] to [76], wherein An has a molecular weight of 5,000 or less.
[78] The compound according to any of [8] to [77], wherein An has a molecular weight of 800 or less.
[79] The compound according to any of [8] to [78], wherein An has a molecular weight of 500 or less.
[80] The compound according to any of [8] to [70], wherein An is a polypeptide.
[81] The compound according to any of [8] to [80], wherein Sp is a bond.
[82] The compound according to any of [8] to [80], wherein Sp is a bifunctional spacer,
   the bifunctional spacer is SpD-SpL-SpX,
   SpD is a divalent group derived from a reactive group capable of constituting a C-C, amino, ether, carbonyl, amide, ester, urea, sulfide, disulfide, sulfoxide, sulfonamide or sulfonyl bond,
   SpL is polyalkylene glycol, polyethylene, a C1 to 20 aliphatic hydrocarbon which may be optionally replaced with a hetero atom(s), a peptide, an oligonucleotide or a combination thereof and
   SpX is a divalent group derived from a reactive group which forms an amide, amino or sulfonamide bond.
[83] The compound according to any of [8] to [81], wherein Sp is a bifunctional spacer,
   the bifunctional spacer is SpD-SpL-SpX,
   SpD is a divalent group derived from a primary amino group,
   SpL is polyethylene glycol or polyethylene and
   SpX is a divalent group derived from a carboxy group.
[84] The compound according to any of [8] to [83], wherein the oligonucleotide chain X and the oligonucleotide chain Y are sequences capable of forming a duplex.
[85] The compound according to any of [8] to [84], wherein the oligonucleotide chain X and the oligonucleotide chain Y contain a complementary base sequence.
[86] The compound according to any of [8] to [85], wherein the oligonucleotide chain X and the oligonucleotide chain Y are each having a length of 1 to 200 bases.
[87] The compound according to any of [8] to [86], wherein the oligonucleotide chain X and the oligonucleotide chain Y are each having a length of 3 to 150 bases.
[88] The compound according to any of [8] to [87], wherein the oligonucleotide chain X and the oligonucleotide chain Y are each having a length of 30 to 150 bases.
[89] The compound according to any of [8] to [88], wherein the oligonucleotide chain X and the oligonucleotide chain Y have a blunt end.
[90] The compound according to any of [8] to [88], wherein the oligonucleotide chain X and the oligonucleotide chain Y have a sticky end.
[91] The compound according to [90], wherein a protruded portion of the sticky end has a length of 1 to 30 bases.
[92] The compound according to [90] or [91], wherein a protruded portion of the sticky end has a length of 2 to 5 bases.
[93] The compound according to any of [90] to [92], wherein the oligonucleotide chain X and the oligonucleotide chain Y have a sticky end and a specific molecular recognition sequence is further bonded to the sticky end.
[94] The compound according to any of [8] to [93], wherein a functional molecule is bound to any one of X and Y.
[95] The compound according to any of [8] to [93], wherein biotin is bound to any one of X and Y.
[96] A compound library which contains the compound(s) according to any of [1], [4], [5] and [8] to [95].
[97] A DNA-encoded library which contains the compound(s) according to any of [1], [4], [5] and [8] to [95].
[98] The library according to [96] or [97], which is constituted by 1,000 or more different compounds.
[99] A method which is a method for producing a compound An-Sp-C-Bn, An is a partial structure constructed by n building blocks α1 to αn (n is an integer of 2 to 10.),
   Sp is a bond or a bifunctional spacer,
   C is a hairpin type head piece having at least one "selectively cleavable site" and
   Bn is a partial structure containing an oligonucleotide which contains a base sequence capable of identifying the structure of An,
   which comprises subjecting to C the following steps of;
      (a) binding α1-Sp, or binding Sp and α1 and
      (b) binding an oligonucletide tag which contains a base sequence capable of identifying a structure of α1,
         to obtain a compound A1-Sp-C-B1 and
         then, subjecting to A(m-1)-Sp-C-B(m-1) (m is an integer of 2 to n) the following steps (c) and (d) by repeating until m from 2 to n in ascending order;
      (c) binding αn to the A portion and
      (d) binding an oligonucletide tag which contains a base sequence capable of identifying a structure of αn to the B portion
   to obtain a compound Am-Sp-C-Bm,
   where the steps (a) and (b) and the steps (c) and (d) can be carried out in an optional order.
[100] A method which is a method for producing An-Sp-C-Bn which is the compound according to any of [9] to [95],
   An is a partial structure constructed by n building blocks α1 to αn (n is an integer of 2 to 10.),
   Sp is a bonding or a bifunctional spacer and
   C is a hairpin type head piece having at least one "selectively cleavable site" and
   Bn is a partial structure containing an oligonucleotide which contains a base sequence capable of identifying the structure of An,
   which comprises subjecting to C the following steps of;
      (a) binding α1-Sp, or binding Sp and α1 and
      (b) binding an oligonucletide tag which contains a base sequence capable of identifying a structure of α1,
         to obtain a compound A1-Sp-C-B1,
         then, subjecting to A(m-1)-Sp-C-B(m-1) (m is an integer of 2 to n) the following steps (c) and (d) by repeating until m from 2 to n in ascending order;
      (c) binding αn to the A portion and
      (d) binding an oligonucletide tag which contains a base sequence capable of identifying a structure of αn to the B portion
   to obtain a compound Am-Sp-C-Bm,
   where the steps (a) and (b) and the steps (c) and (d) can be carried out in an optional order.
[101] A method which is a method for producing An-Sp-C-Bn (An, Sp, C and Bn represent the same meanings as defined above) which is the compound according to any of [9] to [95],
   which comprises subjecting to C the following steps of;
      (a) binding α1-Sp, or binding Sp and α1 and
      (b) binding an oligonucletide tag which contains a base sequence capable of identifying a structure of α1,
         to obtain a compound A1-Sp-C-B1,
         then, subjecting to A(m-1)-Sp-C-B(m-1) (m is an integer of 2 to n) the following steps (c) and (d) by repeating until m from 2 to n in ascending order;
      (c) binding αn to the A portion and
      (d) binding an oligonucletide tag which contains a base sequence capable of identifying a structure of αn to the B portion
   to obtain a compound Am-Sp-C-Bm,
   where the steps (a) and (b) and the steps (c) and (d) can be carried out in an optional order.
[102] A method which is a method for evaluating a compound library containing at least one compound represented by the formula (III)

   An-Sp-C-Bn (III)

   (wherein
   An is a partial structure constructed by n building blocks α1 to αn (n is an integer of 1 to 10.),
   Sp is a bonding or a bifunctional spacer and
   C is a hairpin type head piece having at least one "selectively cleavable site" and
   Bn is a partial structure containing an oligonucleotide which contains a base sequence capable of identifying the structure of An.),
   which is constituted by the following steps of:
      (1) contacting the compound library with a biological target under conditions suitable for binding at least one library molecule of the compound library to the target,
      (2) removing the library molecule that does not bind to the target and selecting a library molecule that have affinity to the biological target,
      (3) cleaving cleavable sites selectively,
      (4) identifying sequences of oligonucleotides constituting Bn and
      (5) using the sequences determined in (4) to identify the structure of one or more compounds that bind to the biological target.
[103] A method which is a method for evaluating a compound library containing at least one compound according to any of [8] to [92] and represented by the formula (III)

   An-Sp-C-Bn (III)

   (wherein
   An is a partial structure constructed by n building blocks α1 to αn (n is an integer of 1 to 10.),
   Sp is a bonding or a bifunctional spacer and
   C is a hairpin type head piece having at least one "selectively cleavable site" and
   Bn is a partial structure containing an oligonucleotide which contains a base sequence capable of identifying the structure of An.),
   which is constituted by the following steps:
      (1) contacting the compound library with a biological target under conditions suitable for binding at least one library molecule of the compound library to the target,
      (2) removing the library molecule that does not bind to the target and selecting a library molecule that have affinity to the biological target,
      (3) cleaving cleavable sites selectively,
      (4) identifying sequences of oligonucleotides constituting Bn and
      (5) using the sequences determined in (4) to identify the structure of one or more compounds that bind to the biological target.
[104] The method according to [102] or [103], which includes a step of amplifying an oligonucleotide constituting Bn between the steps (3) and (4).
[105] The method according to any of [102] to [104], wherein the step of selectively cutting cleavable site is a step of selectively cutting cleavable site with enzyme.
[106] The method according to any of [102] to [104], wherein the step of selectively cutting cleavable site is a step of selectively cutting cleavable site by a combination of an enzyme and change in chemical conditions.
[107] The method according to [105] or [106], wherein the enzyme is at least one selected from glycosylase and nuclease.
[108] The method according to [107], wherein the enzyme is uracil DNA glycosylase.
[109] The method according to [107], wherein the enzyme is endonuclease VIII.
[110] The method according to [107], wherein the enzyme is a combination of uracil DNA glycosylase and endonuclease VIII.
[111] The method according to [107], wherein the enzyme is alkyl adenine DNA glycosylase.
[112] The method according to [107], wherein the enzyme is endonuclease V.
[113] The method according to any of [106] to [112], wherein change in chemical conditions is heating at 50 to 100°C in a solution containing water.
[114] The method according to any of [106] to [113], wherein change in chemical conditions is heating at 80 to 95°C in a solution containing water.
[115] The method according to any of [106] to [114], wherein change in chemical conditions is a basic condition of pH 8 to 13.
[116] The method according to any of [106] to [115], wherein change in chemical conditions is a basic condition of pH 8 to 11.
[117] The method according to any of [106] to [116], wherein change in chemical conditions is a basic condition of pH 9 to 10.
[118] The method according to any of [102] to [117], wherein a cleavable site is provided near the terminal of the DNA tag, if necessary, the site is cleaved to form a new sticky end and a specific molecule identification sequence is ligated to the sticky terminal to identify sequences of oligonucleotides constituting Bn.
[119] The method according to [118], wherein the cleavable site provided near the terminal of the DNA tag and the cleavable site contained in C are cleaved under different conditions.
[120] A method of utilizing as a double-stranded nucleic acid which comprises using a nucleic acid that binds to a compound having a cleavable site and a hairpin structure and cleaving a cleavable site.
[121] The method according to [120], wherein a nucleic acid that is chemically stable than a double-stranded nucleic acid and binds to a compound having a cleavable site and a hairpin structure is used and utilized as a double-stranded nucleic acid by cleaving the cleavable site.
[122] The method according to [120] or [121], wherein a nucleic acid that binds to a compound having a cleavable site and a hairpin structure is used and after subjecting to chemical structure conversion to the compound, it is utilized as a double-stranded nucleic acid by cleaving the cleavable site.
[123] The method according to any of [120] to [122], wherein a nucleic acid that binds to a compound having a cleavable site and a hairpin structure is used and after further subjecting to chemical structure conversion to the nucleic acid, it is utilized as a double-stranded nucleic acid by cleaving the cleavable site.
[124] The method according to any of [120] to [123], wherein a nucleic acid that binds to a compound having a cleavable site and a hairpin structure is used and after further subjecting to nucleic acid elongation reaction to the nucleic acid, it is utilized as a double-stranded nucleic acid by cleaving the cleavable site.
[125] The method according to any of [120] to [124], which is made capable of utilizing as a double-stranded nucleic acid by cleaving the cleavable site using a nucleic acid that binds to a compound having a cleavable site and a hairpin structure, to carry out a PCR reaction.
[126] The method according to any of [120] to [125], which is used for evaluation of functionality of a compound.
[127] The method according to any of [120] to [126], which is used for evaluation of biological activity of a compound.
[128] The method according to any of [120] to [127], which is used for DEL.
[129] The method described in any of [120] to [124], which is used for production of DEL.
[130] A method for converting into DEL having a single-stranded DNA which comprises cleaving a cleavable site to a DEL compound synthesized by using a nucleic acid that binds to a compound having a cleavable site and a hairpin structure.
[131] A method for forming a double strand with a cross linker-modified DNA which comprises cleaving a cleavable site of a DEL compound synthesized by using a nucleic acid that binds to a compound having a cleavable site and a hairpin structure to convert it into DEL having a single-stranded DNA.
[132] A method for synthesizing a cross linker-modified double-stranded DEL compound which comprises cleaving a cleavable site of a DEL compound synthesized by using a nucleic acid that binds to a compound having a cleavable site and a hairpin structure, adding a cross linker-modified primer and elongating the added primer.

### EFFECTS OF THE INVENTION

In the present invention, a DEL containing a cleavable site in a DNA strand and a composition for synthesis thereof are provided and it is possible to produce a DEL that is more convenient than the conventional one.

### BRIEF DESCRIPTION OF THE DRAWINGS

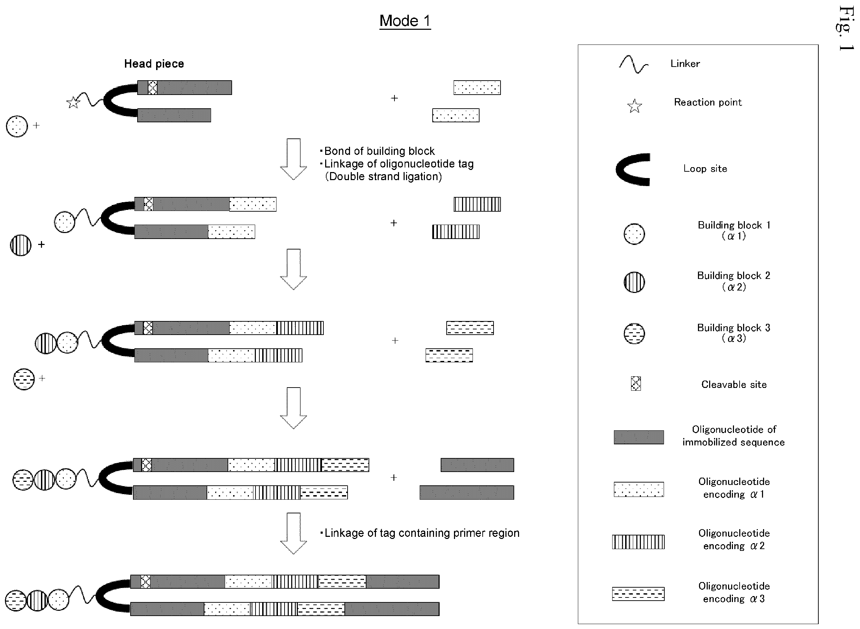

Fig. 1 shows an exemplary method for producing a DEL of Form 1. Using a head piece which contains a first oligonucleotide chain containing a cleavable site in a DNA strand, a loop site and a second oligonucleotide chain as a raw material, binding of building blocks and a double-stranded ligation of the oligonucletide tag corresponding to the building blocks are repeated (three times in Fig. 1) and further, if desired, double-stranded ligation of the oligonucletide tag containing the primer region is carried out to accomplish production of a DEL.

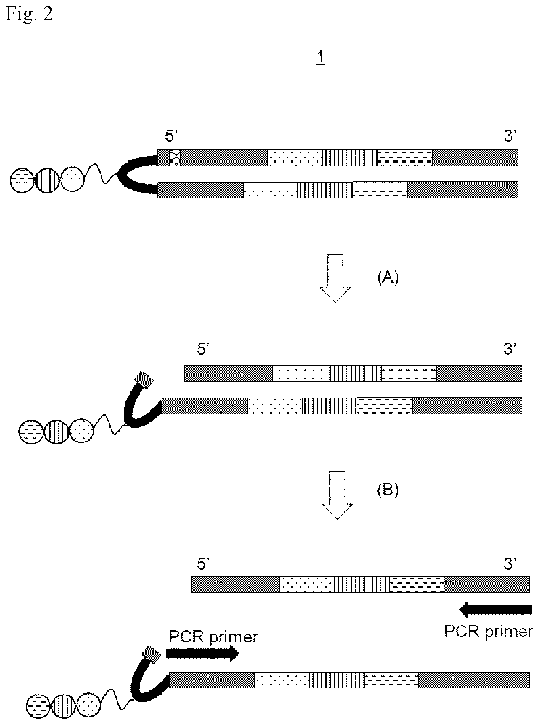

Fig. 2 shows an exemplary method for using a DEL of Form 1. To a DEL containing a cleavable site in a first oligonucleotide chain of a head piece, by cleaving the cleavable site using a cleaving means such as an enzyme, etc. and inducing it to a double-stranded oligonucleotide which is not bound by the loop site, PCR can be carried out with high efficiency.

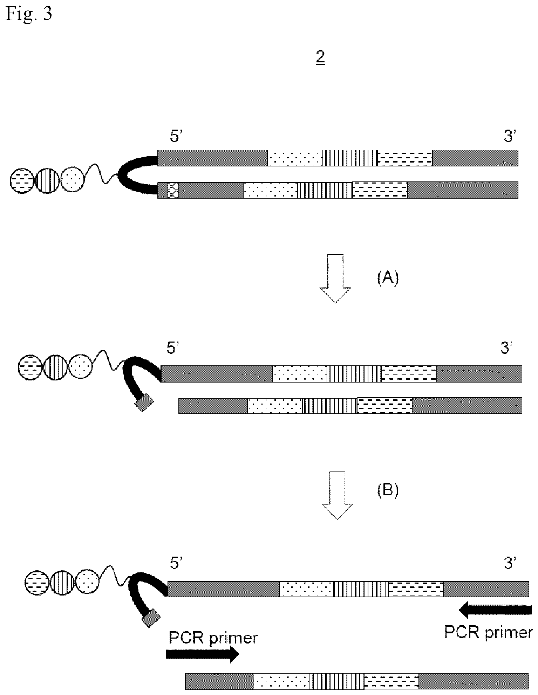

Fig. 3 shows an exemplary method for using a DEL of Form 2. To a DEL containing a cleavable site in a second oligonucleotide chain of a head piece, by cleaving the cleavable site using a cleaving means such as an enzyme, etc. and inducing it to a double-stranded oligonucleotide which is not bound by the loop site, PCR can be carried out with high efficiency.

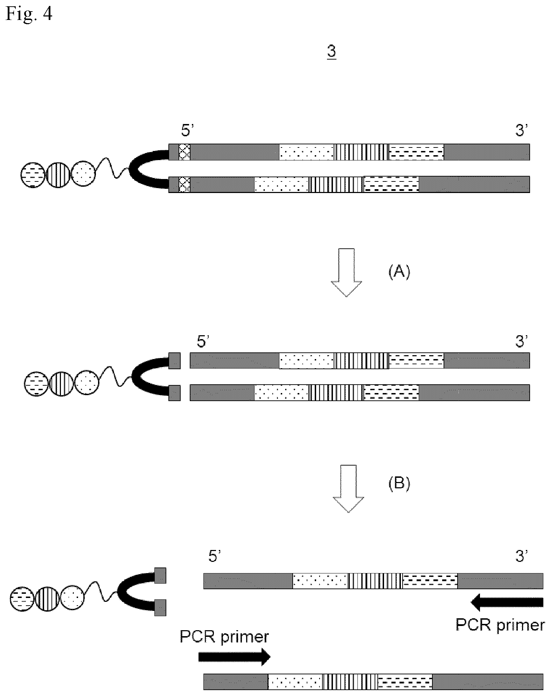

Fig. 4 shows an exemplary method for using a DEL of Form 3. To a DEL containing cleavable sites in a first oligonucleotide chain and a second oligonucleotide chain of a head piece, by cleaving both of the cleavable sites using a cleaving means such as an enzyme, etc. and inducing it to a double-stranded oligonucleotide which is not bound by the loop site, PCR can be carried out with high efficiency.

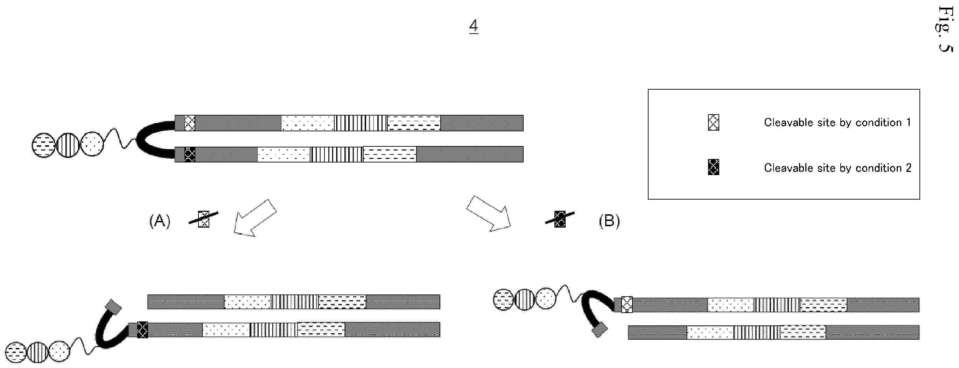

Fig. 5 shows an exemplary method for using a DEL of Form 4. To a DEL containing two kinds of cleavable sites different from each other in a first oligonucleotide chain and a second oligonucleotide chain of a head piece, by selecting the cleavage conditions, one of the first oligonucleotide chain or the second oligonucleotide chain can be selectively cleaved.

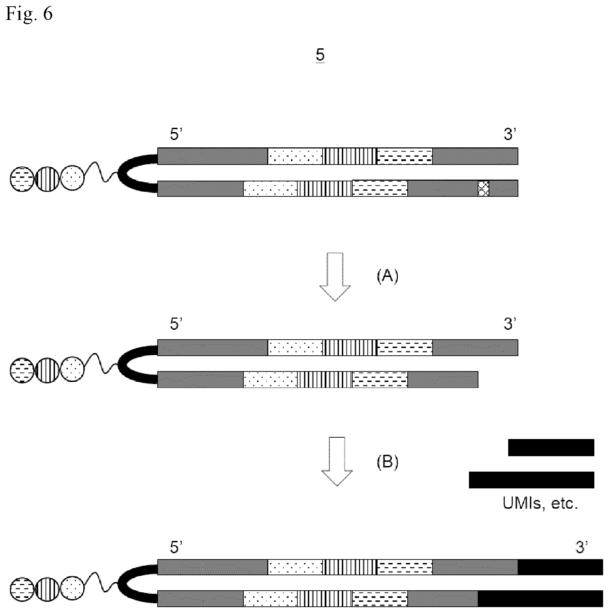

Fig. 6 shows an exemplary method for using a DEL of Form 5. By providing a cleavable site near the terminal of a DNA tag and, if desired, by cleaving the site, a new sticky end can be formed. The sticky end can be utilized as a sticky terminal, a desired nucleic acid sequence, for example, UMIs (a specific molecule identification sequence), etc., can be ligated and a new function can be imparted.

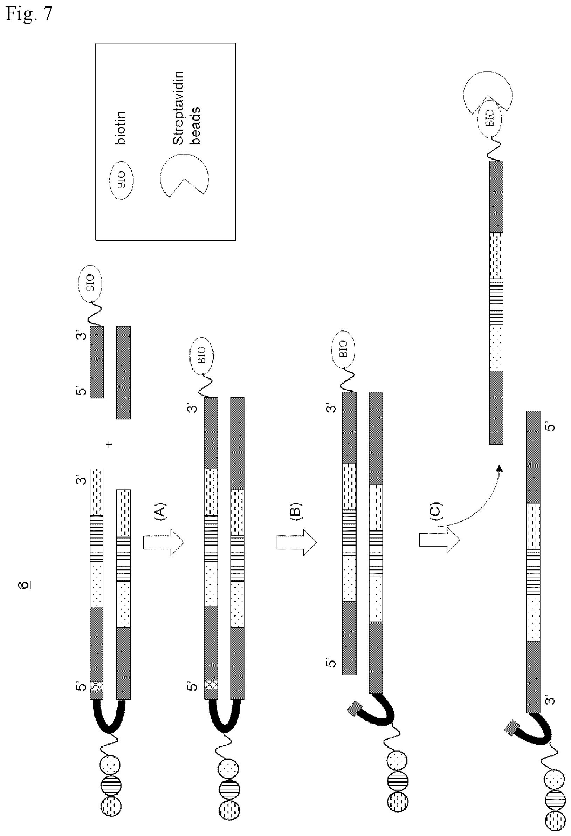

Fig. 7 shows an exemplary method for using a DEL of Form 6. In the present invention, a cleavable site can be used in combination with a modifying group or a functional molecule and for example, it is possible to prepare a DEL in which a hairpin-stranded DNA is converted into a single-stranded DNA. For example, to the synthesized DEL compound, a double-stranded oligonucleotide chain having a functional molecule (for example, biotin) at the 3' terminal is ligated (A), a cleavable site is cleaved (B) and a treatment depending on a function of a functional molecule is applied (C). For example, when the functional molecule is biotin, the oligonucleotide chain to which biotin is bound is selectively removed from the system by using streptavidin beads having biotin affinity. According to it, it is possible to obtain a DEL having a single-stranded DNA.

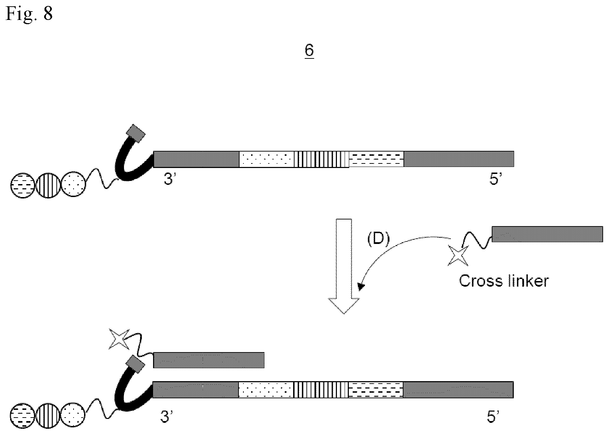

Fig. 8 shows an exemplary method for using a DEL obtained in Form 6. To the DEL having the single-stranded DNA obtained in Form 6, by forming a double strand with a modified oligonucleotide (for example, a cross linker-modified DNA such as a photoreactive cross linker, etc.) having a desired functional site, it is possible to impart a new function.

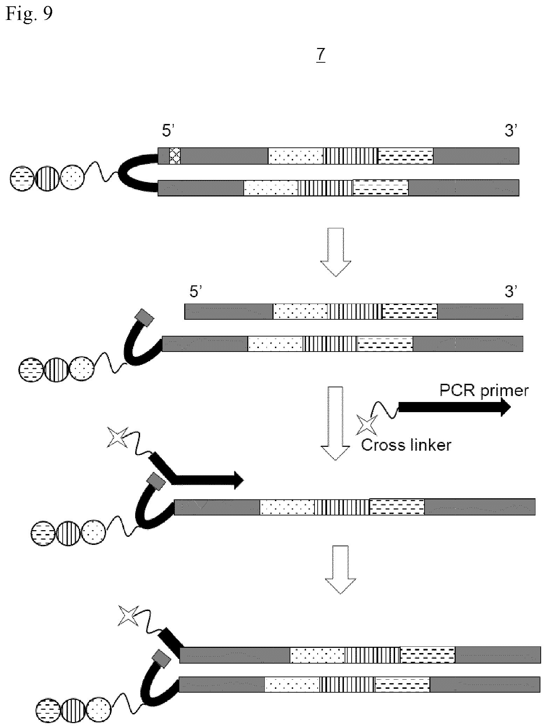

Fig. 9 shows an exemplary method for using a DEL of Form 7. In the present invention, by utilizing a cleavable site, a cross linker can be introduced.
   To the synthesized DEL compound, the cleavable site is cleaved (A), a modified primer is imparted (B) and based on the imparted primer, a cross linker-modified double-stranded DEL compound can be synthesized (C). The cross linker-modified double-stranded DEL compound can markedly improve detection sensitivity in screening of the DEL library (see Non-Patent Documents 5 and 6, etc.).

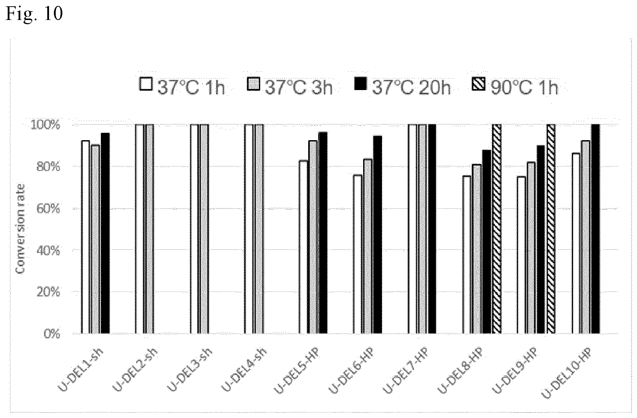

Fig. 10 is a graph representing a conversion rate of the cleavage reaction at each incubation time when the cleavage reaction of a partial structure (10 kinds of U-DEL1-sh, U-DEL2-sh, U-DEL3-sh, U-DEL4-sh, U-DEL5-HP, U-DEL6-HP, U-DEL7-HP, U-DEL8-HP, U-DEL9-HP and U-DEL10-HP) of a hairpin type DEL containing deoxyuridine by a USER (Registered trademark) enzyme was verified in Example 1.

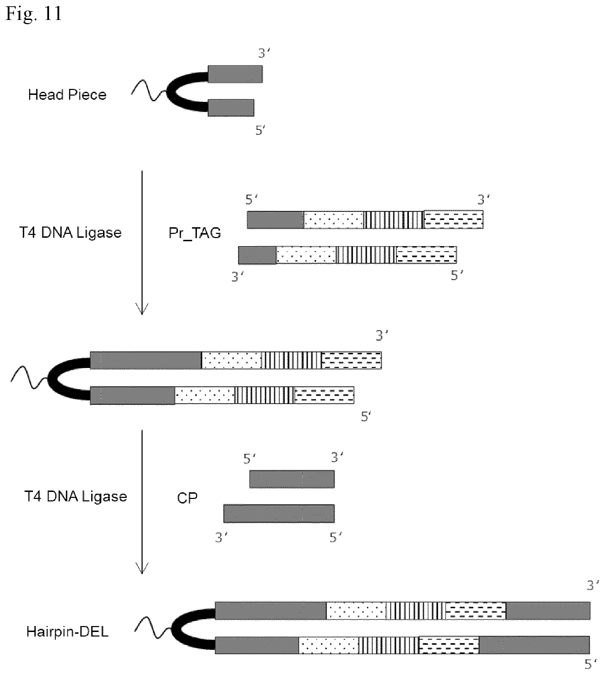

Fig. 11 is a schematic drawing showing synthetic procedure of various kinds of hairpin DEL (U-DEL1, U-DEL2, U-DEL4, U-DEL7, U-DEL8, U-DEL9, U-DEL10, H-DEL, U-DEL5, U-DEL11, U-DEL12, U-DEL13, I-DEL1, I-DEL2, I-DEL3, R-DEL1 and BIO-DEL) in Examples 2, 3, 4, 5 and 7. Head pieces corresponding to each are used as raw materials and a hairpin DEL synthesis is accomplished by two-step double-stranded ligation with a double-stranded oligonucleotide Pr_TAG and CP.

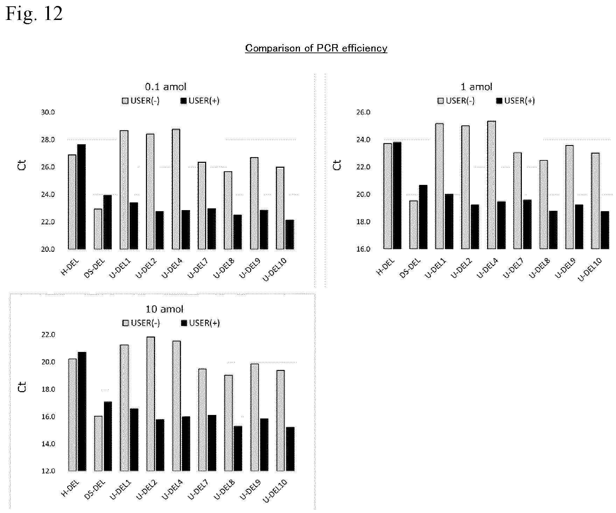

Fig. 12 is a graph showing each sample amount which shows the Ct value measured by the real-time PCR of 8 kinds of hairpin DELs (U-DEL1, U-DEL2, U-DEL4, U-DEL7, U-DEL8, U-DEL9, U-DEL10 and H-DEL) and a double-stranded DEL (DS-DEL) in Example 2. Samples in which various kinds of DEL are treated by USER (Registered trademark) enzyme are indicated to as "USER(+)" and untreated samples are indicated as "USER(-)". Cleavable hairpin DELs (U-DEL1, U-DEL2, U-DEL4, U-DEL7, U-DEL8, U-DEL9 and U-DEL10) containing deoxyuridine show the same Ct values as the Ct value of the double-stranded DEL (DS-DEL) after treatment with a USER (Registered trademark) enzyme.

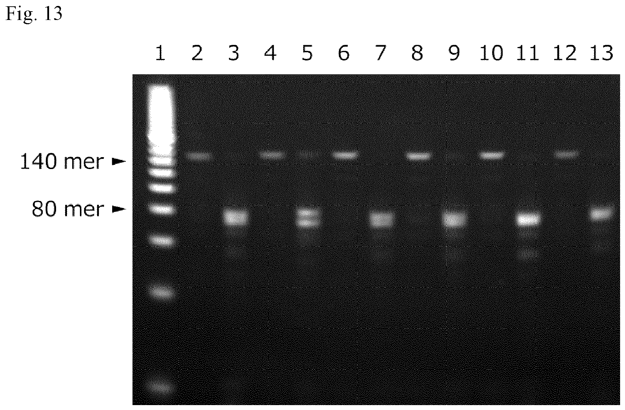

Fig. 13 is an image of a gel obtained by modified polyacrylamide gel electrophoresis showing the progress of the cleavage reaction by a USER (Registered trademark) enzyme of 6 kinds of hairpin DELs (U-DEL5, U-DEL7, U-DEL9, U-DEL11, U-DEL12 and U-DEL13) containing deoxyuridines in Example 3. Incidentally, the numbers in the figure indicate the numbers of each lane.

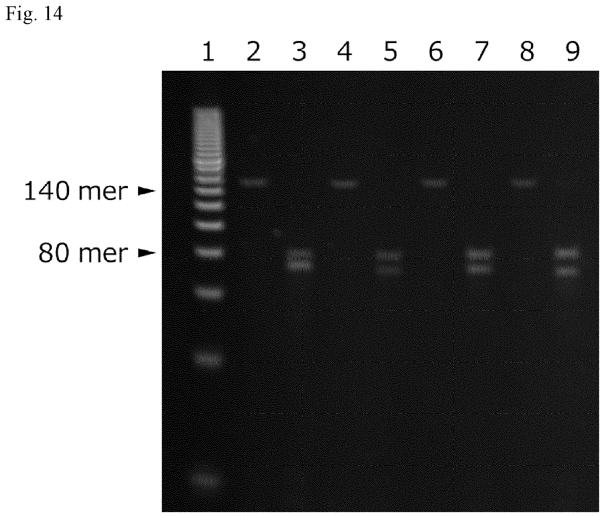

Fig. 14 is an image of a gel obtained by modified polyacrylamide gel electrophoresis showing the progress of the cleavage reaction by endonuclease V of 4 kinds of hairpin DELs (I-DEL1, I-DEL2, I-DEL3 and I-DEL4) containing deoxyinosines in Example 4. Incidentally, the numbers in the figure indicate the numbers of each lane.

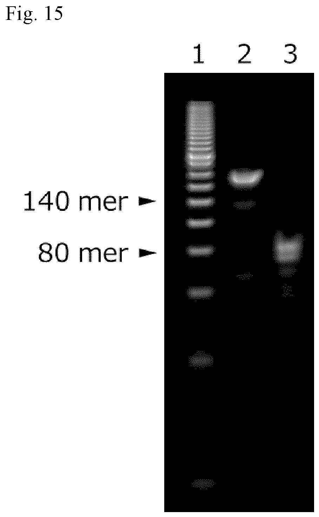

Fig. 15 is an image of a gel obtained by modified polyacrylamide gel electrophoresis showing the progress of the cleavage reaction by RNaseHII of hairpin DEL (R-DEL1) containing ribonucleoside in Example 5. Incidentally, the numbers in the figure indicate the numbers of each lane.

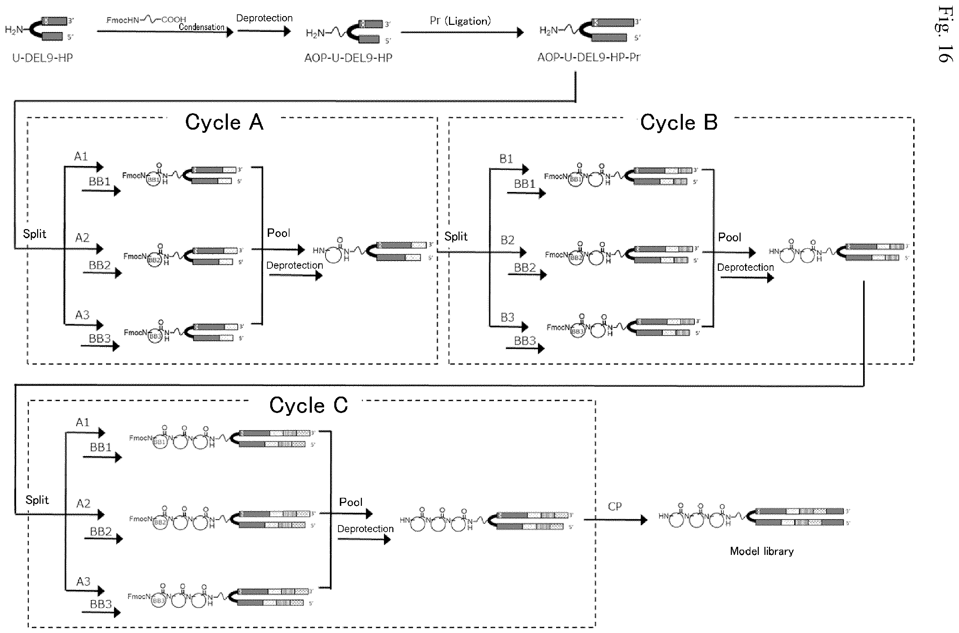

Fig. 16 is a schematic drawing showing a synthetic route of a model library containing 3×3×3 (27) compound species using U-DEL9-HP as a raw material. In Example 6, synthesis of a model library is accomplished by 3 times (Cycles A, B and C) of split-and-pool steps using U-DEL9-HP as a raw material. Also, in each cycle, a ligation reaction of a double-stranded oligonucletide tag and a chemical reaction for introducing building blocks are contained.

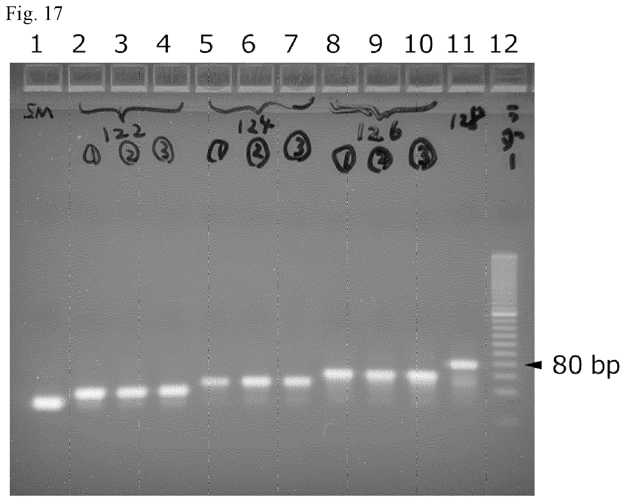

Fig. 17 is an image of a gel obtained by agarose gel electrophoresis showing the progress of a ligation reaction of each cycle in a model library synthesis of Example 6. Incidentally, the numbers in the figure indicate the numbers of each lane.

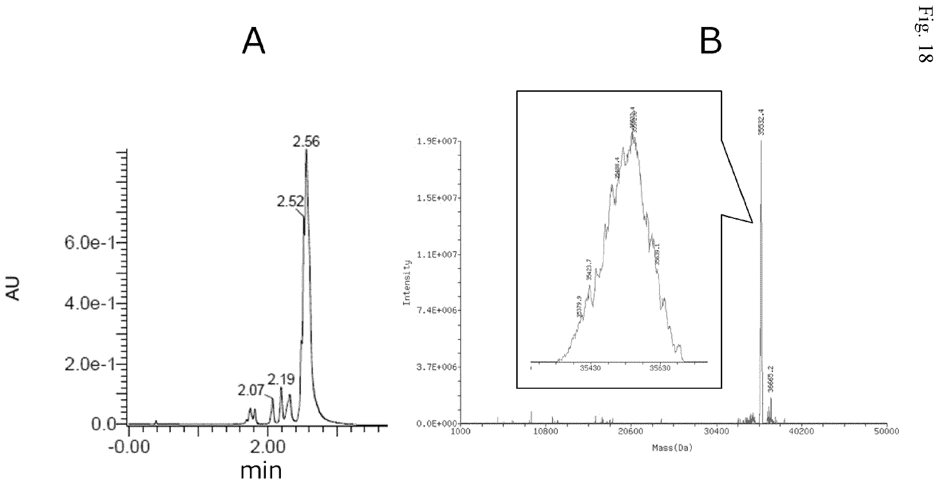

In Fig. 18, Fig. 18A is a chromatograph obtained from a sample after completion of Cycle C in model library synthesis of Example 6. Fig. 18B is a result of deconvolution of MS spectrum obtained by the sample after completion of Cycle C in a model library synthesis of Example 6.

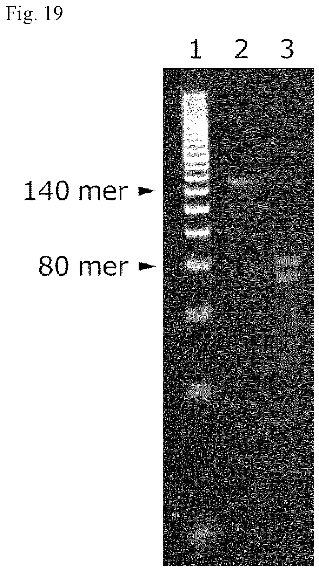

Fig. 19 is an image of a gel obtained by modified polyacrylamide gel electrophoresis showing the progress of the cleavage reaction by a USER (Registered trademark) enzyme of a model library in Example 6. Incidentally, the numbers in the figure indicate the numbers of each lane.

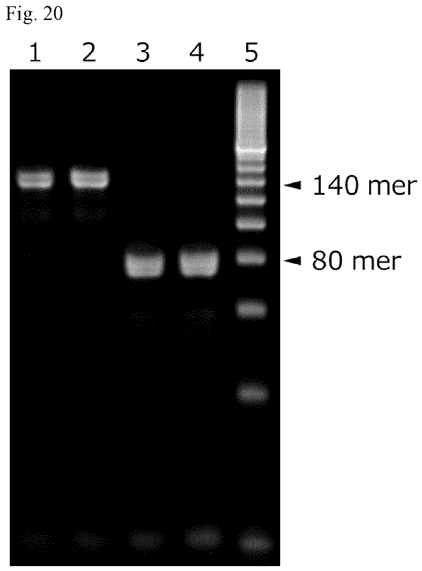

Fig. 20 is an image of a gel obtained by modified polyacrylamide gel electrophoresis showing the progress of the cleavage reaction by a USER (Registered trademark) enzyme of a DEL compound "BIO-DEL" having biotin at the 3' terminal in Example 7. Incidentally, the numbers in the figure indicate the numbers of each lane.

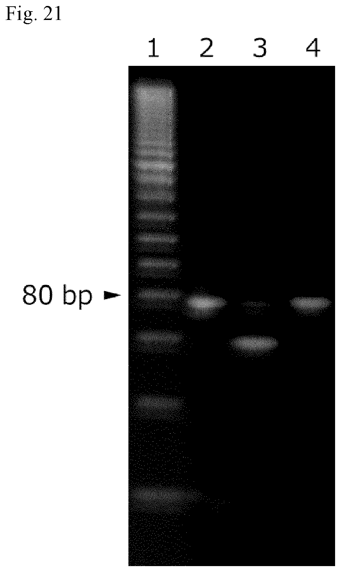

Fig. 21 is an image of a gel obtained by polyacrylamide gel electrophoresis showing the result of subjecting to a primer elongation reaction using a DEL compound "SS-DEL" having a single-stranded DNA and a photoreactive cross linker-modified primer "PXL-Pr" in Example 7. Incidentally, the numbers in the figure indicate the numbers of each lane.

### EMBODIMENTS TO CARRY OUT THE INVENTION

Whereas it is as mentioned above and it is a concept well known to those skilled in the art, in the present invention, a compound library means a group of compound derivatives in which compounds having a specific activity such as a drug candidate compound are systematically collected. This compound library is, in many cases, synthesized based on the synthetic techniques and methodologies of combinatorial chemistry. Combinatorial chemistry is an experimental method for efficiently synthesizing a series of compound libraries enumerated and designed based on combinatorics with a wide variety of compounds by a systematic synthetic route and a research field relating to it.

Whereas it is as mentioned above and it is well known to those skilled in the art, there is a DNA-encoded library as one kind of compound library based on combinatorial chemistry. The DNA-encoded library is appropriately abbreviated as DEL. Also, DEL is essentially synonymous with a DNA-encoded compound library.

In the present invention, the DNA-encoded library means a library in which a tag of DNA is added to each compound in the library. In the tag of DNA, a sequence is so designed that each structure of each compound can be identified and functions as a label of the compound.

Nucleotides are, in general, understood as substances in which a phosphate group is bound to a nucleoside. Whereas nucleotides and nucleosides are terms well known to those skilled in the art, nucleosides are, as one general embodiment, understood as materials in which a nucleic acid base such as a purine base or a pyrimidine base, etc., is subjected to a glycoside bond to the 1-position of a sugar such as a pentose, etc. Nucleosides and nucleotides are also units that constitute nucleic acids such as DNA and RNA, etc.

Also, nucleic acid is a well-known concept for those skilled in the art and as a general embodiment, it is understood as a polymer of nucleotides.

As one embodiment, the nucleic acid of the present invention is a polymer constituted by nucleotides and nucleic acid analogues mentioned later.

Also, in the present specification, in addition to a nucleic acid polymer constituted by nucleotide and nucleic acid analogues, a nucleic acid monomer such as nucleotides and nucleic acid analogues, etc., is also simply referred to as a nucleic acid. The latter usage is also a usage according to common general technical knowledge and can be understood by those skilled in the art according to the context as appropriate.

Nucleotides in a broad sense include, in addition to natural nucleotides (original nucleotides), artificial nucleotides (various kinds of nucleic acid analogues).

Nucleotides in a broad sense in the present invention include the following embodiments.
(A) Nucleotides of natural nucleosides
   (Examples of the nucleosides may be mentioned adenosine, thymidine, guanosine, cytidine, uridine, deoxyadenosine, deoxyuridine, deoxyguanosine, deoxycytidine, inosine or diaminopurine deoxyriboside.)
(B) Nucleotide of nucleoside having analogue of nucleic acid base
   (Examples of the nucleoside having an analogue of nucleic acid base may be mentioned 2-aminoadenosine, 2-thiothymidine, pyrrolopyrimidine deoxyriboside, 3-methyladenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, 6-O-methylguanosine or 2-thiocytidine.)
(C) Nucleotides having intercalated nucleic acid base
(D) Unnatural nucleotides having ribose or 2'-deoxyribose
(E) Nucleotides having modified sugar in sugar moiety
   (Examples of the modified sugar may be mentioned modified ribose, modified 2'-deoxyribose, 2'-O-methylribose, 2'-fluororibose, D-threoninol, arabinose, hexose, anhydrohexytol, altritol or mannitol.)
(F) Nucleic acid analogues
   (Examples of the nucleic acid analogues may be mentioned nucleic acid in which oxygen in cyclohexanyl nucleic acid, cyclohexenyl nucleic acid, morpholinonucleic acid (PMO), locked nucleic acid (LNA), glycol nucleic acid (GNA), threose nucleic acid (TNA), serinol nucleic acid (SNA), acyclic threoninol nucleic acid (aTNA) or ribose is replaced.)
Hereinafter, each nucleic acid analogue will be explained in detail.

### (F1) PMO

PMO is a nucleic acid analogue having a morpholine ring in the sugar moiety and a no electric charge phosphorodiamidate structure in the phosphoric acid diester site.

### (F2) LNA

LNA is a nucleic acid analogue having a crosslinked structure in the sugar moiety and the most typical example is that 2'-hydroxyl of ribose is crosslinked by a C1 to 6 alkylene or C1 to 6 heteroalkylene on the 4'-carbon of the same ribose sugar. Examples of the crosslinked structure may be mentioned methylene, propylene, ether or amino crosslinked structure

Typical LNA may be mentioned, 2',4'-BNA (2'-O,4'-C-methano-crosslinked nucleic acid).

### (F3) GNA

Glycol nucleic acid is also called GNA. For example, R-GNA or S-GNA may be mentioned. In this case, ribose is repriced by a glycol unit(s) bonded to the phosphodiester bond.

### (F4) TNA

Threose nucleic acid is also called TNA. In this case, ribose is repriced by α-L-threofuranosyl-(3'→2').

### (F5) SNA

Serinol nucleic acid is also called SNA. In this case, ribose is repriced by a serinol unit(s) bonded to the phosphodiester bond.

### (F6) aTNA

Acyclic threoninol nucleic acid is also called aTNA. For example, D-aTNA or L-aTNA may be mentioned. In this case, ribose is repriced by a threoninol unit(s) bonded to the phosphodiester bond.

### (F7) Oxygen-replaced sugar in ribose

Specific examples may be mentioned a replaced material of oxygen with S, Se or alkylene (for example, methylene or ethylene may be mentioned.).
(G) Skeletal-modified nucleotide
   (Examples where the skeleton is a modified nucleotide may be mentioned a peptide nucleic acid (the peptide nucleic acid is also called PNA. In this case, 2-aminoethyl-glycine linkage is replaced with ribose and phosphodiester skeleton.).)
(H) Phosphate group-modified nucleotide
   (Examples where the phosphate group is a modified nucleotide may be mentioned phosphorothioate, 5'-N-phosphoroamidite, phosphoroselenate, boranophosphoric acid, boranophosphate, hydrogen phosphonate, phosphoramidate, phosphorodiamidate, alkyl or aryl phosphonate, phosphotriester, crosslinked phosphoramidate, crosslinked phosphorothioate or crosslinked methylene-phosphonate, etc.)

Oligonucleotide, oligonucleotide chain, a double-stranded oligonucleotide, a double-stranded oligonucleotide chain and a double-stranded DNA of the present invention in the following explanation is the nucleotide as defined above.

In the present invention, when it is described as a nucleotide without any particular limitation, it means a natural nucleotide. The natural nucleotide is a term well known to those skilled in the art and is not particularly limited as long as it is essentially naturally existing nucleotide. As one embodiment, the natural nucleotide in the present invention is the nucleotide described in the above (A).

### (Nucleic acid analogue)

Nucleic acid analogue is a term well known to those skilled in the art and the structure of the nucleic acid analogue in the present invention is not limited as long as it has the effect of the present invention

As one embodiment, the nucleic acid analogue is a compound of the embodiments of the above (B) to (H).

As one embodiment, the nucleic acid analogue in the present invention is a compound having a phosphoric acid-corresponding site and a hydroxyl group-corresponding site in the nucleic acid monomer. The nucleic acid analogue is more preferably a compound having a phosphoric acid site and a hydroxyl group.

As one embodiment, the nucleic acid analogue in the present invention is a compound that can be utilized as a monomer in a nucleic acid synthesizer. Whereas it is well known for those skilled in the art, in the nucleic acid synthesizer, by utilizing it as a monomer in which phosphoric acid (or corresponding site) of the nucleic acid analogue is converted into a phosphoramidite and a hydroxyl group (or its corresponding site) is protected by a protective group, a nucleic acid oligomer can be synthesized.

Also, the partial structure other than the phosphoric acid site (or corresponding site) and the hydroxyl group (or corresponding site) in the nucleic acid analogue can be said to be a nucleic acid analogue residue. The structure of the nucleic acid analogue residue is not limited as long as it has the effect of the present invention, here, as a reference, when the characteristics of the respective structures of the natural nucleic acids (deoxyadenosine, thymidine, deoxycytidine, deoxyguanosine) are confirmed, there may be mentioned that the molecular weight is from 322 (thymidine monophosphate) to 347 (deoxyguanosine monophosphate) or so and the number of the atoms between the residues of the hydroxyl group oxygen atom at the 3' position and the phosphorus atom at the 5' position constituting the nucleic acid strand (including the oxygen atom and the phosphorus atom. Hereinafter also referred to as the number of atoms between the residues) is 6. Also, as the nucleic acid analogue capable of utilizing for a nucleic acid synthesizer, the following are known.

| | |
|---|---|
| Amino C6 dT | Molecular weight: 476, Number of atoms between residues: 6 |
| mdC(TEG-Amino) | Molecular weight: 526, Number of atoms between residues: 6 Uni-Link (trademark registration) Amino Modifier |
| | Molecular weight: 227, Number of atoms between residues: 6 (see Literature Nucleic Acid Research 1992, vol. 20, pp. 6253-6259) |
| d-Spacer | Molecular weight: 198, Number of atoms between residues: 6 triethylene glycol phosphoric acid ester(Spacer9) Molecular weight: 230, Number of atoms between residues: 11 |

As a reference, the structure of each nucleic acid analogue is described below.

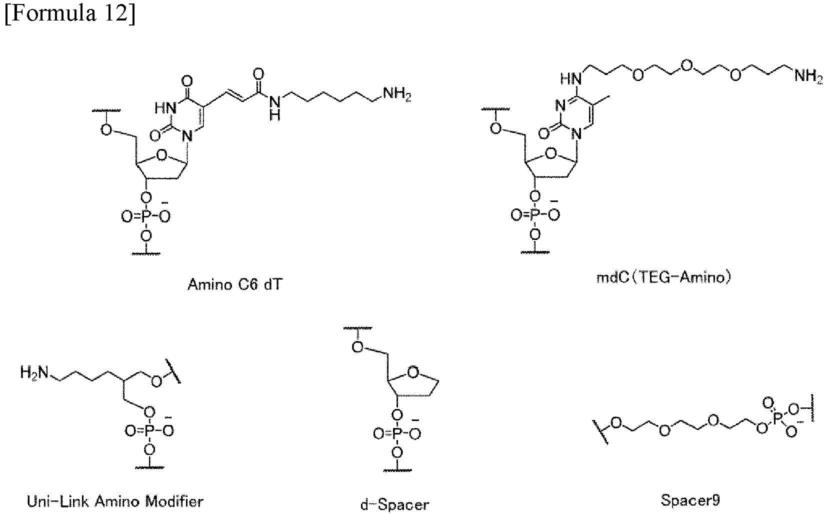

Accordingly, as one embodiment, the nucleic acid analogue is a compound (B 1) characterized by the following.
(B 1 1) It has phosphoric acid (or a corresponding site) and a hydroxyl group (or its corresponding site).
(B12) It is constituted by carbon, hydrogen, oxygen, nitrogen, phosphorus or sulfur.
(B13) The molecular weight is from 142 to 1,500.
(B14) The number of atoms between the residues is 5 to 30.
(B15) The bonding mode of the atoms between the residues is either all single bonds or containing one to two double bonds and the remaining are single bonds.

As one embodiment, the nucleic acid analogue is a compound (B2) characterized by the following.
(B21) It has phosphoric acid and a hydroxyl group.
(B22) It is constituted by carbon, hydrogen, oxygen, nitrogen or phosphorus.
(B23) The molecular weight is from 142 to 1,000.
(B24) The number of atoms between residues is 5 to 20.
(B25) The bonding mode of the atoms between the residues is all single bonds.

As one embodiment, the nucleic acid analogue is a compound (B3) characterized by the following
(B31) It has phosphoric acid and a hydroxyl group.
(B32) It is constituted by carbon, hydrogen, oxygen, nitrogen or phosphorus.
(B33) The molecular weight is from 142 to 700.
(B34) The number of atoms between residues is 5 to 12.
(B35) The bonding mode of the atoms between the residues is all single bonds.

As one embodiment, the nucleic acid analogue is a following compound (B41), (B42), (B43), (B44), (B5), (B51) or (B52).
(B41) d-Spacer
(B42) Amino C6 dT
(B43) mdC(TEG-Amino)
(B44) Uni-Link (trademark registration) Amino Modifier
(B5) Polyalkylene glycol phosphoric acid ester
(B51) Diethylene glycol phosphoric acid ester or triethylene glycol phosphoric acid ester
(B52) Triethylene glycol phosphoric acid ester

In the present invention, an oligonucleotide and oligonucleotide chain mean a polymer of a nucleotide having one or more nucleotides at internal positions between the 5' terminal and the 3' terminal and between the 5' terminal and the 3' terminal.

Mutually complementary base sequence means a sequence of nucleotides which can form the so-called complementary base pairs that form a fixed pair of adenine and thymine (or uracil), or guanine and cytosine between two oligonucleotides of nucleic acids and are linked by hydrogen bonds. Formation of the complementary base pairs is also called hybridization.

Incidentally, the complementary base pairs are a concept generally called "Watson-Crick type base pairs" and "natural type base pairs". Provided that, the base pairs may be Watson-Crick type, Hoogsteen type base pairs, or base pairs by other hydrogen bond motif (for example, diaminopurine and T, 5-methyl C and G, 2-thiothymine and A, 6-hydroxypurine and C, pseudoisocytosine and G) formation, etc. As long as two oligonucleotides are sequences that can form a double strand and can be used for the purpose of the present invention, there is no limitation on the sequence of "mutually complementary base sequence" and there is no limitation on the homology between the two sequences. The homology is preferably, in a more preferable order, 99% or more, 98% or more, 95% or more, 90% or more, 85% or more, 80% or more, 70% or more, 60% or more or 50% or more.

Whereas it is repeated again, to hybridize in the present invention means an act to form a double strand by oligonucleotides or oligonucleotide chains containing mutually complementary base sequences and a phenomenon to form a duplex by oligonucleotides or oligonucleotide chains containing complementary sequences.

The duplex in the present invention means a state that two nucleic acid strands form (hybridize) complementary base pairs. The two nucleic acid strands may be derived from two nucleic acid strands or may be derived from two nucleic acid sequences in one nucleic acid strand molecule.

In the present invention, the double-stranded oligonucleotide and the double-stranded oligonucleotide chain mean a secondary structure formed by hybridizing two or more different oligonucleotide chains. The chain lengths of the two oligonucleotides may be different and may have regions that are not hybridized.

Incidentally, the region where the double strand hybridizes is a duplex.

In the present invention, the double-stranded DNA means a secondary structure formed by hybridizing two different DNA strands. The chain lengths of the respective DNA strands may be different and may have regions that are not hybridized. The DNA strands are not limited to naturally existing deoxyribonucleotides and mean all oligonucleotide chains that can be amplified by DNA polymerase.

In the present invention, "forming a duplex" may be forming a duplex under standard conditions for handling oligonucleotides, for example, at a temperature of 4 to 40°C, an aqueous solvent and a pH of 4 to 10. For example, even if there is a case where no duplex is formed by the specific solvent and conditions, if the nucleic acid forms a duplex under standard conditions, the nucleic acid is a nucleic acid that forms a duplex.

In the present invention, the Tm value refers to a temperature at which half of the DNA molecules are annealed with the complementary strand.

In the present invention, the blunt end means that both terminals of the double-stranded oligonucleotide are paired without protruding.

In the present invention, the sticky end means that, among the terminals of the double-stranded oligonucleotide, one of the chain has a protruded portion. The protruded portion of the sticky end can be of any length and the length is preferably 1 to 50 bases, more preferably 1 to 30 bases, further preferably 1 to 15 bases and most preferably 2 to 6 bases. In a specific embodiment, it is possible that the protruded portion can be used as a hybridizing region for carrying out ligation of the sticky terminal.

PCR means a polymerase chain reaction. PCR is an amplifying means of the oligonucleotide chains and is a technique well known to those skilled in the art. When the outline of the process of PCR is explained, in PCR, (1) the double-stranded oligonucleotide chain to be amplified was dissociated into two single strands by heat treatment, etc. and (2) after adjusting the temperature suitable for the enzymatic reaction, strands complementary to the respective single strands are synthesized by an enzyme (DNA polymerase, etc.) existing in the reaction system. That is, one double-stranded oligonucleotide can be amplified in two. In PCR, oligonucleotide chains can be amplified with high efficiency by repeating the processes (1) and (2) by adjusting the temperature.

In the present invention, the primer means an oligonucleotide that is annealed to an oligonucleotide chain which becomes a template and can be elongated by a polymerase in a template-dependent manner.

In the present invention, the primer sequence for PCR means a sequence of a portion of the oligonucleotide chain to which the primer is annealed and is preferably a sequence suitable for PCR as known in this field of the art and is preferably present at the terminal of the oligonucleotide chain.

In the present invention, the nick means a portion of the double-stranded oligonucleotide chain in which a linkage between the nucleotides is lacking and the oligonucleotide chain is broken. The 5' side of this lacking portion may have a phosphoric acid group or may not have a phosphoric acid group.

In the present invention, the gap means a portion of the double-stranded oligonucleotide chain in which one or more consecutive nucleotides are deleted and the oligonucleotide chains are separated. The 5' side of the deleted portion may have a phosphoric acid group or may not have a phosphoric acid group.

In the present invention, the hairpin strand is a single-stranded structure in which two complementary nucleic acid strands are linked and the characteristics of the hairpin strand and the hairpin strand DEL are as described above. The terms "hairpin site", "hairpin structure" and "hairpin type" used in the present invention are understood as terms derived from the hairpin having the same concept as the above-mentioned "hairpin strand".

In the present invention, the nucleic acid ligation reaction and ligation mean a reaction in which the terminals nucleic acids are linked to each other.

The nucleic acid ligation reaction by an enzyme and enzymatic ligation means a reaction in which the terminals nucleic acids are linked to each other using an enzyme.

An enzyme that can be used in the nucleic acid ligation reaction is, for example, DNA ligase, RNA ligase, DNA polymerase, RNA polymerase or topoisomerase.

As one embodiment, DNA ligase is an enzyme that ligates the terminals of DNA strands with a phosphoric acid diester bond. As one embodiment, DNA ligase is understood as a ligase belonging to EC number: 6.5.1.1 or 6.5.1.2. DNA ligase is also called polydeoxyribonucleotide synthase or polynucleotide ligase, etc. Examples of DNA ligase may be mentioned DNA ligase I, II, III, IV and T4 DNA ligase, etc.

As one embodiment, RNA ligase is an enzyme that ligates the terminals of RNA strands with a phosphoric acid diester bond. As one embodiment, RNA ligase is understood as a ligase belonging to EC number: 6.5.1.3. Also, as one embodiment, RNA ligase belongs to the lineage of poly(ribonucleotide): poly(ribonucleotide) ligase. RNA ligase is also called polyribonucleotide synthase or polyribonucleotide ligase.

In the present invention, the chemical ligation means a reaction in which the terminals of the nucleic acids are bound to each other without using an enzyme.

In the chemical ligation, a ligating portion is formed by reacting the terminals of the nucleic acids having a functional group which becomes a pair of the chemical reaction. The functional group which becomes a pair of the chemical reaction may be mentioned, for example, a pair of an alkynyl group which may be substituted and an azide group which may be substituted, a pair of a diene which may be substituted having a 4π electron system (for example, a 1,3-unsaturated compound which may be substituted, for example, there may be mentioned 1,3-butadiene, 1-methoxy-3-trimethylsilyloxy-1,3-butadiene, cyclopentadiene, cyclohexadiene or furan, each of which may be substituted.) and a dienophile which may be substituted or a heterodienophile which may be substituted (for example, there may be mentioned an alkenyl group which may be substituted or an alkynyl group which may be substituted.) having a 2π electron system, a pair of an amino group which may be substituted and a carboxylic acid group, a pair of a phosphorothioate group and an iodo group (for example, there may be mentioned a phosphorothioate group at the 3' terminal and an iodo group at the 5' terminal.) or a pair of a phosphoric acid group and a hydroxy group (for example, there may be mentioned a pair of a phosphoric acid group at the 5' terminal and a hydroxy group at the 3' terminal or a pair of a hydroxy group at the 5' terminal and a phosphoric acid group at the 3' terminal.).

The chemical ligation is a concept well known to those skilled in the art and those skilled in the art can appropriately achieve chemical ligation based on common general technical knowledge. In addition to the above, it can be also referred to Artificial DNA; PNA & XNA, 2014, vol. 5, e27896, Current Opinion in Chemical Biology, 2015, vol. 26, pp. 80-88, etc.

In the present invention, "selectively cleavable" means that, in a certain compound, only a specific site can be selectively cleaved under predetermined conditions without changing the other molecular structures of the compound.

In the present invention, "selectively cleavable site" means, in a certain compound, a site that can be selectively cleaved under predetermined conditions.

As one embodiment, the preferred structure of the "selectively cleavable site" in the present invention is a "selectively cleavable nucleic acid". The site may be a site constituted by a plurality of nucleic acids, that can be successfully cleaved by a specific sequence, or may be a site constituted by a single nucleic acid. When the cleavable site is a nucleic acid, it is preferable in the viewpoints that (1) the established producing method such as a nucleic acid synthesizer, etc., can be utilized so that production efficiency is good, (2) in the reaction conditions for constructing the building blocks of DEL, it is essential that the nucleic acid at the DNA tag portion is not decomposed, so that the cleavable site is nucleic acid, it does not decompose as well, etc.

More preferred structure of the above-mentioned "selectively cleavable nucleic acid" is nucleic acid containing a nucleotide which is not contained in the sequence of the DNA tag of DEL. If the cleavable site is a nucleotide which is not contained in the sequence of the DNA tag, it is possible to utilize it without limiting the sequence of the DNA tag to avoid cleavage of the DNA tag portion.

As the nucleic acid used for the sequence of the DNA tag, deoxyadenosine, deoxyguanosine, thymidine and deoxycytidine are preferable . Accordingly, the preferred structure of the selectively cleavable site is a nucleic acid that is neither deoxyadenosine, deoxyguanosine, thymidine nor deoxycytidine.

As examples of the "selectively cleavable site", there may be mentioned a "nucleotide having a cleavable base". For example, in the "nucleotide having a cleavable base" in DEL, the N-glycoside bond between the base portion and the sugar portion is cleaved by the action of DNA glycosylase to leave an abasic site. Phosphodiester bond adjacent to the abasic site is cleaved by change in chemical conditions (for example, temperature rise, basic hydrolysis, etc.), or an enzyme having depurine/depyrimidine (AP) endonuclease activity or AP raise activity (for example, endonuclease III, endonuclease IV, endonuclease V, endonuclease VI, endonuclease VII, endonuclease VIII, APE1 (human-derived AP endonuclease), Fpg (formamide pyridine-DNA glycosylase), etc.) to form a gap with one base portion, or a nick.

Examples of the "nucleotide having a cleavable base" may be mentioned deoxyuridine, bromodeoxyuridine, deoxyinosine, 8-hydroxydeoxyguanosine, 3-methyl-2'-deoxyadenosine, N6-etheno-2'-deoxyadenosine, 7-methyl-2'-deoxyguanosine, 2'-deoxyxanthosine, 5,6-dihydroxydeoxythymidine, etc. Nucleotides having other cleavable bases are obvious to those of skill in the art. By incorporating these "nucleotides having a cleavable base" into DEL and using a DNA glycosylase that specifically recognizes the structure, the DEL is selectively debased.

In the present invention, the DNA glycosylase refers to an enzyme which is an optional enzyme having glycosylase activity, recognizes an optional nucleic acid base portion in the oligonucleotide, cleaves an N-glycoside bond between the base portion and the sugar portion and creates an abasic site. For example, there may be mentioned uracil DNA glycosylase (recognizes deoxyuridine), alkyladenine DNA glycosylase (recognizes 3-methyl-2'-deoxyadenosine, 7-methyl-2'-deoxyguanosine and deoxyinosine), Fpg (recognizes 8-hydroxydeoxyguanosine), endonuclease VIII (recognizes 5,6-dihydroxydeoxythymidine or decomposed pyrimidine base such as uracil glycol, etc.), SUMG1 (abbreviation of single-strand selective uracil DNA glycosylase, which recognizes deoxyuridine), etc.

In the present invention, more preferable example of the "selectively cleavable site", deoxyinosine and deoxyuridine are mentioned.

In the present invention, particularly preferable example of the "selectively cleavable site", deoxyuridine is mentioned.

As one embodiment, the "selectively cleavable site" in the present invention is preferably cleaved using an enzyme. The enzyme generally has high substrate specificity and does not recognize the DNA tag portion of DEL and the compound portion constructed by a plurality of building blocks as a substrate and recognizes only the "selectively cleavable site" and acts so that it is preferable. Also, cleavage using the above-mentioned enzyme may be achieved by changing the structure of the "selectively cleavable site" by the enzyme and then changing the chemical conditions. Examples of the enzyme may be mentioned glycosylase and nuclease.

In the present invention, the glycosylase is an enzyme having a function of hydrolyzing a glycoside bond (a covalent bond formed by dehydration condensation of a sugar molecule and another organic compound). Among them, the DNA glycosylase is an enzyme that recognizes the nucleic acid base portion in the oligonucleotide, as mentioned above and hydrolyzes the glycoside bond.

In the present invention, the nuclease is an enzyme having a function of hydrolyzing a phosphodiester bond between the sugar and the phosphoric acid of the nucleic acid. In the nuclease, for example, AP endonuclease, nicking endonuclease and ribonuclease are contained.

The AP endonuclease cleaves a phosphodiester bond adjacent to the abasic site formed by the action of an optional DNA glycosylase as mentioned above. Accordingly, in the present invention, it is preferable to use DNA glycosylase and AP endonuclease in combination.

The nicking endonuclease (for example, Nb. BbvCI, Nb. BsmI, Nb. BsrDI, etc.) recognizes a specific DNA sequence and generates a nick in which a phosphodiester bond is cleaved only one of the strand among the double strand. Also, the endonuclease V can generate a nick in which the second phosphodiester bond is cleaved in the 3' direction from the deoxyinosine, which is useful for carrying out the present invention.

The ribonuclease is an enzyme that decomposes RNA. In the present invention, the ribonucleoside is used as the "selectively cleavable site" and by allowing ribonuclease to act on it, it can be utilized. RNaseHII, which is a kind of the ribonuclease, can generate a nick in which the phosphodiester bond on the 5' side of the ribonucleotide incorporated into the DNA sequence is cleaved, which is useful for carrying out the present invention.

In the present invention, the USER (Registered trademark) means "Uracil-Specific Excision Reagent" Enzyme. The USER is an endonuclease cocktail that removes uracil including uracil DNA glycosylase (UDG) and endonuclease VIII. The USER removes uracil in the double-stranded DNA to generate a gap of one base to cleave the DNA strand. In the process of the USER, UDG firstly removes an uracil base to produce a abasic site. Subsequently, the endonuclease decomposes a phosphodiester bond to liberate a deoxyribose having no base to produce a gap of one base.

In the explanation of the present specification, USER (Registered trademark) enzyme and USER (Registered trademark) Enzyme are USER (Registered trademark) in the above-mentioned definition.

In the present invention, the building block is a portion that has a functional group and can constitute a part of a compound, which may be in the form of a compound.

In the present invention, the base sequence which can identify the respective building blocks means a specific base sequence designed to correspond to the structures of the respective building blocks. To design a sequence means, for example, to assign the nucleic acid base sequence to each structure such as to assign the nucleic acid base sequence AAA to the building block structure A, the nucleic acid base sequence TTT to the structure B and the nucleic acid base sequence CGC to the structure C. The sequence can be freely designed as long as the object of the present invention is achieved. For example, an optional number of base sequences can be assigned to one building block.

In the present invention, the oligonucletide tag is a partial structure containing an oligonucleotide which contains a base sequence capable of identifying the structure of the partial structure constructed by the building blocks. In the present invention, the oligonucletide tag may be an oligonucleotide corresponding to each building block, or may be a longer chain oligonucleotide containing an oligonucleotide corresponding to a plurality of building blocks.

The nucleotide constituting the oligonucletide tag of the present invention is not limited as long as it can accomplish the effect of the present invention and it is desirably a nucleotide suitable for these operations in the viewpoint of amplification by PCR and easiness of analysis by a sequencer Examples of such a preferable nucleotide may be mentioned a nucleotide having the above-mentioned natural nucleic acid base as the base portion and having the above-mentioned ribose or 2'-deoxyribose as the sugar portion and a more preferable example may be mentioned deoxyadenosine, thymidine, deoxycytidine or deoxyguanosine.

### (Head piece)

In the present invention, the head piece means a starting compound for producing a compound library such as DEL, etc. The structure of the head piece of the present invention is not limited as long as it can accomplish the object of the present invention and as the most typical embodiment, it contains in the structure at least one site to which the building block can be linked and at least one site to which the oligonucloetide tag can be ligated and further contains at least one selectively cleavable site in the structure.

As described later, the DNA tag is preferably a double-stranded oligonucleotide chain and the site to which the oligonucleotide tag can be ligated is preferably two.

As one embodiment, the head piece is a compound shown in the following schematic drawing.

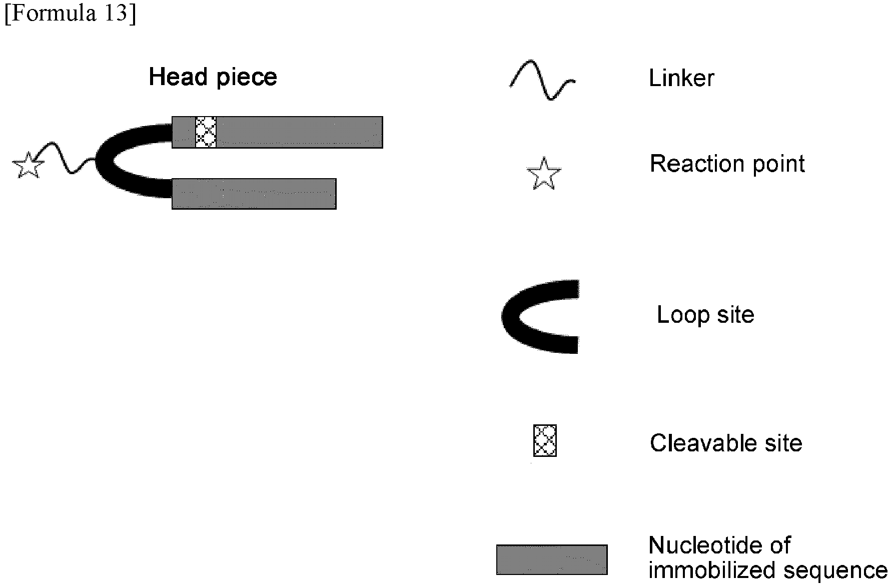

As one embodiment, the head piece is desirably to be chemically stable.

In addition, as one embodiment, the head piece preferably has a structure in which the DNA tag and the building block can be arranged in an appropriate space.

As one embodiment, it is preferable that the head piece has appropriate flexibility.

Here, more appropriate spatial arrangement and flexibility (structural characteristics of the head piece) will be explained. Incidentally, here, the structural characteristics of the head piece to be explained may be achieved by the head piece alone or may be achieved by coupling the head piece and the bifunctional spacer.

As one embodiment, the preferred structural characteristics of the head piece are structural characteristics that the head piece or the DNA tag does not inhibit the forming reaction of the building block and conversely, the head piece or the building block does not inhibit the elongation reaction of the DNA tag.

As one embodiment, the preferred structural characteristics of the head piece are structural characteristics that the head piece or the DNA tag portion does not affect the interaction between the building block compound (library compound) and the target (target protein, etc.).

As one embodiment, the preferred structural characteristics of the head piece are structural characteristics that the DNA tag and the building block site are oriented on opposite sides (for example, 90 degrees or more on the opposite side).

As one embodiment, the preferred structural characteristics of the head piece are structural characteristics that the loop site and the building block of the head piece are separated from several atoms to a dozen atoms in terms of the skeleton of the organic compound.

As one embodiment, the head piece preferably has the DNA tag portion, the building block portion and the appropriate affinity. The appropriate affinity means, for example, chemical reactivity and stability so as to form, maintain and cleave a bond under desired conditions to carry out the present invention.

Incidentally, in the present invention, the bifunctional spacer means a spacer portion having at least two reactive groups that enables binding between the building block site and the head piece.

In the explanation of the present invention, the terms of the "head piece", the "head piece compound" and the "compound for the head piece" are terms indicating compounds of the same concept.

In the explanation of the present invention, a "compound used as a head piece" can be understood essentially the same as "use of a compound as a head piece" from the viewpoint of use and can be understood essentially the same as the "method of using the compound as a head piece" from the viewpoint of method. The same applies to the compound library.

Hereinafter, a preferred structure of the head piece is explained and the structure of the head piece is not limited as long as the effects of the present invention are achieved.

As one embodiment, the head piece is constituted by,
(D) a reactive functional group having at least one site that can be linked directly to the building block, or linked indirectly via a bifunctional spacer,
(L) a linker elongating from the reactive functional group,
(E) a first oligonucleotide chain having one binding site that can be linked to one of the strands of the oligonucletide tag,
(F) a second oligonucleotide chain having one binding site that can be linked to another strand of the oligonucletide tag and
(LP) a loop site that that can be linked to the above-mentioned linker and two oligonucleotide chains and
has at least one selectively cleavable site at any of at least one site of E, F and LP.

As one embodiment, the head piece is a compound represented by the following formula (I).
The compound represented by 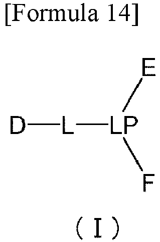
(wherein E and F are each independently
an oligomer constituted by nucleotides or nucleic acid analogues,
provided that E and F contain base sequences, which are complementary to each other and form a duplex oligonucleotide,
LP is a loop site,
L is a linker and
D is a reactive functional group.) and
the compound having at least one selectively cleavable site at any of at least one site of E, F and LP.

Incidentally, in the present invention, among the loop sites, the partial structure of the site that binds to the linker may be sometimes referred to as a linking site or (LS).

Also, in the present invention, E-LP-F may be sometimes collectively referred to as a hairpin site.

### (First and second oligonucleotide chains)

Hereinafter, preferred embodiments of the first oligonucleotide chain (E) and the second oligonucleotide chain (F) will be explained.

The first oligonucleotide chain (E) and the second oligonucleotide chain (F) preferably form a duplex in the molecule via the loop site (LP) and the head piece form a hairpin structure. The chain length preferable for the formation of the duplex in the molecule is 3 bases or more, more preferably 4 bases or more and further preferably 6 bases or more.

The chain length of E and F is, as one embodiment, each 3 to 40, respectively.

The chain length of E and F is, as one embodiment, each 4 to 40, respectively.

The chain length of E and F is, as one embodiment, each 6 to 25, respectively.

The site to which the oligonucleotide tag is linked preferably has a structure suitable for enzymatic ligation or chemical ligation. As one embodiment, the ligation of the head piece and the oligonucleotide tag is carried out by double-stranded ligation using an enzyme. In that case, it is preferred that the first and the second oligonucleotide chains form sticky ends for ligation. The above-mentioned chain length of the sticky end is preferably 2 bases or more, more preferably 2 to 10 bases and further preferably 2 to 5 bases. Accordingly, it is preferable that one of the first and the second oligonucleotide chains is longer than the other chain by the chain length of the sticky end. Also, for ligation with the DNA ligase, among the first and the second oligonucleotide chains, it is preferable that the 5' terminal of the chain having the 5' terminal of the head piece is phosphorylated.

In addition, the first and the second oligonucleotide chains may contain a part or whole of the primer binding sequence for PCR. The appropriate chain length for the primer binding sequence is 17 to 25 bases.

### (Linker)

Hereinafter, preferred embodiments of the linker (L) will be explained.

The linker is, as mentioned above, a site that elongates from the reactive functional group and binds to the linking site. Typically, the linker is a divalent group (-L-) derived from the following embodiments.

As one embodiment, the linker is the following embodiment (L1). (L1) C1 to 20 aliphatic hydrocarbon which may have a substituent(s) and may be replaced with 1 to 3 hetero atoms, or (2) C6 to 14 aromatic hydrocarbon which may have a substituent(s).

As the other embodiments, L is the following embodiment (L2), (L3), (L4) or (L5).
(L2)
   C1 to 6 aliphatic hydrocarbon which may have a substituent(s), 1 to 6 aliphatic hydrocarbon which may be replaced with one or two oxygen atoms, or C6 to 10 aromatic hydrocarbon which may have a substituent(s).
(L3)
   C1 to 6 aliphatic hydrocarbon substitutable with the substituent group ST1, or benzene substitutable with the substituent group ST1. Here, the substituent group ST1 is a group constituted by a C1 to 6 alkyl group, a C1 to 6 alkoxy group, a fluorine atom and a chlorine atom. Provided that when the substituent group ST1 is substituted with the aliphatic hydrocarbon, an alkyl group is not selected from the substituent group ST1.
(L4)
   C1 to 6 alkyl, or benzene which is unsubstituted or substituted by one or two C1 to 3 alkyl group(s) or C1 to 3 alkoxy group(s).
(L5)
   C1 to 6 alkyl.

### (Reactive functional group)

Hereinafter, the preferred embodiments of (D) the reactive functional group will be explained.

As described above, the reactive functional group has at least one site that can be directly linked to the building block, or indirectly linked via a bifunctional spacer and is a site that binds to the linker group. Typically, the reactive functional group becomes a monovalent group (D-) in the head piece and in the DEL, it becomes a "divalent group derived from the reactive functional group" (-D-) based on the above-mentioned (D-).

For example, when D is an amino group, the specific structure of (D-) is (R-HN-) (R is a substituent explained below). For example, it reacts with an activated carboxy group, a reactive sulfonyl group or an isocyanate group to form an amide bond, a sulfonamide bond, or urea bond, respectively. At that time, the specific structure of (-D-) is (-NR-).

R is not limited as long as the effects of the present invention are accomplished and in the following embodiments of (D1) to (D5), R is preferably (1) a hydrogen atom, or (2) a C1 to 6 alkyl group which is unsubstituted or substituted by 1 to 3 substituents selected solely or different from a substituent group consisting of a C1 to 6 alkoxy group, a fluorine atom and a chlorine atom.

R is more preferably a hydrogen atom or a C1 to 3 alkyl group and further preferably a hydrogen atom.

Also, for example, when (D-) is a methylene group having a leaving group (X-), the specific structure of (D-) becomes (X-CH₂-) and, for example, it reacts with a nucleophilic reagent such as an amino group, a hydroxy group or a thiol group to form a carbon-nitrogen bond, a carbon-oxygen bond or a carbon-sulfur bond. At that time, the specific structure of (-D-) becomes (-CH₂-). Also, for example, when (D-) is an aldehyde group, the specific structure of (-D-) is (HOC-). The aldehyde group forms, for example, a carbon-nitrogen bond by the reductive amination reaction with an amino group, at that time, (-D-) becomes -CH₂-, for example, forms a carbon-carbon double bond by the reaction with a phosphorus-iride group, at that time, (-D-) becomes -CH= and for example, forms a carbon-carbon triple bond by the reaction with an α-diazophosphonate group and at that time, (-D-) becomes -C=.

As one embodiment, the site (D-) is the following embodiment (D1)

### (D 1)

Functional groups capable of constituting C-C, amino, ether, carbonyl, amide, ester, urea, sulfide, disulfide, sulfoxide, sulfonamide or sulfonyl bond.
(whereas it is literal, in this case, (-D-) becomes C-C, amino, ether, carbonyl, amide, ester, urea, sulfide, disulfide, sulfoxide, sulfonamide or sulfonyl bond.)

As the other embodiments, (D-) is the following embodiment (D2), (D3), (D4) or (D5).

### (D2)

C1 hydrocarbon having a leaving group, an amino group, a hydroxyl group, a precursor of a carbonyl group, a thiol group or an aldehyde group.

Incidentally, in this case, (-D-) can be -(C1 hydrocarbon)-, -NR-, -O-, -(C=O)-, -S-, -CH₂-, -CH= or -C=, etc.

### (D3)

C1 hydrocarbon having a halogen atom(s), C1 hydrocarbon having a sulfonic acid-based leaving group, an amino group, a hydroxyl group, a carboxy group, a halogenated carboxy group, a thiol group or an aldehyde group.

Incidentally, in this case, (-D-) can be -(C1 hydrocarbon)-, -NR-, -O-, -(C=O)-, -S-, -CH₂-, -CH= or -C=, etc.

### (D4)

-CH₂Cl, -CH₂Br, -CH₂OSO₂CH₃, -CH₂OSO₂CF₃, an amino group, a hydroxyl group or a carboxy group.

Incidentally, in this case, (-D-) can be -CH₂-, -NR-, -O- or -(C=O)-, respectively.

### (D5)

### Primary amino group .

Incidentally, in this case, (-D-) becomes -NH-.

Hereinafter, preferred embodiments of the loop site (LP) will be explained.

The loop site (LP) is preferably so designed that the first oligonucleotide chain (E) and the second oligonucleotide chain (F) form a duplex in the molecule and the head piece can form a hairpin structure. That is, the loop site (LP) preferably has a chain length that makes the loop structure thermodynamically stable and flexibility of bonding.

Accordingly, as one embodiment, the loop site (LP) is as follows.
LP is
a loop site represented by (LP1)p-LS-(LP2)q,
LS is a partial structure selected from a compound group described in the following (A) to (C),
   (A) Nucleotide
   (B) Nucleic acid analogues
   (C) C1 to 14 trivalent group which may have a substituent(s)
LP1 is each a partial structure selected independently or differently with a number of p from a compound group described in the following (1) and (2),
   (1) Nucleotide
   (2) Nucleic acid analogues
LP2 is each a partial structure selected independently or differently with a number of q from a compound group described in the following (1) and (2),
   (1) Nucleotide
   (2) Nucleic acid analogues
and a total number of p and q is 0 to 40.

Further preferred embodiments of the loop site are as explained above.

Hereinafter, the structure of the loop site will be further supplemented.

Here, the nucleotide is the natural nucleotide of the above-mentioned explanation and the nucleic acid analogues is as the above-mentioned explanation.

Here, LP1 is each a partial structure selected independently or differently with a number of p from a compound group described in the following (1) and (2) and LP2 is each partial structure selected solely or differently from the compound groups described in the following (1) and (2) with a number of q.
(1) Nucleotide
(2) Nucleic acid analogues

Selected solely or differently with a number of q is that, for example, when p is 4, LP1 can be selected solely or differently from the compound group described in (1) and (2), like AATG, ATCG, TC (d-Spacer) G or A (d-Spacer) (d-Spacer) C. The same applies to LP2.

Also, the loop site may contain a part or whole of the primer bond sequence for PCR.

### (With regard to LS)

As one embodiment, LS is (A) a nucleotide or (B) a nucleic acid analogue.

When LS is (A) a nucleotide or (B) a nucleic acid analogue, the loop site becomes a nucleic acid oligomer. The nucleic acid oligomer of the present invention refers to an oligomer that is linked the nucleotide or the nucleic acid analogues as a monomer. The oligomer can be also said to be a chain state compound.

Accordingly, the nucleic acid oligomer of the present invention is either of an oligonucleotide chain, a nucleic acid analogue chain, or a mixed chain of a nucleotide and a nucleic acid analogue.

When LS is (A) a nucleotide or (B) a nucleic acid analogue, the loop site becomes a nucleic acid oligomer. In such a case, the head piece can be produced by a nucleic acid synthesizer, which is markedly preferable in practice.

When LS is (A) a nucleotide or (B) a nucleic acid analogue, in the production of the head piece, as one embodiment, a monomer for nucleic acid synthesis in which the linker site (L) and the reactive functional group site (D) are bound to LS is prepared and then a nucleic acid oligomer can be synthesized.

Examples of such a monomer for nucleic acid synthesis may be mentioned the above-mentioned Amino C6 dT, mdC(TEG-Amino), Uni-Link (trademark registration) Amino Modifier, etc.

In the case of this embodiment, for example, among the structures of mdC(TEG-Amino), which is the monomer, the nucleotide portion corresponds to the linking site (LS) and the side chain portion elongating from the base corresponds to the linker site (L) and the reactive functional group site (D).

In the preparation, the reactive functional group (D) may be protected by a protective group.

In such a case, as one embodiment, the nucleic acid analogue is the following compound (B6).
(B6) A compound in which the above-mentioned (-L-D) is bound to the base portion of a nucleotide.

As one embodiment, the nucleic acid analogue is the following compound (B61), (B62), (B63), (B64) or (B65).
(B61) (B6) in which (-L-D) is (-L1-D1)
(B62) (B6) in which (-L-D) is (-L2-D2).
(B63) (B6) in which (-L-D) is (-L3-D3).
(B64) (B6) in which (-L-D) is (-L4-D4).
(B65) The compound described in any of (B61) to (B64) in which (-D) is (-D5).

When LS is (A) a nucleotide or (B) a nucleic acid analogue, in the production of the head piece, as one embodiment, a nucleic acid oligomer is firstly synthesized and then, the above-mentioned linker site (L) and the reactive functional group site (D) can be bound.

In such a case, it is preferable to put the "specific nucleic acid analogue" to which the linker site binds into the hairpin site (nucleic acid analogues oligomer) as the linking site (LS). Examples of the "specific nucleic acid analogue" may be mentioned the above-mentioned Amino C6 dT, mdC(TEG-Amino) and Uni-Link (trademark registration) Amino Modifier.

In the case of this embodiment, for example, mdC(TEG-Amino) itself corresponds to a linking site (LS) and additional sites that further bind from the base side chain are correspond to the linker site (L) and the reactive functional group site (D).

### (With regard to p and q)

As mentioned above, it is preferable that the chain length of the above-mentioned loop site is such that the first oligonucleotide chain (E) and the second oligonucleotide chain (F) form a duplex in the molecule and the head piece has a chain length forming the hairpin structure.
As one embodiment, the total number of p and q is 1 to 40.
As one embodiment, the total number of p and q is 2 to 20.
As one embodiment, the total number of p and q is 2 to 10.
As one embodiment, the total number of p and q is 2 to 7.

As one embodiment, the loop site of the present invention is constituted by (A) a nucleotide
and the following nucleic acid analogue (B41), (B42), (B43), (B44) or (B52).
(B41) d-Spacer
(B42) Amino C6 dT
(B43) mdC(TEG-Amino)
(B44) Uni-Link (trademark registration) Amino Modifier
(B52) triethylene glycol phosphoric acid ester

As one embodiment, LS is preferably B42, B43 or B44.

Also, as one embodiment, LP1 and LP2 are preferably A, B41 or B52.

As one embodiment, the loop site is a nucleic acid oligomer according to the sequences described in the following (X1) to (X9).
(X1) A-B41-B42-B41-A
(X2) A-B41-B43-B41-A
(X3) A-B41-B44-B41-A
(X4) B41-B41-B42-B41-B41
(X5) B41-B41-B43-B41-B41
(X6) B41-B41-B44-B41-B41
(X7) B52-B42-B52
(X8) B52-B43-B52
(X9) A52-A44-A52

In the above-mentioned head piece, a number of the cleavable sites is preferably within 5 and more preferably 1 to 2.

In the above-mentioned head piece, when the cleavable site is two or more, it is preferable that at least one cleavable site is in the first oligonucleotide chain or between the first oligonucleotide chain and the linker binding site and at least one cleavable site is in the second oligonucleotide chain or between the second oligonucleotide chain and the linker binding site.

As one embodiment, in the above-mentioned head piece, the position of the cleavable site is preferably within 20 bases, more preferably within 10 bases and further preferably within 3 bases, starting from the binding portion between the loop site and the first oligonucleotide chain or the second oligonucleotide chain.

Whereas this is the explanation just in case, the preferred embodiment of the "selectively cleavable site" and, for example, preferred embodiments of E, F or LP, etc., are each different concepts. That is, even if the position of the "selectively cleavable site" is included in E, the preferred embodiment of E does not necessarily apply to the "selectively cleavable site".

As one embodiment, the compound constituting the DEL of the present invention is a compound represented by the following formula (II).
A compound represented by 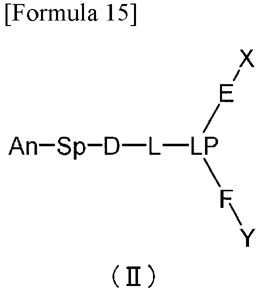 (wherein
X and Y are oligonucleotide chains,
E and F are each independently
an oligomer constituted by nucleotides or nucleic acid analogues,
provided that E and F contain base sequences, which are complementary to each other and form a duplex oligonucleotide,
LP is a loop site,
L is a linker,
D is a divalent group derived from a reactive functional group,
Sp is a bonding or a bifunctional spacer and
An is a partial structure constituted by at least one building block.),
and a compound wherein
X and Y have a sequence capable of forming a duplex at least a part thereof,
X binds to E at the 5' terminal end,
Y binds to F at the 3' terminal end and
has at least one selectively cleavable site at any of at least one site of E, F and LP.

As one embodiment, the preferred embodiments of E, F, LP, L and D in the above-mentioned compound represented by the formula (II) are the same as the preferred embodiments of E, F, LP, L and D explained with respect to the above-mentioned formula (I).

Preferred embodiments of X, Y, Sp and An will be explained separately.

### (Bifunctional spacer)

As described above, the bifunctional spacer is a spacer portion having at least two reactive groups that enables binding between the partial structure An of the compound library and the head piece. As one embodiment, the bifunctional spacer is SpD-SpL-SpX.

SpX is a reactive group that forms a covalent bond with the reactive functional group of the head piece.

SpD is a reactive group that forms a covalent bond with the partial structure An of the compound library.

SpL is a chemically inactive spacing portion

Incidentally, similar to the reactive functional group (D), the reactive group (SpX) becomes a monovalent group (-SpX) in the bifunctional spacer simple substance (the state of the reagent before binding to the head piece) and becomes a "divalent group derived from a reactive group" (-SpX-) based on the above-mentioned (-SpX) in the DEL (the state of being bound to the head piece).

Also, similarly, the reactive group (SpD) becomes a monovalent group (SpD-) in the state before binding to An and becomes a "divalent group derived from a reactive group" (-SpD-) based on the above-mentioned (SpD-) in the DEL (the state of being bound to An).

A preferred embodiment of SpX is a reactive group that forms an amino, carbonyl, amide, ester, urea or sulfonamide bond. As one embodiment, SpX is a structure of the following (SpX1), (SpX2) or (SpX3), which is a reactive group suitable when the reactive functional group of the head piece is an amino group.
(SpX1): a carboxy group, a halogenated carboxy group, an aldehyde group or a halogenated sulfonyl group
(SpX2): a carboxy group or a halogenated sulfonyl group
(SpX3): a carboxy group

A preferred embodiment of SpD is the same as the above-mentioned D.

As one embodiment, SpD is the above-mentioned (D1), (D2), (D3), (D4) or (D5).

A preferred embodiment of SpL is the following embodiments.

As one embodiment, SpL is the above-mentioned (L1), (L2), (L3), (L4) or (L5).

As one embodiment, SpL is the following (SpL1), (SpL2) or (SpL3).

(SpL1) Polyalkylene glycol, polyethylene, C1 to 20 aliphatic hydrocarbon which may be optionally replaced with a hetero atom(s), peptide, oligonucleotide or a combination thereof.

(SpL2) Polyalkylene glycol, polyethylene, C1 to 10 aliphatic hydrocarbon or peptide

(SpL3) Polyethylene glycol or polyethylene

As one embodiment, the bifunctional spacer is as follows.
(Sp1): (D4)-(SpL1)-(SpX1)
(Sp2): (D4)-(SpL2)-(SpX2)
(Sp3): (D4)-(SpL3)-(SpX3)
(Sp4): (D5)-(SpL1)-(SpX1)
(Sp5): (D5)-(SpL2)-(SpX2)
(Sp6): (D5)-(SpL3)-(SpX3)

As one embodiment, the (Sp-D-L) portion of the compound constituting the DEL is so constituted as (SpDL1), (SpDL2), (SpDL3) (SpDL4), (SpDLS), (SpDL6), (SpDL7), (SpDL8), (SpDL9) or (SpDL10).
(SpDL1): (D4)-(L1)
(SpDL2): (D5)-(L1)
(SpDL3): (D4)-(L2)
(SpDL4): (D5)-(L2)
(SpDL5): (Sp1)-(D5)-(L5)
(SpDL6): (Sp2)-(D5)-(L5)
(SpDL7): (Sp3)-(D5)-(L5)
(SpDL8): (Sp4)-(D5)-(L5)
(SpDL9): (Sp5)-(D5)-(L5)
(SpDL10): (Sp6)-(D5)-(L5)

Incidentally, in (SpDL1), (SpDL2), (SpDL3), (SpDL4), Sp means a bond.

In carrying out the present invention, it is advantageous if the head piece is synthesized by a nucleic acid synthesizer. In this practice, as mentioned above, as one embodiment, a monomer for synthesizing a nucleic acid in which the linker site (L) and the reactive functional group site (D) are bound to LS is prepared and then, a nucleic acid oligomer can be synthesized. Examples of such a monomer for synthesis of a nucleic acid may be mentioned the above-mentioned Amino C6 dT, mdC(TEG-Amino) and Uni-Link (trademark registration) Amino Modifier, etc.

On the other hand, when the above-mentioned commercially available nucleic acid synthesis monomer or a nucleic acid analogue that can be used in a nucleic acid synthesizer is used, there is a possibility that the length of the linker site is limited. In such a case, as one embodiment, by introducing an appropriate bifunctional spacer, it becomes possible to adjust the distance between the head piece and An, which is advantageous in carrying out the invention.

In the explanation of the present invention, "of C1 to C6" or "C1 to 6" in the terms of a "C1 to C6 alkyl group" or a "C1 to 6 alkyl group" means that a carbon number of which is 1 to 6. Similarly, when m and n are integers and there are descriptions of "Cm to Cn" of "Cm to n", the description means that a carbon number of which is m to n. Accordingly, a "C1 to C6 alkyl group" or a "C1 to 6 alkyl group" means an alkyl group a carbon number of which is 1 to 6 and a "C1 to C6 alkylene" or a "C1 to 6 alkylene" mean an alkylene a carbon number of which is 1 to 6.

In the present invention, the "C1 to 6 alkyl" means a linear or branched alkyl group a carbon number of which is 1 to 6. Specific examples may be mentioned methyl, ethyl, propyl, isopropyl, butyl, isobutyl, sec-butyl, tert-butyl, pentyl, hexyl, etc.

In the present invention, the "C1 to 3 alkyl" means a linear or branched alkyl group a carbon number of which is 1 to 3. Specific examples are methyl, ethyl, propyl and isopropyl.

In the present invention, the "C1 to 6 alkoxy" means a linear or branched alkoxy a carbon number of which is 1 to 6. Specific examples may be mentioned methoxy, ethoxy, propoxy, isopropoxy, butoxy, isobutoxy, sec-butoxy, tert-butoxy, pentyloxy, hexyloxy, etc.

In the present invention, The "C1 to 3 alkoxy" means a linear or branched alkoxy a carbon number of which is 1 to 3. Specific examples are methoxy, ethoxy, propoxy and isopropoxy.

In the present invention, the "hydrocarbon" means a linear, branched or cyclic saturated or unsaturated compound constituted by carbon atom and hydrogen atom alone.

In the present invention, the "aliphatic hydrocarbon" means a non-aromatic material among the hydrocarbons. The "aliphatic hydrocarbon" may be linear, branched or cyclic, or may be saturated or unsaturated. Specific examples of the structure may be mentioned alkyl, alkenyl, alkynyl, cycloalkyl or cycloalkenyl, or a structure comprising a combination thereof.

In the present invention, the "C1 to 20 aliphatic hydrocarbon" mean an aliphatic hydrocarbon having a number of the carbon atoms of 1 to 20.

In the present invention, the "C1 to 10 aliphatic hydrocarbon" means an aliphatic hydrocarbon a number of the carbon atom of 1 to 10.

In the present invention, the "C1 to 6 aliphatic hydrocarbon" means an aliphatic hydrocarbon a number of the carbon atom of 1 to 6.

In the present invention, the "aromatic hydrocarbon" means an aromatic material among the hydrocarbons.

In the present invention, the "C6 to 14 aromatic hydrocarbon" means an aromatic hydrocarbon having 6 to 14 carbon atoms. Specific examples may be mentioned benzene, naphthalene and anthracene.

In the present invention, the "C6 to 10 aromatic hydrocarbon" means an aromatic hydrocarbon having 6 to 10 carbon atoms. Specific examples are benzene or naphthalene.

The aromatic heterocyclic ring of the present invention is an aromatic heterocyclic ring having an element(s) selected solely or differently from the group consisting of nitrogen, oxygen and sulfur as a hetero atom(s) in the cyclic structure.

As one embodiment, the aromatic heterocyclic ring is a "C1 to 9 aromatic heterocyclic ring" having 1 to 9 carbon atoms and as one embodiment, the "C1 to 9 aromatic heterocyclic ring" is a "5 to 10-membered aromatic heterocyclic ring".

As one embodiment, the aromatic heterocyclic ring is a "C1 to 5 aromatic heterocyclic ring" having 1 to 5 carbon atoms and as one embodiment, the "C1 to 5 aromatic heterocyclic ring is a "5 to 10-membered aromatic heterocyclic ring".

As one embodiment, the aromatic heterocyclic ring is a "C2 to 9 aromatic heterocyclic ring" having 2 to 9 carbon atoms and as one embodiment, the "C2 to 9 aromatic heterocyclic ring is a "5 to 10-membered aromatic heterocyclic ring".

As one embodiment, the aromatic heterocyclic ring is a "C2 to 5 aromatic heterocyclic ring" having 2 to 5 carbon atoms and as one embodiment, the "C2 to 5 aromatic heterocyclic ring is a "5 to 6-membered aromatic heterocyclic ring".

The nitrogen-containing aromatic heterocyclic ring of the present invention is an aromatic heterocyclic ring having nitrogen in the cyclic structure as a hetero atom.

As one embodiment, the nitrogen-containing aromatic heterocyclic ring is a "C1 to 5 nitrogen-containing aromatic heterocyclic ring" having 1 to 5 carbon atoms and as one embodiment, the "C1 to 5 nitrogen-containing aromatic heterocyclic ring" is a "5 to 6-membered aromatic heterocyclic ring".

As one embodiment, the nitrogen-containing aromatic heterocyclic ring is a "C2 to 5 nitrogen-containing aromatic heterocyclic ring" having 2 to 5 carbon atoms and As one embodiment, the "C2 to 5 nitrogen-containing aromatic heterocyclic ring" is a "5 to 6-membered aromatic heterocyclic ring".

The non-aromatic heterocyclic ring of the present invention is a non-aromatic heterocyclic ring having an element(s) selected solely or differently from the group consisting of nitrogen, oxygen and sulfur as a hetero atom(s) in the cyclic structure.

The non-aromatic heterocyclic ring may contain a partially unsaturated bond.

As one embodiment, the non-aromatic heterocyclic ring is a "C2 to 9 non-aromatic heterocyclic ring" having 2 to 9 carbon atoms and as one embodiment, the "C2 to 9 non-aromatic heterocyclic ring" is a "5 to 10-membered non-aromatic heterocyclic ring".

In the present invention, the "trivalent group of C1 to 14" means a trivalent group derived from a compound having a number of the carbon atom of 1 to 14. As long as the effect of the present invention is achieved, the structure is not limited.

In the present invention, when there is a description that "may be replaced with a hetero atom(s)", the hetero atom means an atom other than carbon and hydrogen.

The hetero atom is preferably an oxygen atom, a nitrogen atom, a silicon atom, a phosphorus atom or a sulfur atom and more preferably an oxygen atom, a nitrogen atom or a sulfur atom.

Accordingly, for example, when propyl (-CH₂-CH₂-CH₃) is mentioned as examples of the hydrocarbon, the "propyl which may be replaced with a hetero atom(s)" is a concept containing a structure such as an ether ((-CH₂-O-CH₃) or (-O-CH₂-CH₃)) in which the methylene (-CH₂-) in the alkyl is replaced with oxygen, or an amine ((-CH₂-NH-CH₃) or (-NH-CH₂-CH₃)) in which it is replaced with nitrogen, etc.

In the present invention, when there is a description that "may have a substituent(s)", the substituent is not limited as long as it achieves the object of the present invention.

The substituent is preferably a C1 to 6 alkyl group, a C1 to 6 alkoxy group, an amino group, a hydroxy group, a nitro group, a cyano group, an oxo group or a halogen atom.

The substituent is more preferably a C1 to 6 alkyl group, a C1 to 6 alkoxy group, a fluorine atom or a chlorine atom.

In the present invention, the polypeptide and peptide mean a compound or a partial structure formed by connecting amino acids. The amino acid is a general term for organic compounds having both functional groups of an amino group and a carboxy group. The amino acid that constitutes the polypeptide and peptide of the present invention is not particularly limited and includes a modified amino acid, etc. In accordance with general usage in the field of life science, in the present invention, proline (classified as imino acid) is also included in the amino acids. The amino acid that constitutes the polypeptide and peptide of the present invention is preferably α amino acid and more preferably an "amino acid that "constitutes a protein".

The halogen atom of the present invention may be mentioned a fluorine atom, a chlorine atom, a bromine atom and an iodine atom.

A C-C, amino, ether, carbonyl, amide, ester, urea, sulfide, disulfide, sulfoxide, sulfonamide and sulfonyl bond are chemical bonds having chemical structures understood by their respective names. Those skilled in the art understand that, for example, an ether bond is a bond that can generally be represented by "-O-" and a carbonyl bond is a bond that can generally be represented by "-C(=O)-". An amino, amide and urea bond have a hydrogen atom or other substituent(s) on the nitrogen atom, but the structure on the nitrogen atom is not limited as long as it has the effect of the present invention. The above-mentioned substituent(s) on the nitrogen atom is/are preferably a C1 to 6 alkyl group(s) or a hydrogen atom(s) and more preferably a hydrogen atom(s). Also, it is needless to say, the C-C bond means a carbon-carbon bond. The C-C bond includes a single bond, a double bond and a triple bond. As one embodiment, in the steps a and/or c in the production method of the present invention, a bond appropriately selected from the above-mentioned 11 kinds is constructed. These 11 kinds of bonds are particularly basic bonding modes in organic chemistry and the reactions for constructing them are also well known to those skilled in the art. Accordingly, in designing and constructing the partial structure An of the compound library of the present invention, these 11 kinds of bonds can be appropriately combined and used for those skilled in the art.

The organic compound constituted by an element selected alone or differently from the element group consisting of H, B, C, N, O, Si, P, S, F, Cl, Br and I is an organic compound constructed by the bond of the above-mentioned 12 kinds of elements.

As one embodiment, the partial structure An of the compound library of the present invention is constructed by the above-mentioned 12 kinds of elements. These 12 kinds of elements are particularly basic elements in organic compounds and the reactions for constructing them are also well known to those skilled in the art. Accordingly, in designing and constructing the partial structure An of the compound library of the present invention, these 12 kinds of elements can be appropriately combined and used for those skilled in the art.

A low molecular weight organic compound having a substituent(s) selected alone or differently from a substituent group consisting of an aryl group, a non-aromatic cyclyl group, a heteroaryl group and a non-aromatic heterocyclyl group is a low molecular weight organic compound having a chemical structure understood by each name. The low molecular weight compound is a concept well known to those skilled in the art and examples of the preferred molecular weight of the low molecular weight compound in the present invention will be mentioned separately.

The aryl group of the present invention is preferably a C6 to 10 aryl group and more preferably a phenyl group.

The non-aromatic cyclyl group of the present invention is preferably a 5-membered to 8-membered non-aromatic cyclyl group and more preferably a 5-membered or 6-membered non-aromatic cyclyl group. The non-aromatic cyclyl group may contain a partially unsaturated bond.

The heteroaryl group and non-aromatic heterocyclyl group of the present invention are groups having an element selected alone or differently from the group consisting of nitrogen, oxygen and sulfur as a hetero atom(s) in the cyclic structure. The heteroaryl group and non-aromatic heterocyclyl group of the present invention is preferably a 5-membered to 8-membered group more preferably a 5-membered or 6-membered group and the non-aromatic heterocyclyl group may contain a partially unsaturated bond.

As one embodiment, the partial structure An of the compound library of the present invention has the above-mentioned 4 kinds of groups. These 4 kinds of groups are particularly basic partial structures in organic compounds and reactions for constructing them in the compounds are also well known to those skilled in the art. Accordingly, in designing and constructing the partial structure An of the compound library of the present invention, these 4 kinds of groups can be appropriately combined and used for those skilled in the art.

The above-mentioned preferred embodiments, that is, a compound library constructed by 11 kinds of bonds, 12 kinds of elements and/or 4 kinds of groups, has particular core value. Accordingly, those skilled in the art will understand that, in compound libraries constructed without these preferred embodiments, use thereof will be generally limited and the commercial value will be limited in many cases.

The synthesis history of An means a record of all the operations carried out until An is synthesized and in particular, it means the structure of the building blocks used until An is synthesized and the order thereof. For example, when the reaction is carried out using each different building blocks and/or different reaction conditions in two or more separate reaction vessels, an oligonucleotide chain having a previously determined sequence is ligated to the products in the respective reaction vessels, before and after the reaction, the synthesis history is imparted as sequence information of the oligonucleotide. By repeating such an operation until An is constructed, an oligonucleotide of Bn having a synthetic history of An is constructed

The split and pool synthesis is a synthetic method developed by Geisen et al., as a constructing method of combinatorial chemical for a peptide library utilizing a solid-phase synthetic method in the early days of combinatorial chemistry. The split and pool synthesis is also called a split-mix method, etc.

In accordance with the above-mentioned sequence of events, when the synthesis of a peptide library utilizing a solid-phase synthetic method is explained as an example, in the split and pool synthesis, each step of increasing the terminal of the peptide, without cutting out the sample from the solid-phase carrier to which amino acids are peptide-bonded, and after N kinds of carriers are once mixed and homogenized, they are divided into equal parts to increase the terminal by the next N kinds of amino acids.

That is, one kind of peptide chain is formed for each carrier and when all 20 kinds of natural amino acids are applied at each stage, a peptide library that is combinable with all peptides having specific lengths is to be constructed.

If this peptide library is to be screened by antigen presentation or receptor binding, an assay can be carried out by utilizing a peptide on a solid-phase carrier when an ELISA method, etc., is utilized. That is, it is not necessary to cut out the peptide of the sample from the carrier, and the carrier particles that have reacted in the assay are picked up (for example, the carrier particles that are fluorescently labeled about 0.1 mm are picked up with an optical microscope). Then, the objective peptide sequence can be determined by the peptide of the particles using an instrument analyzer (peptide analyzer, etc.), or the peptide sequence that is indirectly becomes a candidate for screening can be determined by the other combinatorial chemical identification method (for example, tag method), etc.

Further, in the production method of the present invention, it is explained as an example in the case where v kinds of structures when m is 2 and w kinds when m is 3 are synthesized by the split and pool synthesis. Incidentally, in this explanation, the steps are repeated in the order of (c) and (d).

### (m=2)

In the step of m=2, to A1-Sp-C-B1, α2 is added in the step (c) and β2 in the step (d), respectively, to produce A2-Sp-C-B2.

Here, α2 (α2 (a-v)) with v kinds of structures and v kinds of β2 β2 (a-v)) corresponding thereto are prepared and the steps (c) and (d) are each carried out for each structure, then, v kins of A2-Sp-C-B2 (A2(a)-Sp-C-B2(a), A2(b)-Sp-C-B2(b) ... A2(v)-Sp-C-B2(v): that is, A2(a-v)-Sp-C-B2(a-v)) can be obtained. In the split and pool synthesis, v kinds of A2-Sp-C-B2 are mixed and then divided into a number of w. Division means, most specifically, it is subdivided into reaction vessels with a number of w.

### (m=3)

In the step of m=3, to A2-Sp-C-B2, α3 is added in the step (c) and β3 in the step (d), respectively, to produce A3-Sp-C-B3.

Here, α3 (α3 (a-w)) with w kinds of structures and w kinds of β3 (β2 (a-w)) corresponding thereto are prepared and to (A2 (a-v)-Sp-C-B2 (a-v) mixture) with a number of w, the steps (c) and (d) are each carried out. Then, through the steps of n=2 and 3, (v×w) kinds of A3-Sp-C-B3 is to be efficiently synthesized by (v+w) times of syntheses.

### (Biological evaluation)

When the obtained products with a number of w are mixed, then a mixture of (v×w) kinds of A3-Sp-C-B3 compound library is obtained. For example, if a binding test of a drug receptor is carried out to this mixture, screening of (v×w) kinds of compounds can be carried out one time. By washing away the compounds that did not bind to the drug receptor, only the bound compounds can be isolated. In a DEL like the present invention, the DNA of the isolated A3-Sp-C-B3 compound is amplified to an amount that can be sequenced and the structure of A3 can be grasped from the sequence information.

Incidentally, compound library, building blocks, split and pool, etc., are terms well known to those skilled in the art in fields such as combinatorial chemistry, etc. and can be carried out in a timely manner with reference to the following Literature, etc.
(1) Takashi Takahashi, Takayuki Doi "Combinatorial chemistry", Journal of The Society of Synthetic Organic Chemistry, 2002, vol. 60, pp. 426-433
(2) Combinatorial Chemistry Edited by Study Group "Combinatorial Chemistry", Kagakudojin Publishing

A DNA-encoded library (or DEL) is a compound library comprising a group of DNA, or compounds (DNA-encoded compound) labeled with oligonucleotides having substantially the same function as DNA. By the split and pool synthesis as mentioned above, the structure or synthesis history of each compound is imparted to the labeled DNA as sequence information. From such characteristics, the DNA-encoded library is screened in the form of a mixture of 10² to 10²⁰ kinds of compounds and the DNA sequences contained in the obtained compounds are identified by techniques known in the art (for example, use of next-generation sequencers and/or use of microarrays), it is possible to identify the structure of the compound. As one embodiment of the above-mentioned screening method, a method of contacting a target such as a protein, etc., with a DNA-encoded library and selecting a compound bound to the target can be selected.

"Biological target" is a term well known to those skilled in the art and as one embodiment, in the present invention, a "biological target" is a biological substance group that can be a target in the development of a drug, etc., represented by a medical and agrochemical drug and for example, an enzyme (for example, kinase, phosphatase, methylase, demethylase, protease and DNA repair enzyme), a protein involved in protein:protein interaction (for example, ligand of receptor), receptor target (for example, GPCR), ion channel, cell, bacteria, virus, parasite DNA, RNA, prion or sugar is contained.

"Biological activity evaluation" is a term well known to those skilled in the art and as one embodiment, in the present invention, the "biological activity evaluation" is to evaluate the presence or absence, or strength of the biological activity (for example, an ability to bind to a biological target, inhibitory function of enzyme activity, promotion function of enzyme activity, etc.) possessed by a compound. As specific examples of the biological activity evaluation, the above-mentioned Patent Documents 2 and 3, Non-Patent Documents 1 to 6, etc., can be also referred to.

"Functionality evaluation" is a term well known to those skilled in the art and as one embodiment, in the present invention, the "functionality evaluation" is to evaluate the presence or absence, or strength of a specific function (for example, binding ability, biological activity, luminescence property, etc.) possessed by a compound.

The present invention provides a plurality of methods having several advantages with respect to DEL and a method for producing DEL by using a DNA strand having a cleavable site. Forms 1 to 7 will be described in detail below.

### Form 1

The present invention provides a DEL using the above-mentioned "hairpin type head piece having a cleavable site".

As exemplified in Fig. 1, in Form 1, a head piece which contains a first oligonucleotide chain containing a cleavable site in a DNA strand, a loop site and a second oligonucleotide chain is used as a raw material, linking of building blocks and double strand ligation of an oligonucletide tag corresponding to the building blocks are repeated (three times in Fig. 1) and further, if desired, a double strand ligation of an oligonucletide tag containing a primer region is carried out whereby production of a DEL is achieved.

As exemplified in Fig. 2, in Form 1, to a DEL containing the cleavable site in the first oligonucleotide chain of the head piece, the cleavable site is cleaved using a cleaving means such as an enzyme and induced to a double strand oligonucleotide that is not bound at the loop site, whereby PCR can be carried out with high efficiency.

### (With regard to Form 2)

As exemplified in Fig. 3, in the DEL using the "hairpin type head piece having a cleavable site", the cleavable site may exist in the second oligonucleotide chain. The characteristics of Form 2 are the same as those of Form 1 except for the cleavable site.

### (With regard to Form 3)

As exemplified in Fig. 4, in the DEL using the "hairpin type head piece having a cleavable site", the cleavable site may exist in both the first and second oligonucleotide chains. In this embodiments, the loop sites are cleaved by both of the oligonucleotide chains, whereby it is expected to further improve PCR efficiency.

### (With regard to Form 4)

As exemplified in Fig. 5, in the present invention, the cleavable site may exist in both the first oligonucleotide chain (E) and the second oligonucleotide chain (F) and further the structures of the cleavable sites may be different. In such a case, by utilizing the difference in characteristics of the two (or more) cleavable sites, the cleaving site can be controlled.

For example, deoxyuridine may be used as the cleavable site of the first oligonucleotide chain (E) and deoxyinosine may be used as the cleavable site of the second oligonucleotide chain (F).

In this case, when the USER enzyme is used, deoxyuridine of the first oligonucleotide chain (E) can be selectively cleaved.

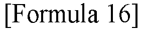

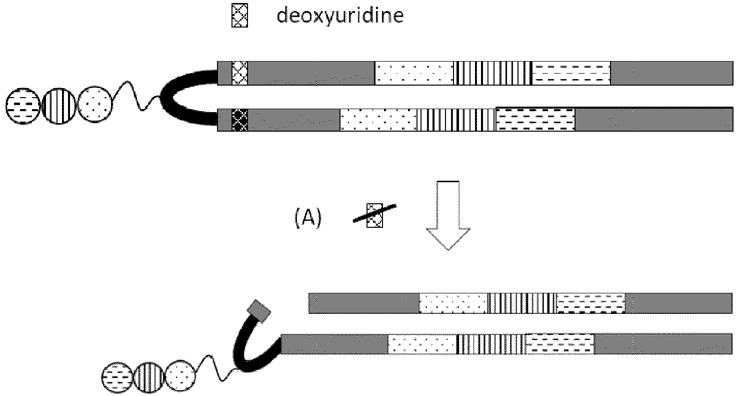

On the other hand, when the alkyladenine DNA glycosylase and endonuclease VIII are used, in the second oligonucleotide chain (F), the cleavage site originating from deoxyinosine can be selectively cleaved.

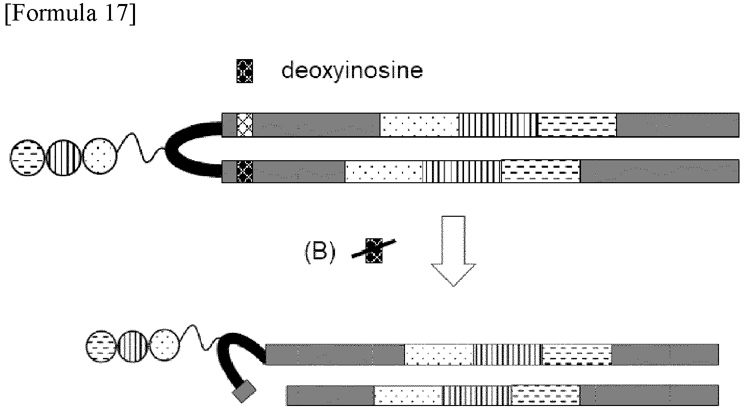

Like this, by selecting the cleavage site as desired, a wider range of modifications of the DEL becomes possible and a wider means can be applied to the evaluation thereafter.
can be expected.

### (With regard to Form 5)

As exemplified in Fig. 6, in the present invention, the cleavable site may be provided at the DNA tag portion (for example, oligonucleotide chain (Y)). The cleavable site is provided near the terminal of the DNA tag and if desired, the site is cleaved to form a new sticky end.

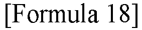

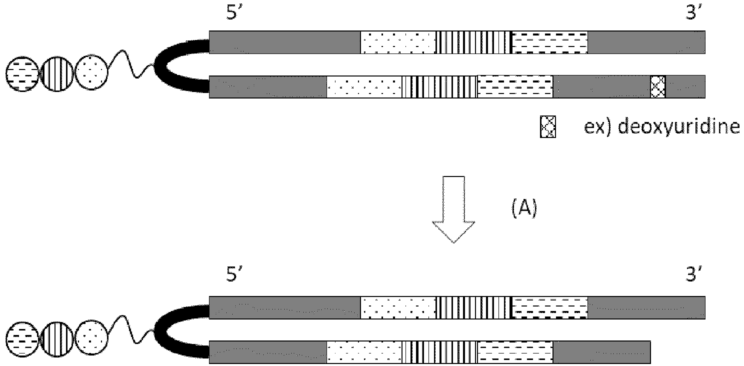

The sticky end is utilized as a sticky terminal and a desired nucleic acid sequence, for example, UMIs (a specific molecule identification sequence), etc., can be ligated.

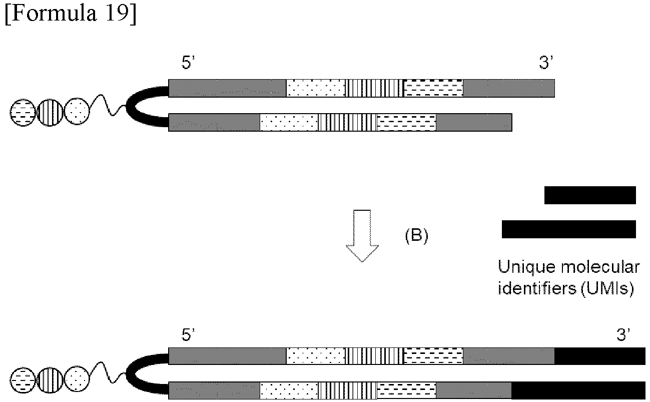

After the biological evaluation, to the selected DEL compound, the UMIs region is imparted as mentioned above and by subjecting to DNA sequencing, it is possible to carry out the analysis in which amplification bias by PCR is reduced

Like this, in the present invention, by having a selectively cleavable site in the nucleic acid sequence, it is possible to impart unconventional properties in the aspect of production and use of the DEL compound.

Here, UMIs (a specific molecule identification sequence) is a molecular identifier that gives individual DNA sequence to each DNA molecule by imparting it to the DNA contained in a certain sample (refer to Nature Method, 2012, vol. 9, pp. 72-74). By providing such a molecular identifier before amplification of PCR, when a number of DNA molecules having a specific sequence in the sample is quantified, it is possible to identify duplication of PCR (sequence derived from the same molecule) and quantification in reducing PCR amplification bias is possible.

### (With regard to Form 6)

As exemplified in Fig. 7, in the present invention, a cleavable site can be used in combination with a modifying group or a functional molecule and for example, it is possible to prepare a DEL in which a hairpin-stranded DNA is converted into a single-stranded DNA.
In accordance with Fig. 7, a DEL compound using a head piece having a cleavable site in an E portion is mentioned as an example.
(Step A) A double-stranded oligonucleotide chain having a modifying group which is removable with a solid-phase-carrier (for example, biotin) at the 3' terminal is ligated to the synthesized DEL compound.
(Step B) The cleavable site is cleaved.
(Step C) A treatment according to the function of the modifying group is applied. For example, in the case of biotin, by using streptavidin beads, etc., having biotin affinity, the oligonucleotide chain to which biotin is bound is selectively removed from the system. According to it, it is possible to obtain a DEL having a single-stranded DNA.

Here, the functional molecule is a molecule having a specific chemical or biological function (for example, solubility, photoreactivity, substrate-specific reactivity, target protein degradation-inducing property) and by imparting it to a DEL, it is possible to carry out evaluation or purification of the DEL depending on the function.

Here, biotin means all biotins that bind to avidin and includes not only vitamin B₇ but also, for example, desthiobiotin.

As exemplified in Fig. 8, a DEL having a single-stranded DNA is formed by forming a double strand with a modified oligonucleotide (for example, a cross linker-modified DNA such as photoreactive cross linker, etc.) having a desired functional site, whereby it is possible to impart a new function.

### (With regard to Form 7)

As exemplified in Fig. 9, in the present invention, a cross linker can be introduced by utilizing a cleavable site.

In accordance with Fig. 9, a DEL compound using a head piece having a cleavable site in an E portion is mentioned as an example.
(Step A) A cleavable site is cleaved with respect to the synthesized DEL compound.
(Step B) A modified primer (for example, a cross linker-modified primer such as a photo-reactive cross linker, etc.) having a desired functional site is imparted.
(Step C) The imparted primer is elongated to synthesize a cross linker-modified double-stranded DEL compound.

In the scene of DEL evaluation, when the building block compound (library low molecular weight compound) binds to the target protein, the cross linker-modified double-stranded DEL compound can further bind the cross-link structure to the target protein, whereby detection sensitivity can be markedly improved (refer to Non-Patent Documents 5 and 6, etc.). In practice of the DEL technique for evaluating a large number of library compounds, it is extremely useful to enhance the affinity of the library compounds and to improve the detection sensitivity.

The present invention is to provide a novel and highly efficient method for producing a cross linker-modified double-stranded DEL compound, which is extremely useful.

Hereinafter, Example are shown and the present invention will be described in more detail, but the present invention is not limited to these Example.

Incidentally, various kinds of nucleic acids of sequences in Examples can be prepared, for example, according to a conventional method by an automated polynucleotide synthesizer. Examples of the automated polynucleotide synthesizer may be mentioned nS-8II (manufactured by GeneDesign, Inc.), etc. In addition, for the preparation of the nucleic acid, consignment synthesis, contract labs, etc., can be also used. As the contract labs well known to those skilled in the art, there may be mentioned GeneDesign, Inc., LGC Biosearch Technologies, etc. In general, these contract labs prepare nucleic acids of the sequence specified by the consignor and deliver them to the consignor under a confidentiality agreement.

### Example 1

### [Verification of cleavage reaction by USER (registered trademark) enzyme of partial structure of hairpin type DEL containing deoxyuridine]

The compound of the sequence shown in Table 1 was prepared using an automated polynucleotide synthesizer nS-8II (manufactured by GeneDesign, Inc.). Incidentally, in the sequence notation in Table 1, as is obvious to those skilled in the art, each sequence unit is bound by a phosphoric acid diester bond, "A" means deoxyadenosine, "T" means thymidine, "G" means deoxyguanosine, "C" means deoxycytidine, "(dU) " means deoxyuridine, "(p) " means phosphoric acid and "(aminoC6-dT)" means the modified nucleic acid represented by the following formula (1) 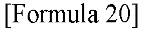 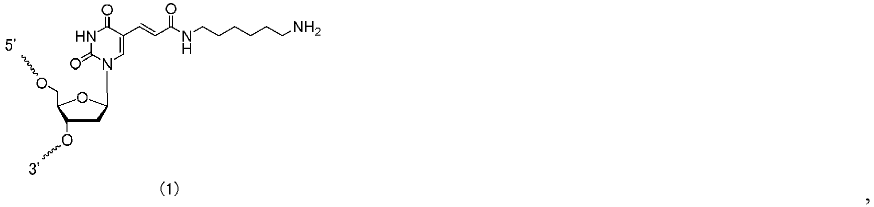
"(amino-NC6-dT)" means the modified nucleic acid represented by the following formula (2) 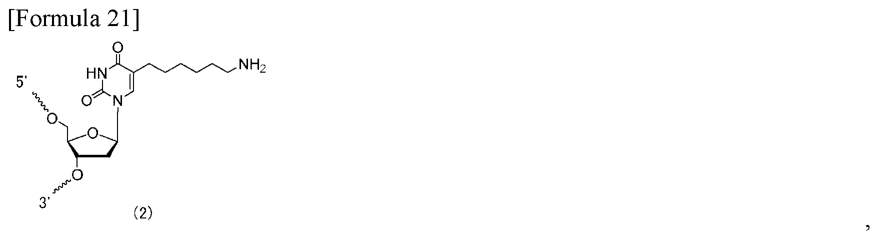
"(dSpacer)" means the group represented by the following formula (3) 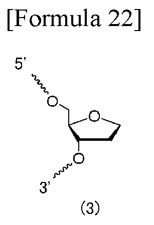
and "(aminoC7)" means the group represented by the following formula (4) 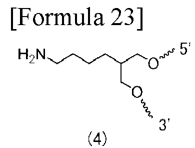
Also, amino-NC6-dT was introduced using the nucleic acid synthetic reagent of the following formula (5) 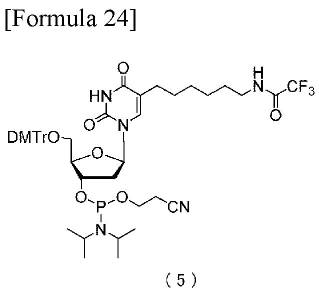 synthesized according to the method described in (Journal of the American Chemical Society, 1993, vol. 115, pp. 7128-7134).

In Table 1, "No." in the left column represents SEQ ID NO: and "Seq." in the right column represents a sequence. The left side of the sequence represents the 5' side and the right side represents 3' side. Also, the names of the compounds corresponding to each SEQ ID NO: (No.) are as follows.

| | |
|---|---|
| No. 1: U-DEL1-sh | No. 2: U-DEL2-sh |
| No. 3: U-DEL3-sh | No. 4: U-DEL4-sh |
| No. 5: U-DEL5-HP | No. 6: U-DEL6-HP |
| No. 7: U-DEL7-HP | No. 8: U-DEL8-HP |
| No. 9: U-DEL9-HP | No. 10: U-DEL10-HP |

**[Table 1]**

| No. | Seq. |
|---|---|
| 1 | CATCGATTTGGGAGTCA(dU)T(amino-C6-dT)TTTGACTCCCAAATC6ATGTG |
| 2 | CATCGATTTGGGAGTCATT(amino-C6-dT)T(dU)TGACTCCCAAATCGATGTG |
| 3 | CATCGATTTGGGAGTCATT(amino-C6-dT)TTTGACTCCCAAATCGA(dU)GTG |
| 4 | CATCGATTTGGGAGTCA(dU)T(amino-C6-dT)T(dU)TGACTCCCAAATCGATGTG |
| 5 | (p)GAGTCATT(amino-NC6-dT)T(dU)TGACTCCC |
| 6 | (p)GAGTCA(dU)T(amino-NC6-dT)T(dU)TGACTCCC |
| 7 | (p)GAGTCA(dU)T(dSpacer)(dSpacer)(AminoC7)(dSpacer)(dSpacer)TTTGACTCCC |
| 8 | (p)GAGTCATT(dSpacer)(dSpacer)(AminoC7)(dSpacer)(dSpacer)T(dU)TGACTCCC |
| 9 | (p)GAGTCAA(dSpacer)(dSpacer)(AminoC7)(dSpacer)(dSpacer)(dU)TGACTCCC |
| 10 | (p)GAGTCAT(dSpacer)(AminoC7)(dSpacer)(dU)TGACTCCC |

A 0.1 mM aqueous solution of each of the compounds having the sequences shown in Table 1 was prepared and investigation of the cleavage reaction by a USER (Registered trademark) enzyme was carried out by the following procedure.

To a PCR tube were added 1 µL of 0.1 mM aqueous solution of the compound of the sequence shown in Table 1; 10 µL of CutSmart (Registered trademark) Buffer (available from New England BioLabs, Catalog number: B7204S) and 79 µL of deionized water. To the solution was added 10 µL of a USER (Registered trademark) enzyme (available from New England BioLabs, Catalog number: M5505S) and incubation of the obtained solution was started at 37°C.

Each reaction solution was sampled with each 20 µL after starting the incubation, 1 hour and 3 hours lapsed, respectively. U-DEL1-sh, U-DEL5-HP, U-DEL6-HP, U-DEL7-HP, U-DEL8-HP, U-DEL9-HP and U-DEL10-HP were sampled with each 20 µL after 20 hours lapsed. U-DEL8-HP and U-DEL9-HP were further incubated at 90°C for 1 hour and sampled with each 20 µL, respectively.

Among the sampled solutions, U-DEL1-sh, U-DEL2-sh, U-DEL3-sh and U-DEL4-sh were analyzed by Analytical condition 1 shown below and U-DEL5-HP, U-DEL6-HP, U-DEL7-HP, U-DEL8-HP, U-DEL9-HP and U-DEL10-HP were analyzed by Analytical condition 2 shown below.

Analytical condition 1:
Device: maXis (manufactured by Bruker), UltiMate 3000 (manufactured by Dionex)
Column: ACQUITY UPLC Oligonucleotide BEH C18 Column (130Å, 1.7 µm, 2.1×50 mm)
Column temperature: 50°C
Solvent:
   Solution A: water (0.75% v/v hexafluoroisopropanol; 0.038% v/v triethylamine; 5 µM ethylenediamine tetraacetic acid)
   Solution B: 90% v/v methanol aqueous solution (0.75% v/v hexafluoroisopropanol; 0.038% v/v triethylamine; 5 µM ethylenediamine tetraacetic acid)
Gradient conditions:
   By fixing the flow rate of 0.36 mL/min and the mixing ratio of Solution A and Solution B to 95/5 (v/v), the measurement was started and after 0.56 minute, the mixing ratio of Solution A and Solution B was linearly changed to 40/60 (v/v) in 5.5 minutes.
   Detection wavelength: 260 nm

Analytical condition 2:
Device: Waters ACQUITY UPLC/SQ Detector
Column: ACQUITY UPLC Oligonucleotide BEH C18 Column (130Å, 1.7 µm, 2.1×50 mm)
Column temperature: 50°C
Solvent:
   Solution A: water (0.75% v/v hexafluoroisopropanol; 0.038% v/v triethylamine; 5 µM ethylenediamine tetraacetic acid)
   Solution B: 90% v/v methanol aqueous solution (0.75% v/v hexafluoroisopropanol; 0.038% v/v triethylamine; 5 µM ethylenediamine tetraacetic acid)
Gradient conditions:
   By fixing the flow rate of 0.36 mL/min and the mixing ratio of Solution A and Solution B to 95/5 (v/v), the measurement was started and after 0.56 minute, the mixing ratio of Solution A and Solution B was linearly changed to 40/60 (v/v) in 5.5 minutes.
   Detection wavelength: 260 nm

The sequences and the expected molecular weights of the products (abasic product of deoxyuridine portion and cleaved fragments) assumed in each reaction solution and the molecular weight observed in each reaction solution are shown in Table 2 and Table 3. Incidentally, in Table 2 and Table 3, the notation of each column is as follows.

"Entry" (leftmost):
It indicates the experimental number and the substrates corresponding to each experimental number (Entry) are as follows.

| | |
|---|---|
| Entry. 1: U-DEL1-sh | Entry. 2: U-DEL2-sh |
| Entry. 3: U-DEL3-sh | Entry. 4: U-DEL4-sh |
| Entry. 5: U-DEL5-HP | Entry. 6: U-DEL6-HP |
| Entry. 7: U-DEL7-HP | Entry. 8: U-DEL8-HP |
| Entry. 9: U-DEL9-HP | Entry. 10: U-DEL10-HP |

"No." (second from the left):
It indicates the sequence number. Incidentally, among the respective SEQ ID NOs (No.), Nos. 1, 2, 3, 4, 5, 6, 7, 8, 9 and 10 are substrates of each reaction solution, Nos. 11, 14, 17, 20, 22, 25, 29, 31, 33 and 35 are dibasic products of the deoxyuridine portion of each substrate and the remaining SEQ ID NOs are fragments of each substrate cleaved.

"Seq. " (third from the left):
It indicates the sequence, the left side represents the 5' side and the right side represents the 3' side.

Incidentally, in the sequence notation, "(B)" means the group (debasic site) represented by the following formula (6) 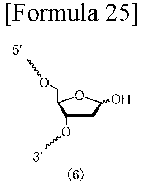 and other notations are the same as in Table 1.

"Expected MW." (fourth from the left):
It indicates the numerical value of the expected molecular weight (Da) of each sequence.

"Observed MW." (rightmost):
It indicates the numerical value of the observed molecular weight (Da) identified as each sequence. Incidentally, "-" notation indicates that it has not been detected.

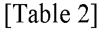

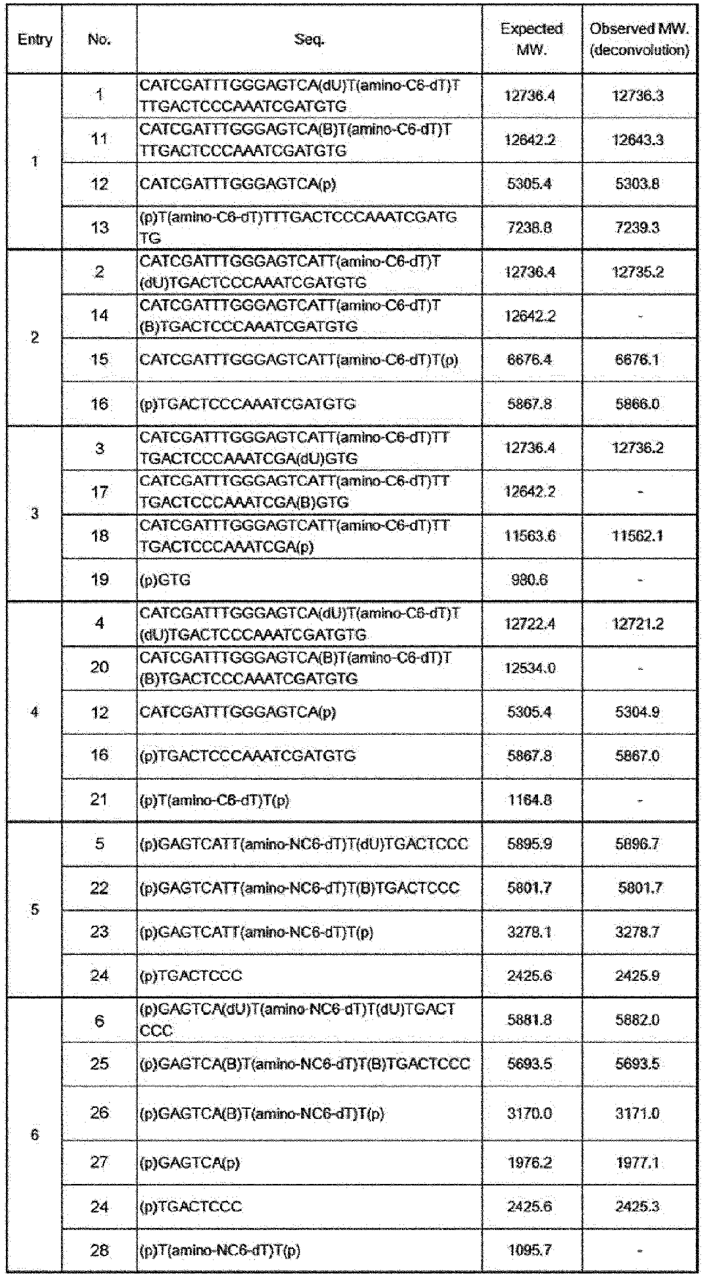

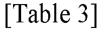

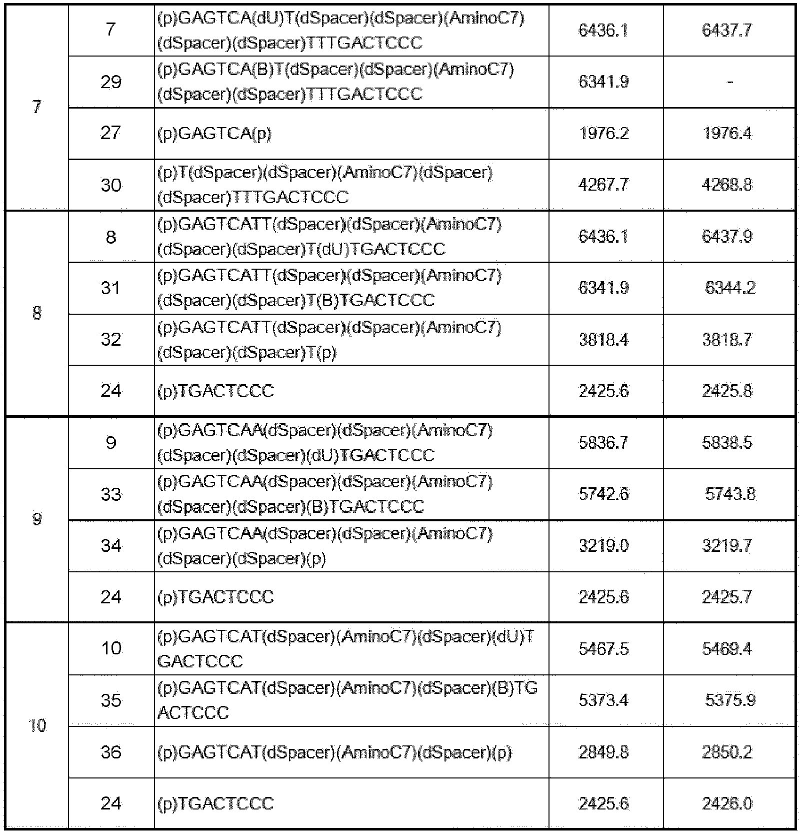

From the area ratio of the peak corresponding to each sequence detected, the the conversion rate of debasic reaction and of the cleavage reaction were calculated. In the debasic reaction, 99% or more was converted in all the substrate at the stage of 37°C and 1 hour (the peak of the substrate is less than 1% and the remaining peak is the debasic product and the cleaved fragment alone).

Also, a graph showing the conversion rate of the cleavage reaction is shown in Fig. 10. As shown in the graph, in all the substrates except for U-DEL8-HP and U-DEL9-HP, the cleavage reaction proceeded 95% or more by 20 hours at 37°C and in U-DEL8-HP and U-DEL9-HP, by adding the incubation of 1 hour at 90°C, 100% of the cleavage reaction was completed.

From the above results, at the partial structure of the hairpin type DEL containing various kinds of deoxyuridines, it was shown that, at deoxyuridine site, a debasic reaction by the USER (Registered trademark) enzyme and subsequently a cleavage reaction proceeded.

### Example 2

### [Comparison of PCR efficiency between conventional type hairpin DEL and cleavable hairpin DEL (hairpin type DEL containing deoxyuridine)]

As in the schematic diagram shown in Fig. 11, the compounds (hairpin DEL) having the sequence shown in Table 4 were synthesized by the following procedure. Incidentally, in the sequence notation in Table 4, "S" means the group represented by the following formula (7) 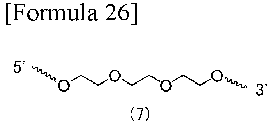 and other notations are the same as in Table 1.
The names of the compounds corresponding to each SEQ ID NO: (No.) are as follows.

| | |
|---|---|
| No. 37: U-DEL1 | No. 38: U-DEL2 |
| No. 39: U-DEL4 | No. 40: U-DEL7 |
| No. 41: U-DEL8 | No. 42: U-DEL9 |
| No. 43: U-DEL10 | No. 44: H-DEL |

**[Table 4]**

| No. | Seq. | Expected MW. | Observed MW. (deconvolution) |
|---|---|---|---|
| 37 | GCAGGTGAAGCTTGTCTGAAGACTGCTTCTGGAAC CACCATCGCACTTCCTGACCACATCGATTTGGGAGT CA(dU)T(amino-C6-dT)TTTGACTCCCAAATCGATGTG GTCAGGAAGTGCGATGGTGGTTCCAGAAGCAGTCT TCAGACAAGCTTCACCTGC | 46707.3 | 46702.9 |
| 38 | GCAGGTGAAGCTTGTCTGAAGACTGCTTCTGGAAC CACCATCGCACTTCCTGACCACATCGATTTGGGAGT CATT(amino-C6-dT)T(dU)TGACTCCCAAATCGATGTG GTCAGGAAGTGCGATGGTGGTTCCAGAAGCAGTCT TCAGACAAGCTTCACCTGC | 46707.3 | 46711.8 |
| 39 | GCAGGTGAAGCTTGTCTGAAGACTGCTTCTGGAAC CACCATCGCACTTCCTGACCACATCGATTTGGGAGT CA(dU)T(amino-C6-dT)T(dU)TGACTCCCAAATCGATGT GGTCAGGAAGTGCGATGGTGGTTCCAGAAGCAGTC TTCAGACAAGCTTCACCTGC | 46693.2 | 46695.5 |
| 40 | G CAGG TG AAG CTTG TCTG AAG ACTG CTTCTG G AAC CACCATCG CACTTCCTG ACCACATCG ATTTG G G AG T CA (dU)T (dSpacer) (dSpacer) (AminoC7) (dSpacer) (dSpacer)TTTG ACTCCCAAATCG ATG TG G TCAG G AAG TG CG ATG G TG G TTCCAG AAG CAG TCTTCAG ACAAG CTTCACCTG C | 47178.4 | 47184.8 |
| 41 | GCAGGTGAAGCTTGTCTGAAGACTGCTTCTGGAAC CACCATCGCACTTCCTGACCACATCGATTTGGGAGT CATT(dSpacer)(dSpacer)(AminoC7)(dSpacer)(dSpacer)T (dU)TGACTCCCAAATCGATGTGGTCAGGAAGTGCGA TGGTGGTTCCAGAAGCAGTCTTCAGACAAGCTTCAC CTGC | 47178.4 | 47189.7 |
| 42 | GCAGGTGAAGCTTGTCTGAAGACTGCTTCTGGAAC CACCATCGCACTTCCTGACCACATCGATTTGGGAGT CAA(dSpacer)(dSpacer)(AminoC7)(dSpacer) (dSpacer)(dU)TGACTCCCAAATCGATGTGGTCAGGAA GTGCGATGGTGGTTCCAGAAGCAGTCTTCAGACAA GCTTCACCTGC | 46579.1 | 46592.0 |
| 43 | GCAGGTGAAGCTTGTCTGAAGACTGCTTCTGGAAC CACCATCGCACTTCCTGACCACATCGATTTGGGAGT CAT(dSpacer)(AminoC7)(dSpacer)(dU)TGACTCCCAAA TCGATGTGGTCAGGAAGTGCGATGGTGGTTCCAGA AGCAGTCTTCAGACAAGCTTCACCTGC | 46209.9 | 46220.0 |
| 44 | GCAGGTGAAGCTTGTCTGAAGACTGCTTCTGGAAC CACCATCGCACTTCCTGACCACATCGATTTGGGAGT CAS(AminoC7)STGACTCCCAAATCGATGTGGTCAGG AAGTGCGATGGTGGTTCCAGAAGCAGTCTTCAGAC AAGCTTCACCTGC | 45679.6 | 45666.6 |

Incidentally, the names of the compounds which are raw material head piece for synthesizing each hairpin DEL are each as follows.
Hairpin DEL: Raw material of head piece
U-DEL1: U-DEL1-HP
U-DEL2: U-DEL2-HP
U-DEL4: U-DEL4-HP
U-DEL7: U-DEL7-HP
U-DEL8: U-DEL8-HP
U-DEL9: U-DEL9-HP
U-DEL10: U-DEL10-H
H-DEL: H-DEL-HP
Further, SEQ ID NO: "No." of U-DEL1-HP, U-DEL2-HP, U-DEL4-HP and the sequence "Seq" of H-DEL-HP are as shown in Table 5.

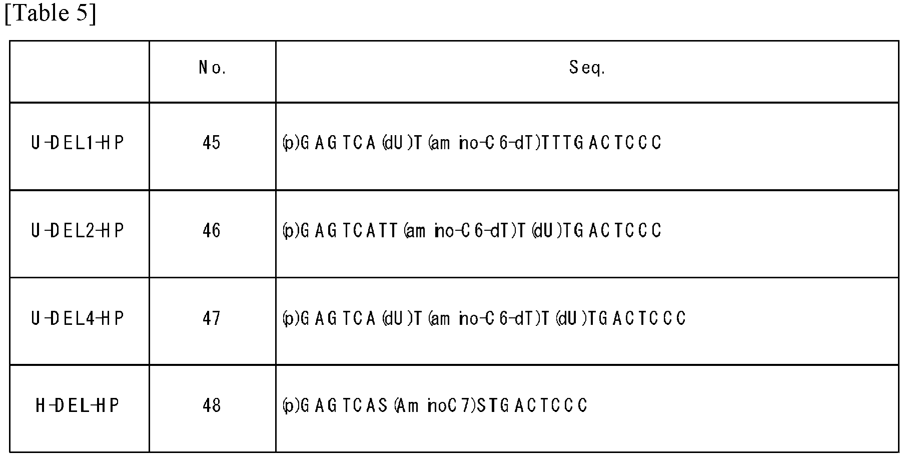

The raw material head piece shown in Table 5 were prepared using an automated polynucleotide synthesizer nS-8II (manufactured by GeneDesign, Inc.) similarly to Example 1.

To a PCR tube were added 2.0 µL of 1 mM aqueous solution of various kinds of the raw material head piece; 2.4 µL of 1 mM aqueous solution of Pr_TAG (it was prepared by annealing Pr_TAG_a and Pr_TAG_b synthesized in the same manner as in Example 1, the sequence is shown in Table 6); 0.8 µL of 10X ligase buffer (500 mM Tris hydrochloride, pH 7.5; 500 mM sodium chloride; 100 mM magnesium chloride; 100 mM dithiothreitol; and 20 mM adenosine triphosphate) and 2.0 µL of deionized water. To the solution was added 0.8 µL of a 10-fold diluted aqueous solution of T4DNA ligase (available from Thermo Fisher, Catalog number: EL0013) and the obtained solution was incubated at 16°C for 24 hours. Incidentally, the sequence notation in Table 6 is the same as in Table 1. Also, the names of the compounds corresponding to each SEQ ID NO: (No.) are as follows.

| | |
|---|---|
| No. 49: Pr_TAG_a | No. 50: Pr_TAG_b |

**[Table 6]**

| No. | Seq. |
|---|---|
| 49 | (p)GACTGCTTCTGGAACCACCATCGCACTTCCTGACCACATC6ATTTGG |
| 50 | (p)AAATCGATGTGGTCAGGAAGTGCGATGGTGGTTCCAGAAGCAGTCTT |

The reaction solution was treated with 0.8 µL of a 5 M aqueous sodium chloride solution and 17.6 µL of cooled (-20°C) ethanol and allowed to stand at -78°C for 2 hours. After centrifugation, the supernatant was removed and the obtained pellets were air-dried. To each pellet was added 2.0 µL of deionized water to prepare a solution.

To the obtained each solution were added 2.4 µL of 1 mM aqueous solution of CP (it was prepared by annealing CP_a and CP_b synthesized in the same manner as in Example 1, the sequences are shown in Table 7); 0.8 µL of 10X ligase buffer (500 mM Tris hydrochloride, pH 7.5; 500 mM sodium chloride; 100 mM magnesium chloride; 100 mM dithiothreitol; and 20 mM adenosine triphosphate) and 2.0 µL of deionized water. To the solution was added 0.8 µL of a 10-fold diluted aqueous solution of T4DNA ligase (available from Thermo Fisher, Catalog number: EL0013) and the obtained solution was incubated at 16°C for 24 hours. Incidentally, the sequence notation in Table 7 is the same as in Table 1. Also, the names of the compounds corresponding to each SEQ ID NO: (No.) are as follows.

| | |
|---|---|
| No. 51: CP_a | No. 52: CP_b |

**[Table 7]**

| No. | Seq. |
|---|---|
| 51 | GCAGGTGAAGCTTGTCTGAA |
| 52 | (p)CAGACAAGCTTCACCTGC |

The reaction solution was treated with 0.8 µL of 5 M aqueous sodium chloride solution and 17.6 µL of cooled (-20°C) ethanol and allowed to stand at -78°C for 2 hours. After centrifugation, the supernatant was removed and the obtained pellets were air-dried. To the pellets was added 10 µL of deionized water to prepare a solution.

Of the obtained solution, 1.0 µL was sampled and after diluting with deionized water, mass spectrometry by ESI-MS was carried out under the conditions of Analytical condition 2 of Example 1 to identify the target product (the expected molecular weight and the observed molecular weight of each sequence are shown in Table 4). After lyophilizing the rest of the solution, deionized water was added to prepare the solution to 20 µM.

Among the eight kinds of the hairpin type DEL obtained as mentioned above, H-DEL is a conventional type hairpin DEL and the remaining seven kinds are cleavable hairpin DELs containing deoxyuridine. Real-time PCR analysis was carried out to compare the PCR efficiency of various kinds of hairpin type DELs before treatment with USER (Registered trademark) enzyme and the PCR efficiency after treatment. Also, as the double-stranded DEL to be compared, DS-DEL (it was prepared by annealing the compounds of sequences No. 47 and No. 48) shown in Table 7 was used. Incidentally, in the sequence notation in Table 8, "(amino-C6-L)" means the group represented by the following formula (8) 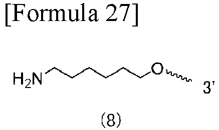 and other notations are the same as in Table 1.

**[Table 8]**

| | No. | Seq. |
|---|---|---|
| DS-DEL | 53 | (amino-C6-L)TGACTCCCAAATCGATGTGGTCAGGAAGTGCGATGGTGGTTCCAGAAGCAGTC TTCAGACAAGCTTCACCTGC |
| | 54 | GCAGGTGAAGCTTGTCTGAAGACTGCTTCTGGAACCACCATCGCACTTCCTGACCACATCGA TTTGGGAGTCA |

### <Treatment step with USER (Registered trademark) enzyme>

Treatment of eight kinds of hairpin DELs and double-stranded DEL (DS-DEL) with USER (Registered trademark) enzyme was carried out by the following procedure.

To a PCR tube were added 1 µL of various kinds of 20 µM DEL aqueous solution; 1 µL of CutSmart (Registered trademark) Buffer (available from New England BioLabs, Catalog number: B7204S) and 7 µL of deionized water. To the solution was added 1 µL of USER (Registered trademark) enzyme (available from New England BioLabs, Catalog number: M5505S) and the obtained solution was incubated at 37°C for 1 hour.

### <Preparation of DEL samples>

Samples of various kinds of DELs before treatment with USER (Registered trademark) enzyme and the reaction solutions after treatment were each diluted with deionized water to prepare DEL samples with 0.05 pM, 0.5 pM and 5 pM.

### <Measurement of Ct value by real-time PCR>

The Ct value of various kinds of DEL samples obtained as mentioned above was measured by real-time PCR and the PCR efficiencies were compared. The conditions are as mentioned below and the results are shown in Fig. 12. Incidentally, the Ct value is the number of cycles in which the fluorescent signal generated by amplification of DNA reaches an arbitrary threshold value in real-time PCR. That is, when the initial number of DNA molecules is the same, the higher the PCR efficiency, the lower the Ct value.

Device: 7500 real-time PCR system (manufactured by Applied Biosystems) Plate: MicroAmp 96-Well plate (manufactured by Applied Biosystems, Catalog number: N8010560)

### PCR the reaction solution:

| | |
|---|---|
| ·TB Green Premix Ex taqII (available from Takara Bio Inc., Catalog number: RR820) | : 10 µL |
| ·Forward primer (Table 9, SEQ ID NO:55) | : 0.80 µL |
| ·Reverse primer (Table 9, SEQ ID NO:56) | : 0.80 µL |
| ·ROX Refference DyeII (available from Takara Bio Inc., Catalog number: RR39LR) | : 0.40 µL |
| ·Various kinds of aqueous solutions (0.05 pM, 0.5 pM, 5 pM)^{∗}1 of DEL samples | : 2.0 µL |
| ·Deionized water | : 6.0 µL |

| | |
|---|---|
| ^{∗}1: The number of moles of the DEL sample is 0.1 amol, 1 amol and 10 amol. | |

### Temperature conditions:

·After holding at 95°C for 2 minutes, the following cycle was repeated for 35 cycles.
·95°C, 5 seconds
·52°C, 30 seconds
·72°C, 30 seconds

**[Table 9]**

| No. | Seq. |
|---|---|
| 55 | TGACTCCCAAATCGA |
| 56 | GCAGGTGAAGCTTGTC |

Incidentally, the sequence notations in Table 9 are the same as in Table 1.

As shown in Fig. 12, in the conventional type hairpin DEL (H-DEL), the Ct value does not change before and after the USER (Registered trademark) enzyme treatment, but in the cleavable hairpin DEL (U-DEL1, U-DEL2, U-DEL4, U-DEL7, U-DEL8, U-DEL9 and U-DEL10) containing deoxyuridine, the Ct value was lowered as the same level of DS-DEL, which is a double-stranded DEL, after the USER (Registered trademark) enzyme treatment.

This result shows that the DEL cleaved by the USER (Registered trademark) enzyme has improved PCR efficiency than that before cleavage and that the cleavable hairpin DEL containing deoxyuridine was cleaved by the USER (Registered trademark) enzyme with high efficiency and high selectively.

### Example 3

### [Verification of cleavage reaction by USER (Registered trademark) enzyme of hairpin DEL containing deoxyuridine]

### <Synthesis of four kinds of hairpin DELs (U-DEL5, U-DEL11, U-DEL12 and U-DEL13)>

The compounds (hairpin DEL) having the sequences shown in Table 10 were synthesized by the following procedure. Incidentally, in the sequence notations in Table 10, "[mdC(TEG-amino)]" means a group represented by the following formula (9) 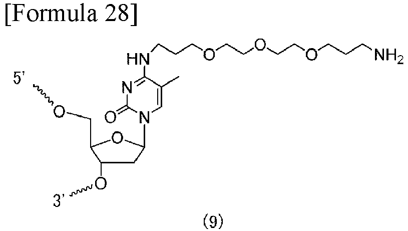 , and other notations are the same as in Table 4.
The names of the compounds corresponding to each SEQ ID NO: (No.) are as follows.

| | |
|---|---|
| No. 57: U-DEL5 | No. 58: U-DEL11 |
| No. 59: U-DEL12 | No. 60: U-DEL13 |

**[Table 10]**

| No. | Seq. | Expected MW . | Observed MW . (deconvolution) |
|---|---|---|---|
| 57 | GCAGGTGAAGCTTGTCTGAAGACTGCTTCTGGAACCACCATCGCACTTCCTGACC ACATCGATTTGGGAGTCATT(amino-NC6-dT)T(dU)TGACTCCCAAATCGATGTGGTC AGGAAGTGCGATGGTGGTTCCAGAAGCAGTCTTCAGACAAGCTTCACCTGC | 46638.2 | 46674.1 |
| 58 | G CAG G TG AAG C TTG TC TG AAG AC TG C TTC TG G AAC CAC CATC G CAC TTC C TG ACC AC ATC G ATTTG G G AG TC AAS (am inoC 7)S (dU )TG AC TC C C AAATC G ATG TG G TC AG G AAG TG C G ATG G TG G TTC C AG AAG C AG TC TTC AG AC AAG C TTC AC C TG C | 46283.0 | 46311.0 |
| 59 | GCAGGTGAAGCTTGTCTGAAGACTGCTTCTGGAACCACCATCGCACTTCCTGACC ACATCGATTTGGGAGTCAA[mdC(TEG-amino)](dU)TGACTCCCAAATCGATGTGGT CAGGAAGTGCGATGGTGGTTCCAGAAGCAGTCTTCAGACAAGCTTCACCTGC | 46156.0 | 46174.6 |
| 60 | G CAG G TG AAG C TTG TC TG AAG AC TG C TTC TG G AACCACCATCG CAC TTCC TG ACC ACATCG ATTTG G G AG TCATT[m dC (TEG -am ino)](dU )TTG AC TCCCAAATCG ATG TG G TCAG G AAG TG CG ATG G TG G TTCCAG AAG CAG TC TTCAG ACAAG C TTCACC TG C | 46755.4 | 46769.6 |

Incidentally, the names of the compounds of the raw material head piece for synthesizing each hairpin DEL are each as follows.

| | |
|---|---|
| Hairpin DEL | : Raw material head piece |
| U-DEL5 | : U-DEL5-HP |
| U-DEL11 | : U-DEL11-HP |
| U-DEL12 | : U-DEL12-HP |
| U-DEL13 | : U-DEL13-HP |

Further, SEQ ID NO: "No." of U-DEL11-HP, U-DEL12-HP and U-DEL13-HP and the sequences "Seq" are as mentioned in the following Table 11. Incidentally, the notations in Table 11 are the same as in Table 10.

**[Table 11]**

| | No. | Seq. |
|---|---|---|
| U-DEL11-HP | 61 | (p)GAGTCAAS(aminoC7)S(dU)TGACTCCC |
| U-DEL12-HP | 62 | (p)GAGTCAA[mdC(TEG-amino)](dU)TGACTCCC |
| U-DEL13-HP | 63 | (p)GAGTCATT[mdC(TEG-amino)](dU)TTGACTCCC |

Among the raw material head piece shown in Table 11, U-DEL12-HP and U-DEL13-HP were prepared using an automated polynucleotide synthesizer nS-8II (manufactured by GeneDesign, Inc.) in the same manner as in Example 1. U-DEL11-HP was also prepared according to a conventional method.

Similar to Example 2, using various kinds of raw material head pieces, two-step double-stranded ligation with the double-stranded oligonucleotide Pr_TAG and CP was carried out.

A part of the obtained solution was sampled and after diluting with deionized water, mass spectrometry by ESI-MS was carried out under Analytical condition 3 shown below to identify the target product (the expected molecular weight and the observed molecular weight of each sequence are shown in Table 10). After lyophilizing the rest of the solution, deionized water was each added to adjust the solution to 20 µM.

Analytical condition 3:
Device: Waters ACQUITY UPLC/SQ Detector
Column: ACQUITY UPLC Oligonucleotide BEH C18 Column (130Å, 1.7 µm, 2.1×50 mm)
Column temperature: 60°C
Solvent:
   Solution A: Water (0.75% v/v hexafluoroisopropanol; 0.038% v/v triethylamine; 5 µM ethylenediamine tetraacetic acid)
   Solution B: 90% v/v methanol aqueous solution (0.75% v/v hexafluoroisopropanol; 0.038% v/v triethylamine; 5µM ethylenediamine tetraacetic acid)
Gradient conditions:
   By fixing the flow rate of 0.36 mL/min and the mixing ratio of Solution A and Solution B to 95/5 (v/v), the measurement was started and after 0.56 minute, the mixing ratio of Solution A and Solution B was linearly changed to 40/60 (v/v) in 5.5 minutes.
Detection wavelength: 260 nm
Deconvolution:
   Ion signals were analyzed using ProMass for MassLynx Software (manufactured by Waters).

### <Cleavage reaction by USER (Registered trademark) enzyme>

Verification of the cleavage reaction by the USER (Registered trademark) enzyme of the hairpin DEL (U-DEL5, U-DEL7, U-DEL9, U-DEL11, U-DEL12 and U-DEL13) containing 6 kinds of deoxyuridines was carried out by the following procedure.

To a PCR tube were added 2 µL of various kinds of 20 µM hairpin DEL aqueous solution; 2 µL of CutSmart (Registered trademark) Buffer (available from New England BioLabs, Catalog number: B7204S) and 14 µL of deionized water. To the solution was added 2 µL of the USER (Registered trademark) enzyme (available from New England BioLabs, Catalog number: M5505S), and after incubating the obtained solution at 37°C for 16 hours, it was further incubated at 90°C for 1 hour.

### <Confirmation of product after cleavage by LC-MS measurement>

Among the obtained reaction solutions, 5.0 µL was sampled and after diluting with deionized water, mass spectrometry by ESI-MS was carried out under Analytical condition 3. The sequences and the expected molecular weights of the products after cleavage assumed in each reaction solution and the molecular weight observed in each reaction solution are shown in Table 12. Incidentally, the substrates corresponding to each experimental numbers (Entry) are as follows, and other notations are the same as in Table 10.

| | |
|---|---|
| Entry. 1: U-DEL5 | Entry. 2: U-DEL7 |
| Entry. 3: U-DEL9 | Entry. 4: U-DEL11 |
| Entry. 5: U-DEL12 | Entry. 6: U-DEL13 |

**[Table 12]**

| Entry | No. | Seq. | Expected M W. | Observed M W. (deconvolution) |
|---|---|---|---|---|
| 1 | 64 | GCAGGTGAAGCTTGTCTGAAGACTGCTTCTGGAACCACCATCGCACTTCCTGACCA CATCGATTTGGGAGTCATT(amino-NC6-dT)T(p) | 23829.5 | 23831.6 |
| | 65 | (p)TGACTCCCAAATCGATGTGGTCAGGAAGTGCGATGGTGGTTCCAGAAGCAGTCT TCAGACAAGCTTCACCTGC | 22616.6 | 226174 |
| 2 | 66 | G C AG G TG AAG C TTG TC TG AAG AC TG C TTC TG G AAC C AC C ATC G C AC TTC C TG AC C A C ATC G ATTTG G G AG TC A(p) | 22527.5 | 22527.6 |
| | 67 | (p)T (dSpacer) (dSpacer) (aminoC7) (dSpacer) (dSpacer)T T T G A C T C C C A A A T C G A T G T G G T C A G G A A G T G C G A T G G T G G T T C C A G A A G C A G T C T T C A G A C A A G C T T C A C C T G C | 24458.7 | 24459.9 |
| 3 | 68 | GCAGGTGAAGCTTGTCTGAAGACTGCTTCTGGAACCAGCATCGCACTTCCTGACCA CATCGATTTGGGAGTCAA(dSpacer)(dSpacer)(aminoC7)(dSpacer)(dSpacer)(p) | 23770.3 | 23780.4 |
| | 65 | (p)TGACTCCCAAATCGATGTGGTCAGGAAGTGCGATGGTGGTTCCAGAAGCAGTCT TCAGACAAGCTTCACCTGC | 22616.6 | 22615.8 |
| 4 | 69 | GCAGGTGAAGCTTGTCTGAAGACTGCTTCTGGAACCACCATCGCACTTCCTGACCA CATCGATTTGGGAGTCAAS(aminoC7)S(p) | 23474.2 | 23475.7 |
| | 65 | (p)TGACTCCCAAATCGATGTGGTCAGGAAGTGCGATGGTGGTTCCAGAAGCAGTCT TCAGACAAGCTTCACCTGC | 22616.6 | 22617.7 |
| 5 | 70 | GCAGGTGAAGCTTGTCTGAAGACTGCTTCTGGAACCACCATCGCACTTCCTGACCA CATCGATTTGGGAGTCAA[mdC(TEG-amino)](p) | 23347.2 | 23327.2 |
| | 65 | (p)TGACTCCCAAATCGATGTGGTCAGGAAGTGCGATGGTGGTTCCAGAAGCAGTCT TCAGACAAGCTTCACCTGC | 22616.6 | 22614.6 |
| 6 | 71 | GCAGGTGAAGCTTGTCTGAAGACTGCTTCTGGAACCACCATCGCACTTCCTGACCA CATCGATTTGGGAGTCATT[mdC(TEG-amino)](p) | 23642.4 | 23646.3 |
| | 72 | (p)TTG AC TCC CAAATCG ATG TGG TCAGG AAG TGCG ATGG TGG TTCCAG AAG CAG TC TTCAG AC AAG CTTCACC TG C | 22920.8 | 22929.0 |

In any of the samples, no MS of the substrate was detected, and the MS of the product after cleavage was observed as the main peak.

### <Confirmation of cleavage reaction by gel electrophoresis>

Also, among the obtained reaction solutions, a part thereof was sampled, and analyzed by modified polyacrylamide gel electrophoresis under the conditions shown below. From the results shown in Fig. 13, it was confirmed that the cleavage reaction proceeded in high yield for all the substrates. Incidentally, the samples of each Lane in Fig. 13 are as follows .
Lane 1: 20 bp DNA ladder (manufactured by Lonza, Lonza 20 bp DNA Ladder, Catalog number: 50330)
Lane 2: U-DEL5
Lane 3: Sample after subjecting to cleavage reaction of U-DEL5
Lane 4: U-DEL7
Lane 5: Sample after subjecting to cleavage reaction of U-DEL7
Lane 6: U-DEL9
Lane 7: Sample after subjecting to cleavage reaction of U-DEL9
Lane 8: U-DEL11
Lane 9: Sample after subjecting to cleavage reaction of U-DEL11
Lane 10: U-DEL12
Lane 11: Sample after subjecting to cleavage reaction of U-DEL12
Lane 12: U-DEL13
Lane 13: Sample after subjecting to cleavage reaction of U-DEL13
Modified polyacrylamide gel electrophoresis:
   Gel: Novex (merchandise mark) 10% TBE-urea gel (available from Invitrogen by ThermoFisher SCIENTIFIC, Catalog number: EC68755BOX)
   Loading Buffer: Novex (merchandise mark) 10% TBE-Urea Sample Buffer (2×) (available from Invitrogen by ThermoFisher SCIENTIFIC, Catalog number: LC6876)
   Temperature: 60°C
   Voltage: 180V
   Electrophoresis time: 30 min
   Dyeing reagent: SYBER (merchandise mark) GreenII Nucleic Acid Gel Stain (available from Takara Bio Inc., Catalog number: 5770A)

From the above results, in the hairpin type DEL containing various kinds of deoxyuridines, it was shown that, the cleavage reaction by the USER (Registered trademark) enzyme at the deoxyuridine site proceeded.

### Example 4

### [Verification of cleavage reaction by endonuclease V of hairpin DEL containing deoxyinosine]

### <Syntheses of hairpin DELs (I-DEL1, I-DEL2, I-DEL3 and I-DEL4) containing 4 kinds of deoxyinosines>

The compounds (hairpin DEL) having the sequence shown in Table 13 were synthesized by the following procedure. Incidentally, in the sequence notation in Table 13, "I" means deoxyinosine, and other notations are the same as in Table 2. The names of the compounds corresponding to each SEQ ID NO: (No.) are as follows.

| | |
|---|---|
| No. 73: I-DEL1 | No. 74: I-DEL2 |
| No. 75: I-DEL3 | No. 76: I-DEL4 |

**[Table 13]**

| No. | S eq. | Expected MW. | Observed MW. (deconvolution) |
|---|---|---|---|
| 73 | G C AG G TG AAG C TTG TC TG AAG AC TG C TTC TG G AACC ACC ATC G C AC TTC C TG ACC AC ATC G ATTTG G G AG TC AA (dSpacer) (dSpacer) (am inoC 7) (dSpacer) (dS pacer) TTTG A C TC C C AAATC G ATG TG G TC AG G AAG TG C G ATG G TG G TTC C AG AAG C AG TC TTC A G AC AAG C TTC ACC TG C | 47211.5 | 47241.5 |
| 74 | G CAG G TG AAG C TTG TC TG AAG AC TG C TTC TG G AAC C AC C ATC G C AC TTC C TG AC C AC ATC G ATTTG G G AG TC AT (dSpacer) (dSpacer) (am inoC 7) (dS pacer) (dS pacer) TTG AC T C C C AAATC G ATG TG G TC AG G AAG TG C G ATG G TG G TTC C AG AAG C AG TC TTC AG A C AAG C TTC AC C TG C | 46594.1 | 46621.2 |
| 75 | GCAGGTGAAGCTTGTCTGAAGACTGCTTCTGGAACCACCATCGCACTTCCTGACC ACATCGATTTGGGAGTCAAT(dSpacer)(dSpacer)(aminoC7)(dSpacer)(dSpacer)TTTGA CTCCCAAATCGATGTGGTCAGGAAGTGCGATGGTGGTTCCAGAAGCAGTCTTCA GACAAGCTTCACCTGC | 47211.5 | 47249.6 |
| 76 | G CAG G TG AAG C TTG TC TG AAG AC TG C TTC TG G AAC CAC CATCG CAC TTCC TG ACC AC ATCG ATTTG G G AG TCATA (dSpacer) (dSpacer) (am inoC 7) (dSpacer) (dSpacer) TTTG A C TCCC AAATCG ATG TG G TCAG G AAG TG CG ATG G TG G TTCC AG AAG CAG TC TTC A G AC AAG C TTC ACC TG C | 47211.5 | 47356.8 |

Incidentally, the names of the compounds of the raw material head piece for synthesizing each hairpin DEL are each as follows.

| | |
|---|---|
| Hairpin DEL | : Raw material head piece |
| I-DELI | : I-DEL1-HP |
| I-DEL2 | : I-DEL2-HP |
| I-DEL3 | : I-DEL3-HP |
| I-DEL4 | : I-DEL4-HP |

Further, SEQ ID NO: "No." of I-DEL1-HP, I-DEL2-HP, I-DEL3-HP and I-DEL4-HP and the sequence "Seq" are as shown in Table 14. Incidentally, the notations in Table 14 are the same as in Table 13.

**[Table 14]**

| | No. | S eq. |
|---|---|---|
| I-DEL1-HP | 77 | (p)GAGTCIAA(dSpacer)(dSpacer)(aminoC7)(dSpacer)(dSpacer)TTTGACTCCC |
| I-DEL2-HP | 78 | (p)GAGTCAT(dSpacer)(dSpacer)(aminoC7)(dSpacer)(dSpacer)ITGACTCCC |
| I-DEL3-HP | 79 | (p)GAGTGAAT(dSpacer)(dSpacer)(aminoC7)(dSpacer)(dSpacer)ITTGACTCCC |
| I-DEL4-HP | 80 | (p)GAGTCATA(dSpacer)(dSpacer)(aminoC7)(dSpacer)(dSpacer)TITGACTCCC |

The raw material head pieces shown in Table 14 were prepared according to a conventional method.

Similar to Example 2, using various kinds of raw material head pieces, two-step double-stranded ligation with the double-stranded oligonucleotide Pr_TAG and CP was carried out.

A part of the obtained solution was sampled and after diluting with deionized water, mass spectrometry by ESI-MS was carried out under Analytical condition 3 to identify the target product (the expected molecular weight and the observed molecular weight of each sequence are shown in Table 13). After lyophilizing the rest of the solution, deionized water was added to prepare the solution to 20 µM.

### <Cleavage reaction by endonuclease V>

Verification of the cleavage reaction by endonuclease V of the 4 kinds of hairpin DELs (I-DEL1, I-DEL2, I-DEL3, I-DEL4) containing deoxyinosines was carried out by the following procedure.

To a PCR tube were added 1 µL of various kinds of 20 µM hairpin DEL aqueous solution; 2 µL of NEBuffer (Registered trademark) 4 (available from New England BioLabs, Catalog number: B7004) and 15 µL of deionized water. To the solution was added 2 µL of Endonuclease V (available from New England BioLabs, Catalog number: M0305S), and the obtained solution was incubated at 37°C for 24 hours.

### <Confirmation of product after cleavage by LC-MS measurement>

Among the obtained reaction solutions, 8.0 µL was sampled and after diluting with deionized water, mass spectrometry by ESI-MS was carried out under Analytical condition 3. The sequences and the expected molecular weights of the products after cleavage assumed in each reaction solution and the molecular weight observed in each reaction solution are shown in Table 15. Incidentally, the substrates corresponding to each experimental numbers (Entry) are as follows, and other notations are the same as in Table 13.

| | |
|---|---|
| Entry. 1: I-DEL1 | Entry. 2: I-DEL2 |
| Entry. 3: I-DEL3 | Entry. 4: I-DEL4 |

**[Table 15]**

| Entry | No. | S eq. | Expecied MW. | Observed MW. (deconvolution) |
|---|---|---|---|---|
| 1 | 81 | G C A G G T G A A G C T T G T C T G A A G A C T G C T T C T G G A A C C A C C A T C G C A C T T C C T G A C C A C A T C G A T T T G G G A G T C A | 22761.7 | 22758.0 |
| | 82 | (p)A (dSpacer)(dSpacer)(aminoC7)(dSpacer)(dSpacer)TTTGACTCCCAAATCGATGTGG TCAGGAAGTGCGATGGTGGTTCCAGAAGCAGTCTTCAGACAAGCTTCACCTGC | 24467.8 | 24464.7 |
| 2 | 83 | G C A G G T G A A G C T T G T C T G A A G A C T G C T T C T G G A A C C A C C A T C G C A C T T C C T G A C C A C A T C G A T T T G G G A G T C A T (dS pacer) (dS pacer) (am inoC 7) (dS pacer) (dS pacer) TT | 24299.7 | 24297.5 |
| | 84 | (p)GACTCCCAAATCGATGTGGTCAGGAAGTGCGATGGTGGTTCCAGAAGCAGTCTT CAGACAAGCTTCACCTGC | 22312.4 | 22316.7 |
| 3 | 85 | GCAGGTGAAGCTTGTCTGAAGACTGCTTCTGGAACCACCATCGCACTTCCTGACCA CATCGATTTGGGAGTCAAT(dSpacer)(dSpacer)(aminoC7)(dSpacer)(dSpacer)TT | 24612.9 | 24614.4 |
| | 65 | (p)TGACTCCCAAATCGATGTGGTCAGGAAGTGCGATGGTGGTTCCAGAAGCAGTCT TCAGACAAGCTTCACCTGC | 22616.6 | 22615.0 |
| 4 | 86 | G C AG G TG AAG C TTG TC TG AAG AC TG C TTC TG G AAC C AC C ATC G C AC TTC C TG AC C A C ATC G ATTTG G G AG TC ATA (dS pacer) (dS pacer) (am ino C 7) (dS pacer) (dS pacer) TTT | 24917.1 | 24920.5 |
| | 84 | (p)GACTCCCAAATCGATGTGGTCAGGAAGTGCGATGGTGGTTCCAGAAGCAGTCTT CAGACAAGCTTCACCTGC | 22312.4 | 22310.3 |

In any of the samples, no MS of the substrate was detected, and the MS of the product after cleavage was observed as the main peak.

### <Confirmation of cleavage reaction by gel electrophoresis>

Also, among the obtained reaction solutions, a part thereof was sampled, and analyzed by modified polyacrylamide gel electrophoresis under the conditions same as Example 3. From the results shown in Fig. 14, it was confirmed that the cleavage reaction proceeded in high yield for all the substrates. Incidentally, the samples of each Lane in Fig. 14 are as follows .
Lane 1: 20 bp DNA ladder (manufactured by Lonza, Lonza 20 bp DNA Ladder, Catalog number: 50330)
Lane 2: I-DEL1
Lane 3: Sample after subjecting to cleavage reaction of I-DEL1
Lane 4: I-DEL2
Lane 5: Sample after subjecting to cleavage reaction of I-DEL2
Lane 6: I-DEL3
Lane 7: Sample after subjecting to cleavage reaction of I-DEL3
Lane 8: I-DEL4
Lane 9: Sample after subjecting to cleavage reaction of I-DEL4

From the above results, in the various kinds of hairpin type DEL containing deoxyinosines, it was shown that, the second phosphodiester bond in the 3' direction from the deoxyinosine is cleaved by endonuclease V.

### Example 5

### [Verification of cleavage reaction by RNaseHII of hairpin DEL containing ribonucleoside]

### <Synthesis of hairpin DEL (R-DEL1) containing ribonucleoside>

The compound (hairpin DEL) of the sequence shown in Table 16 was synthesized by the following procedure. Incidentally, in the sequence notations in Table 16, "u" means uridine, and other notations are the same as in Table 2.
The name of the compound corresponding to SEQ ID NO: (No.) is as follows. No. 87: R-DEL1

**[Table 16]**

| No. | Seq. | Expected MW. | Observed MW. (deconvolution) |
|---|---|---|---|
| 87 | GCAGGTGAAGCTTGTCTGAAGACTGCTTCTGGAACCACCATCGCACTTCCTGACC ACATCGATTTGGGAGTCAAA(dSpacer)(dSpacer)(aminoC7)(dSpacer)(dSpacer)TuTG ACTCCCAAATCGATGTGGTCAGGAAGTGCGATGGTGGTTCCAGAAGCAGTCTTCA GACAAGCTTCACCTGC | 47212.5 | 47237.7 |

Incidentally, the name of the compound of the raw material head piece for synthesizing each hairpin DEL is as follows.

| | |
|---|---|
| Hairpin DEL | : Raw material head piece |
| R-DEL1 | : R-DEL1-HP |

Further, SEQ ID NO: "No." of R-DEL1-HP and sequence "Seq" are as shown in Table 17. Incidentally, the notations in Table 17 are the same as in Table 16.

**[Table 17]**

| | No. | Seq. |
|---|---|---|
| R-DEL1-HP | 88 | (p)GAGTCAAA(dSpacer)(dSpacer)(aminoC7)(dSpacer)(dSpacer)TuTGACTCCC |

The raw material head pieces shown in Table 17 were prepared according to a conventional method.

Similar to Example 2, using raw material head pieces, two-step double-stranded ligation with the double-stranded oligonucleotide Pr_TAG and CP was carried out.

A part of the obtained solution was sampled and after diluting with deionized water, mass spectrometry by ESI-MS was carried out under Analytical condition 3 to identify the target product (the expected molecular weight and the observed molecular weight of each sequence are shown in Table 16). After lyophilizing the rest of the solution, deionized water was each added to adjust the solution to 200 µM.

### <Cleavage reaction by RNaseHII>

Verification of the cleavage reaction by RNaseHII of the hairpin DEL (R-DEL1) containing ribonucleoside was carried out by the following procedure.

To a PCR tube were added 0.5 µL of 200 µM hairpin DEL aqueous solution; 4.9 µL of ThermoPol (Registered trademark) Reaction Buffer Pack (available from New England BioLabs, Catalog number: B9004) and 43.6 µL of deionized water. To the solution was added 1 µL of RNase HII (available from New England BioLabs, Catalog number: M0288S), and the obtained solution was incubated at 37°C for 8 hours.

### <Confirmation of product after cleavage by LC-MS measurement>

Among the obtained reaction solutions, 10 µL was sampled and after diluting with deionized water, mass spectrometry by ESI-MS was carried out under Analytical condition 3. The sequences and the expected molecular weights of the products after cleavage assumed in each reaction solution and the molecular weight observed in each reaction solution are shown in Table 18. Incidentally, the substrates corresponding to each experimental numbers (Entry) are as follows, and other notations are the same as in Table 16.

### Entry. 1: R-DEL1

**[Table 18]**

| Entry | No. | Seq. | Expected MW. | Observed MW. (deconvolution) |
|---|---|---|---|---|
| 1 | 89 | GCAGGTGAAGCTTGTCTGAAGACTGCTTCTGGAACCACCATCGCACTTCCTGACCA CATCGATTTGGGAGTCAAA (dSpacer)(dSpacer)(aminoC7)(dSpacer)(dSpacer)T | 24307.7 | 24303.6 |
| | 90 | (b)uTG AC TCCCAAATCG ATG TGG TCAG GAAG TGCG ATGG TGG TTCCAG AAG CAG TC TTCAG ACAAG CTTCACCTG C | 22922.8 | 22918.3 |

In any of the samples, no MS of the substrate was detected, and the MS of the product after cleavage was observed as the main peak.

### <Confirmation of cleavage reaction by gel electrophoresis>

Also, among the obtained reaction solutions, a part thereof was sampled, and analyzed by modified polyacrylamide gel electrophoresis under the conditions same as Example 3. From the results shown in Fig. 15, it was confirmed that the cleavage reaction proceeded in high yield for all the substrates. Incidentally, the samples of each Lane in Fig. 15 are as follows .
Lane 1: 20 bp DNA ladder (manufactured by Lonza, Lonza 20 bp DNA Ladder, Catalog number: 50330)
Lane 2: R-DEL1
Lane 3: Sample after subjecting to cleavage reaction of R-DEL1

From the above results, in the hairpin type DEL containing ribonucleoside, it was shown that, the phosphodiester bond at the 5' side of the ribonucleotide is cleaved by RNaseHII.

### Example 6

### [Creation of model library using U-DEL9-HP as raw material]

As in the schematic diagram shown in Fig. 16, using U-DEL9-HP as a raw material, synthesis of the model library containing 3×3×3 (27) compound species was carried out by the split and pool synthesis using the following reagents.
·U-DEL9-HP
·3 kinds of building blocks (BB 1, BB2 and BB3): 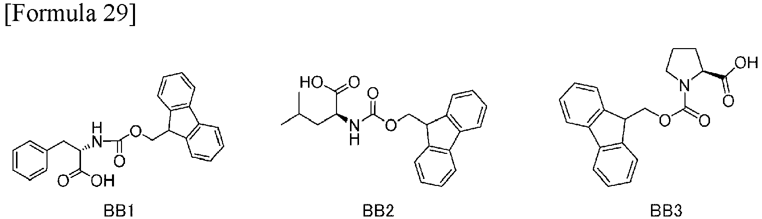
·10 kinds of double-stranded oligonucletide tags (tag number: Pr, A1, A2, A3, B1, B2, B3, C1, C2 and C3 in Table 19)

In Table 19, "Tag No." (leftmost) represents a tag number, "No." (second from the left) represents SEQ ID NO:, and "Seq." (third from the left) represents a sequence. Incidentally, the sequence notations are the same as in Table 1.

Incidentally, each double-stranded oligonucletide tag was prepared by, as shown in Table 19, annealing 2 kinds of oligonucleotides having a SEQ ID NO: corresponding to each tag number.

**[Table 19]**

| Tag No. | No. | Seq. |
|---|---|---|
| Pr | 91 | (p)TCCTGACCACATCGATTTGG |
| | 92 | (p)AAATCGATGTGGTCAGGAAG |
| A1 | 93 | (p)GCAACCACT |
| | 94 | (p)TGGTTGCGT |
| A2 | 95 | (p)GGAGACACT |
| | 96 | (p)TGTCTCCGT |
| A3 | 97 | (p)TAGGCGACT |
| | 93 | (p)TCGCCTAGT |
| B1 | 99 | (p)CACGCATAC |
| | 100 | (p)ATGCGTGGA |
| B2 | 101 | (p)CGTCAACAC |
| | 102 | (p)GTTGACGGA |
| B3 | 103 | (p)GCATGTCAC |
| | 104 | (p)GACATGCGA |
| C1 | 105 | (p)CTCTCCTTC |
| | 106 | (p)AGGAGAGTT |
| C2 | 107 | (p)GGATCGTTC |
| | 103 | (p)ACGATCCTT |
| C3 | 109 | (p)TGAACGCTC |
| | 110 | (p)GCGTTCATT |

### <Synthesis of compound "AOP-U-DEL9-HP">

The compound "AOP-U-DEL9-HP" having a sequence shown in Table 20 was synthesized by the following procedure. Incidentally, in the sequence notations in Table 20, "(AOP-AminoC7)" means a group represented by the following formula (10) 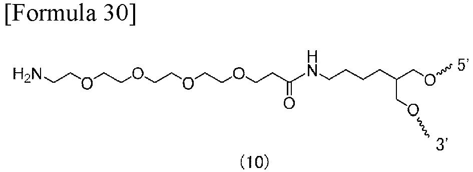 , and other notations are the same as in Table 2.

**[Table 20]**

| No. | Seq. | Expected MW. | Observed MW. (deconvolution) |
|---|---|---|---|
| 111 | (p)G A G TC A A (dSpacer)(dSpacer)(A O P-Am inoC 7) (dSpacer)(dSpacer)(dU )TG A C TC C C | 6084.0 | 6082.9 |

To four violamo centrifuge tubes was added a solution (2.5 mL, 1 mM) of U-DEL9-HP in a sodium borate buffer (150 mM, pH 9.4) cooled to 10°C. To the respective tubes were added 40 equivalent of N-Fmoc-15-amino-4,7,10,13-tetraoxa-octadecanoic acid (250 µL, 0.4M N,N-dimethylacetamide solution), subsequently 40 equivalents of 4-(4,6-dimethoxy[1.3.5]triazin-2-yl)-4-methylmorpholinium chloride hydrate (DMTMM) (200 µL, 0.5 M aqueous solution), and the obtained solution was shaken at 10°C for 5 hours.

The above-mentioned solutions were each treated by 295 µL of a 5 M aqueous sodium chloride solution and 9.7 mL of cooled (-20°C) ethanol, and allowed to stand at -78°C overnight. After centrifugation, the supernatant was removed and the obtained pellets were air-dried. To the pellets were each added 2.75 mL of deionized water to dissolve therein, 306 µL of piperidine was added thereto at 0°C, and the mixture was shaken at 10°C for 3 hours. After the mixture was centrifuged, the precipitates were removed by filtration, and washed with 1.47 mL of deionized water twice. The obtained filtrates were each treated with 600 µL of a 5 M aqueous sodium chloride solution and 19.8 mL of cooled (-20°C) ethanol and allowed to stand at -78°C overnight. After centrifugation, the supernatant was removed and the obtained pellets were air-dried.

To the obtained pellets was added 10 mL of deionized water to make it a solution. Of the obtained solution, a part thereof was sampled, diluted with deionized water, and then mass spectrometry by ESI-MS was carried out under the conditions of Analytical condition 2 of Example 1 to identify the target product (the expected molecular weight and the observed molecular weight of the compound are shown in Table 20). After lyophilizing the rest of the solution, deionized water was each added to adjust the solution to 5 mM.

### introduction of double-stranded oligonucletide tag "Pr">

The compound "AOP-U-DEL9-HP-Pr" of the sequence shown in Table 21 was synthesized by ligating the compound "AOP-U-DEL9-HP" and the double-stranded oligonucletide tag "Pr" according to the following procedure. Incidentally, the sequence notations in Table 21 are the same as in Table 20.

**[Table 21]**

| No. | Seq. | Expected MW. | Observed MW. (deconvolution) |
|---|---|---|---|
| 112 | (p)TCCTGACCACATCGATTTGGGAGTCAA(dSpacer)(dSpacer)((AOP-AminoC7) (dSpacer)(dSpacer)(dU)TGACTCCCAAATCGATGTGGTCAGGAAG | 18506.0 | 18501.4 |

To a violamo centrifuge tube were added 40 µL of 5 mM aqueous solution of the compound "AOP-U-DEL9-HP"; 160 µL of 100 mM aqueous sodium hydrogen carbonate solution; 240 µL of 1 mM aqueous solution of the double-stranded oligonucletide tag "Pr"; 80 µL of 10X ligase buffer (500 mM Tris hydrochloride, pH 7.5; 500 mM sodium chloride; 100 mM magnesium chloride; 100 mM dithiothreitol; and 20 mM adenosine triphosphate) and 272 µL of deionized water. To the solution was added 8.0 µL of T4DNA ligase (available from Thermo Fisher, Catalog number: EL0013), and the obtained solution was incubated at 16°C for 24 hours.

The reaction solution was treated with 80 µL of 5 M aqueous sodium chloride solution and 2640 µL of cooled (-20°C) ethanol and allowed to stand at -78°C for 2 hours. After centrifugation, the supernatant was removed, and 400 µL of deionized water was added to the obtained pellets. The obtained solution was concentrated by Amicon (Registered trademark) Ultra Centrifugal filter (30 kD cutoff). A part of the obtained solution was sampled, and mass spectrometry by ESI-MS was carried out under the conditions of Analytical condition 2 to identify the target product (the expected molecular weight and the observed molecular weight of the compound are shown in Table 21). According to the above procedures, 133 nmol of the compound "AOP-U-DEL9-HP-Pr" with a purity of 84.5% was obtained. To the obtained compound "AOP-U-DEL9-HP-Pr" was added a 100 mM aqueous sodium hydrogen carbonate solution to adjust the solution to 1 mM.

### <Cycle A>

To each of three PCR tubes were added 20 µL of 1 mM solution of the compound "AOP-U-DEL9-HP-Pr" obtained as mentioned above; 30 µL of a 1 mM aqueous solution of one of the double-stranded oligonucletide tags A1 to A3; 8.0 µL of 10X ligase buffer (500 mM Tris hydrochloride, pH 7.5; 500 mM sodium chloride; 100 mM magnesium chloride; 100 mM dithiothreitol; and 20 mM adenosine triphosphate) and 21.6 µL of deionized water. To the solution was added 0.4 µL of T4DNA ligase (available from Thermo Fisher, Catalog number: EL0013), and the obtained solution was incubated at 16°C for 18 hours.

The reaction solutions were each treated with 8.0 µL of a 5 M aqueous sodium chloride solution and 264 µL of cooled (-20°C) ethanol, and allowed to stand at -78°C for 30 minutes. After centrifugation, the supernatant was removed, and the obtained pellets were each dissolved in 20 µL of 150 mM sodium borate buffer (pH 9.4).

To each tube were added 40 equivalents of one of the building blocks BB 1 to BB3 (4.0 µL, 200 mM N,N-dimethylacetamide solution), subsequently 40 equivalents of 4-(4,6-dimethoxy[1.3.5]triazin-2-yl)-4-methylmorpholinium chloride hydrate (DMTMM) (4.0 µL, 200 mM aqueous solution), and the mixture was shaken at 10°C for 2 hours. Further, to each tube were added 20 equivalents of building blocks (2.0 µL, 200 mM N,N-dimethylacetamide solution), subsequently 20 equivalents of DMTMM (2.0 µL, 200 mM aqueous solution), and the mixture was shaken at 10°C for 30 minutes.

The reaction solutions were each treated with 3.2 µL of a 5 M aqueous sodium chloride solution and 106 µL of cooled (-20°C) ethanol, and allowed to stand at -78°C for 30 minutes. After centrifugation, the supernatant was removed, and after the obtained pellets were each added 18 µL of deionized water, 3 kinds of the solutions were mixed in one PCR tube.

To the mixed solution was added 6.0 µL of piperidine at 0°C, and the mixture was shaken at room temperature for 1 hour. The reaction solution was treated with 6.0 µL of a 5 M aqueous sodium chloride solution and 198 µL of cooled (-20°C) ethanol, and allowed to stand at -78°C for 18 hours. After centrifugation, the supernatant was removed, and 400 µL of deionized water was added to the obtained pellets. The obtained solution was concentrated by Amicon (Registered trademark) Ultra Centrifugal filter (30 kD cutoff), a 100 mM aqueous sodium hydrogen carbonate solution was added to adjust the solution to 1 mM, and used in the next step as a raw material.

### <Cycle B>

To each of three PCR tubes were added 13.7 µL of 1 mM solution obtained in Cycle A as a raw material; 20.6 µL of 1 mM aqueous solution of one of the three double-stranded oligonucletide tags B1 to B3; 5.5 µL of 10X ligase buffer (500 mM Tris hydrochloride, pH 7.5; 500 mM sodium chloride; 100 mM magnesium chloride; 100 mM dithiothreitol; and 20 mM adenosine triphosphate) and 14.8 µL of deionized water. To the solution was added 0.3 µL of T4DNA ligase (available from Thermo Fisher, Catalog number: EL0013), and the obtained solution was incubated at 16°C for 16 hours.

The reaction solutions were each treated with 5.5 µL of a 5 M aqueous sodium chloride solution and 181 µL of cooled (-20°C) ethanol, and allowed to stand at -78°C for 30 minutes. After centrifugation, the supernatant was removed, the obtained pellets were each dissolved in 13.7 µL of 150 mM sodium borate buffer (pH 9.4).

To each tube were added 80 equivalents of one of the building blocks BB1 to BB3 (5.5 µL, 200 mM N,N-dimethylacetamide solution), subsequently 80 equivalents of DMTMM (5.5 µL, 200 mM aqueous solution), and the mixture was shaken at 10°C for 1 hour. Further, to each tube were added 40 equivalents of the building block (2.3 µL, 200 mM N,N-dimethylacetamide solution), subsequently 40 equivalents of DMTMM (2.3 µL, 200 mM aqueous solution), and the mixture was shaken at 10°C for 2 hours.

The reaction solutions were each treated with 2.5 µL of a 5 M aqueous sodium chloride solution and 81.4 µL of cooled (-20°C) ethanol, and allowed to stand at -78°C for 30 minutes. After centrifugation, the supernatant was removed, and after the obtained pellets were each added 12.3 µL of deionized water, 3 kinds of the solutions were mixed in one PCR tube.

To the mixed solution was added 4.1 µL of piperidine at 0°C, and the mixture was shaken at room temperature for 3 hours. The reaction solution was treated with 4.1 µL of a 5 M aqueous sodium chloride solution and 136 µL of cooled (-20°C) ethanol, and allowed to stand at -78°C for 3 hours. After centrifugation, the supernatant was removed, and 400 µL of deionized water was added to the obtained pellets. The obtained solution was concentrated by Amicon (Registered trademark) Ultra Centrifugal filter (30kD cutoff), a 100 mM aqueous sodium hydrogen carbonate solution was added to adjust the solution to 0.48 mM, and used in the next step as a raw material.

### <Cycle C>

To each of three PCR tubes were added 14.5 µL of 0.48 mM solution obtained in Cycle B as a raw material; 10.5 µL of 1 mM aqueous solution of one of the three double-stranded oligonucletide tags C1 to C3; and 2.8 µL of 10X ligase buffer (500 mM Tris hydrochloride, pH 7.5; 500 mM sodium chloride; 100 mM magnesium chloride; 100 mM dithiothreitol; and 20 mM adenosine triphosphate). To the solution was added 0.14 µL of T4DNA ligase (available from Thermo Fisher, Catalog number: EL0013), and the obtained solution was incubated at 16°C for 16 hours.

The reaction solutions were each treated with 2.8 µL of a 5 M aqueous sodium chloride solution and 92 µL of cooled (-20°C) ethanol, and allowed to stand at -78°C for 30 minutes. After centrifugation, the supernatant was removed, and the obtained pellets were each dissolved in 7.0 µL of 150 mM sodium borate buffer (pH 9.4).

To each tube were added 80 equivalents of one of the building blocks BB1 to BB3 (2.8 µL, 200 mM N,N-dimethylacetamide solution), subsequently 80 equivalents of DMTMM (2.8 µL, 200 mM aqueous solution), and the mixture was shaken at 10°C for 1 hour. Further, to each tube were added 40 equivalents of building block (1.4 µL, 200 mM N,N-dimethylacetamide solution), subsequently 40 equivalents of DMTMM (1.4 µL, 200 mM aqueous solution), and the mixture was shaken at 10°C for 2 hours.

The reaction solutions were each treated with 1.3 µL of a 5 M aqueous sodium chloride solution and 41.4 µL of cooled (-20°C) ethanol, and allowed to stand at -78°C for 30 minutes. After centrifugation, the supernatant was removed, and after the obtained pellets were each added 6.3 µL of deionized water, 3 kinds of the solutions were mixed in one PCR tube.

To the mixed solution was added 2.1 µL of piperidine at 0°C, and the mixture was shaken at room temperature for 2 hours. The reaction solution was treated with 2.1 µL of a 5 M aqueous sodium chloride solution and 69 µL of cooled (-20°C) ethanol, and allowed to stand at -78°C for 3 hours. After centrifugation, the supernatant was removed, and 400 µL of deionized water was added to the obtained pellets. The obtained solution was concentrated by Amicon (Registered trademark) Ultra Centrifugal filter (30 kD cutoff), a 100 mM aqueous sodium hydrogen carbonate solution was added to adjust the solution to 0.41 mM, and used in the next step as a raw material.

### < Ligation of CP>

To a PCR tube were added 12.2 µL of 0.41 mM solution of a raw material obtained in Cycle C; 6.0 µL of 1 mM aqueous solution of CP (the same as that used in Example 2); 2.1 µL of 10X ligase buffer (500 mM Tris hydrochloride, pH 7.5; 500 mM sodium chloride; 100 mM magnesium chloride; 100 mM dithiothreitol; and 20 mM adenosine triphosphate) and 0.7 µL of deionized water. To the solution was added 0.1 µL of T4DNA ligase (available from Thermo Fisher, Catalog number: EL0013), and the obtained solution was incubated at 16°C for 16 hours.

The reaction solution was treated with 2.1 µL of a 5 M aqueous sodium chloride solution and 69.6 µL of cooled (-20°C) ethanol, and allowed to stand at -78°C for 30 minutes. After centrifugation, the supernatant was removed, and 400 µL of deionized water was added to the obtained pellets. The obtained solution was concentrated by Amicon (Registered trademark) Ultra Centrifugal filter (30 kD cutoff), and deionized water was added to adjust the solution to 20 µM.

### <Results>

The samples after ligation of the double-stranded oligonucletide tag for each cycle were analyzed by electrophoresis using a 2.2% agarose gel (manufactured by Lonza, FlashGel (Registered trademark) cassette, Catalog number: 57031). From the results shown in Fig. 17, in each cycle, it was confirmed that coding with the double-stranded oligonucletide tag was achieved with high efficiency. Incidentally, the samples of each Lane in Fig. 17 are as follows.
Lane 1: AOP-U-DEL9-HP-Pr
Lane 2: Sample after ligation of the double-stranded oligonucletide tag A1 of Cycle A
Lane 3: Sample after ligation of the double-stranded oligonucletide tag A2 of Cycle A
Lane 4: Sample after ligation of the double-stranded oligonucletide tag A3 of Cycle A
Lane 5: Sample after ligation of the double-stranded oligonucletide tag B1 of Cycle B
Lane 6: Sample after ligation of the double-stranded oligonucletide tag B2 of Cycle B
Lane 7: Sample after ligation of the double-stranded oligonucletide tag B3 of Cycle B
Lane 8: Sample after ligation of the double-stranded oligonucletide tag C1 of Cycle C
Lane 9: Sample after ligation of the double-stranded oligonucletide tag C2 of Cycle C
Lane 10: Sample after ligation of the double-stranded oligonucletide tag C3 of Cycle C
Lane 11: Sample after CP ligation
Lane 12: 20 bp DNA ladder (manufactured by Lonza, Lonza 20 bp DNA Ladder, Catalog number: 50330)

The samples after completion of Cycle C were analyzed under Analytical condition 3. Fig. 18 shows the results of chromatograph and mass spectrum. By deconvolution of the obtained mass spectrum, 35532.4 was observed as an average molecular weight. This result is consistent with the average molecular weight (35514.2) expected after the completion of Cycle C, and it is shown that the reaction for synthesis of library (ligation of double-stranded oligonucletide tag and introduction of building blocks) was achieved with high efficiency.

According to the above, by the above-mentioned procedure of synthesis, synthesis of the model library containing the 3×3×3 (27) compound species using U-DEL9-HP as a raw material was achieved.

### <Cleavage of obtained model library by USER (Registered trademark) enzyme>

The cleavage reaction by the USER (Registered trademark) enzyme of the obtained model library as mentioned above was carried out by the following procedure.

To a PCR tube were added 2.0 µL of 20 µM model library aqueous solution; 2 µL of CutSmart (Registered trademark) Buffer (available from New England BioLabs, Catalog number: B7204S) and 14 µL of deionized water. To the solution was added 2 µL of USER (Registered trademark) enzyme (available from New England BioLabs, Catalog number: M5505S), and the obtained solution was incubated at 37°C for 16 hours, and then, further incubated at 90°C for 1 hour.

Among the obtained reaction solutions, a part thereof was sampled, and analysis was carried out by modified polyacrylamide gel electrophoresis under the same conditions as in Example 3. From the results shown in Fig. 19, it was confirmed that the model library using U-DEL9-HP as a raw material was able to proceed with the cleavage reaction by the USER (Registered trademark) enzyme with high efficiency. Incidentally, the samples of each Lane in Fig. 19 are as follows.
Lane 1: 20 bp DNA ladder (manufactured by Lonza, Lonza 20 bp DNA Ladder, Catalog number: 50330)
Lane 2: Model library
Lane 3: Sample after subjecting to cleavage reaction by USER (Registered trademark) enzyme of model library

### Example 7

### [Conversion of DEL compound from hairpin DNA to single-stranded DNA and addition of new function]

### <Synthesis of DEL compound "BIO-DEL" having biotin at 3' terminal>

Similar to Example 2, the DEL compound "BIO-DEL" having the sequence shown in Table 22 was synthesized by the following procedure. Incidentally, in the sequence notations in Table 22, "(BIO)" means a group represented by the following formula (11) 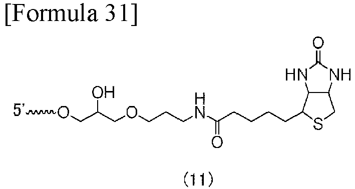 , and other notations are the same as in Table 20.

**[Table 22]**

| No. | Seq. | Expected MW. | Observed MW. (deconvolution) |
|---|---|---|---|
| 113 | GCAGGTGAAGCTTGTCTGAATACTCGGTCACTTGCCACTGCCTTGCTTCCTGA CCACATCGATTTGGGAGTCAA(dSpacer)(dSpacer)(AOP-AminoC7) (dSpacer)(dSpacer)(dU)TGACTCCCAAATCGATGTGGTCAGGAAGCAAGGCAGT GGCAAGTGACCGAGTATTCAGACAAGCTTCACCTGC(BD) | 47263.8 | 47288.8 |

To a PCR tube were added 20µL of 1 mM aqueous solution of AOP-U-DEL9-HP (synthesized in Example 6); 24 µL of 1 mM aqueous solution of Pr_TAG2 (prepared by annealing Pr_TAG2_a and Pr_TAG2_b synthesized in the same manner as in Example 1, the sequence is shown in Table 23); 8 µL of 10X ligase buffer (500 mM Tris hydrochloride, pH 7.5; 500 mM sodium chloride; 10 mM magnesium chloride; 100 mM dithiothreitol; and 20 mM adenosine triphosphate) and 20 µL of deionized water. To the solution was added 8 µL of a 10-fold diluted aqueous solution of T4DNA ligase (available from Thermo Fisher, Catalog number: EL0013), and the obtained solution was incubated at 16°C for 22 hours. Incidentally, the sequence notations in Table 23 are the same as in Table 1 Also, the names of the compounds corresponding to each SEQ ID NO: (No.) are as follows.

| | |
|---|---|
| No. 114: Pr_TAG2_a | No. 115: Pr_TAG2_b |

**[Table 23]**

| No. | Seq. |
|---|---|
| 114 | (p)TACTCGGTCACTTGCCACTGCCTTGCTTCCTGACCACATCGATTTGG |
| 115 | (p)AAATGGATGTGGTGAGGAAGGAAGGGAGTGGGAAGTGAGGGAGTATT |

The reaction solution was treated with 8 µL of 5 M aqueous sodium chloride solution and 264 µL of cooled (-20°C) ethanol, and allowed to stand at -78°C overnight. After centrifugation, the supernatant was removed and the obtained pellets were air-dried. The pellets were dissolved in deionized water, and purified by reverse phase HPLC using Phenomenex Gemini C18 column. Using a dual mobile phase gradient profile, the target product was eluted using 50 mM triethyl ammonium acetate buffer (pH 7.5) and acetonitrile/50 mM triethyl ammonium acetate buffer (9:1, v/v). Fractions containing the target product were collected, mixed and concentrated. The obtained solution was desalted with an Amicon (Registered trademark) Ultra Centrifugal filter (3 kD cutoff) and ethanol precipitation was carried out, and then, 25 µL of deionized water was added to the pellets to make it a solution.

Of the obtained solution, a part thereof was sampled and after diluting with deionized water, mass spectrometry by ESI-MS was carried out under the conditions of Analytical condition 2 of Example 1 to identify the target product (the expected molecular weight and the observed molecular weight of the compound are shown in Table d). After lyophilizing the rest of the solution, a 100 mM aqueous sodium hydrogen carbonate solution was each added to adjust the solution to 1 mM.

To 6.2 µL of the solution obtained as mentioned above were added 7.4 µL of 1 mM CP-BIO aqueous solution (prepared by annealing CP_a and CP-BIO_b synthesized in the same manner as in Example 1, the sequence is shown in Table 24); 2.5 µL of 10X ligase buffer (500 mM Tris hydrochloride, pH 7.5; 500 mM sodium chloride; 10 mM magnesium chloride; 100 mM dithiothreitol; 20 mM adenosine triphosphate) and 6.2 µL of deionized water. To the solution was added 2.47 µL of 10-fold diluted aqueous solution of T4DNA ligase (available from Thermo Fisher, Catalog number: EL0013), and the obtained solution was incubated at 16°C for 16 hours. Incidentally, the sequence notations in Table 24 are the same as in Table 23. Also, the names of the compounds corresponding to each SEQ ID NO: (No.) are as follows.

| | |
|---|---|
| No. 51: CP_a | No. 116: CP-BIO_b |

**[Table 24]**

| No. | Seq. |
|---|---|
| 51 | GCAGGTGAAGCTTGTCTGAA |
| 116 | (p)CAGACAAGCTTCACCTGC(BIO) |

The reaction solution was treated with 2.5 µL of 5 M aqueous sodium chloride solution and 81.5 µL of cooled (-20°C) ethanol, and allowed to stand at -78°C for 30 minutes. After centrifugation, the supernatant was removed, and the obtained pellets were air-dried, and the pellets were dissolved in deionized water. The obtained solution was desalted with an Amicon (Registered trademark) Ultra Centrifugal filter (3 kD cutoff).

Among the obtained supernatant, a part thereof was sampled and after diluting with deionized water, mass spectrometry by ESI-MS was carried out under Analytical condition 3 of Example 3 to identify the target product (the expected molecular weight and the observed molecular weight are shown in Table 22). After lyophilizing the rest of the solution, deionized water was each added to adjust the solution to 120 µM, whereby BIO-DEL was obtained.

### <Cleavage of BIO-DEL by USER (Registered trademark) enzyme>

The cleavage reaction of the DEL compound "BIO-DEL" obtained as mentioned above by the USER (Registered trademark) enzyme was carried out by the following procedure so synthesize the DEL compound "DS-BIO-DEL" having the double-stranded nucleic acids of the sequence shown in Table 25. Incidentally, the sequence notations in Table 25 are the same as in Table 22, and it means that DS-BIO-DEL is formed by the double strand of the oligonucleotide chains of SEQ ID NO:118 and SEQ ID NO:119.

**[Table 25]**

| | No. | Seq. | Expected MW. | Observed MW. (deconvolution) |
|---|---|---|---|---|
| DS-BIO-DEL | 117 | (p)TGACTCCCAAATCGATGTGGTCAGGAAGCAAGGCAGTGGCA AGTGACCGAGTATTCAGACAAGCTTCACCTGC(BD) | 23081.1 | 23083.6 |
| | 118 | G C AG G TG AAG C TTG TC TG AATAC TC G G TC AC TTG C C AC TG C C T TG C TTC C TG AC C AC ATC G ATTTG G G AG TC AA (dS pacer) (dS pacer) (A0 P-Am inoC 7) (dS pacer) (dS pacer) (p) | 23990.5 | 24002.0 |

To three PCR tubes were each added 10 µL of 120 µM aqueous solution of the DEL compound "BIO-DEL"; 100 µL of CutSmart (Registered trademark) Buffer (available from New England BioLabs, Catalog number: 7240S) and 860 µL of deionized water. To the solutions was each added 30 µL of USER (Registered trademark) enzyme (available from New England BioLabs, Catalog number: 5505S), and the obtained solution was incubated at 37°C for 24 hours.

The obtained reaction solutions were each desalted with an Amicon (Registered trademark) Ultra Centrifugal filter (3 kD cutoff), deionized water was added thereto to adjust it to 60 µL of a solution. Thereafter, the respective solutions were treated with 6 µL of 5 M aqueous sodium chloride solution and 198 µL of cooled (-20°C) ethanol, and allowed to stand at -78°C for 30 minutes. After centrifugation, the supernatant was removed, and deionized water was added to the obtained pellets to make it a solution, which was combined in one tube.

A part of the obtained solution was sampled and after diluting with deionized water, mass spectrometry by ESI-MS was carried out under Analytical condition 3 of Example 3, so that the objective DEL compound "DS-BIO-DEL" having the double-stranded nucleic acids was identified (the expected molecular weight and the observed molecular weight of the compound are shown in Table 25)

Also, among the obtained reaction solution, a part was sampled and analysis thereof by modified polyacrylamide gel electrophoresis under the same conditions as in Example 3 was carried out. From the results shown in Fig. 20, it was confirmed that BIO-DEL was cleaved with high yield and converted into DS-BIO-DEL. Incidentally, the samples of each Lane in Fig. 20 were as follows.
Lane 1: BIO-DEL (concentration 1: BIO-DEL is adjusted to be about 40 ng)
Lane 2: BIO-DEL (concentration 2: BIO-DEL is adjusted to be about 80 ng)
Lane 3: Sample (concentration 1: the target product is adjusted to be about 40 ng) after subjecting to cleavage reaction of BIO-DEL by USER (Registered trademark) enzyme
Lane 4: Sample (concentration 2: the target product is adjusted to be about 80 ng) after subjecting to cleavage reaction of BIO-DEL by USER (Registered trademark) enzyme
Lane 5: 20 bp DNA ladder (manufactured by Lonza, Lonza 20 bp DNA Ladder, Catalog number: 50330)

### <Preparation of DEL having single-stranded DNA using streptavidin beads>

The DEL compound "DS-BIO-DEL" having the double-stranded nucleic acid obtained as mentioned above was treated with streptavidin beads, and a DEL compound "SS-DEL" having a single-stranded DNA was prepared by the following procedure. Incidentally, SS-DEL is the oligonucleotide chain of SEQ ID NO:119 in Table 25.

To two PCR tubes was each added 450 µL of Magnosphere (merchandise mark) MS160/Streptavidin (JSR Life Sciences, Catalog number: J-MS-S160S), and after the supernatant was removed by magnetic separation, 900 µL of 1×binding buffer (10 mM Tris hydrochloride, pH 7.5; 0.5 mM ethylenediamine tetraacetic acid; 1 M sodium chloride; and 0.05% v/v Tween 20) was added and the supernatant was removed by magnetic separation. To the obtained particles were each added DS-BIO-DEL aqueous solution k (700 pmol, 450 µL) and 450 µL of 2×binding buffer (20 mM Tris hydrochloride, pH 7.5; 1 mM ethylenediamine tetraacetic acid; 2 M sodium chloride; and 0.1% v/v Tween 20) and mixed, and shaken at room temperature for 20 minutes.

The supernatant was removed from the mixture by magnetic separation, and washing of particles using 900 µL of 1×binding buffer (10 mM Tris hydrochloride, pH 7.5; 0.5 mM ethylenediamine tetraacetic acid; 1 M sodium chloride; and 0.05% v/v Tween20) and removal of the supernatant by magnetic separation were each repeated three times. Thereafter, each 900 µL of an aqueous solution (0.1 M sodium hydroxide; and 0.1 M sodium chloride) was added, and the supernatant was recovered by magnetic separation.

To the obtained supernatant was each added 900 µL of 3-(N-morpholino)-propanesulfonic acid buffer (1.0 M, pH 7.0) and desalted with an Amicon (Registered trademark) Ultra Centrifugal filter (3 kD cutoff). The obtained supernatants were combined into one tube, treated with 13.6 µL of 5 M aqueous sodium chloride solution and 448 µL of cooled (-20°C) ethanol, and allowed to stand at -78°C for 60 minutes. After centrifugation, the supernatant was removed and the obtained pellets were air-dried. To the pellets was added 60 µL of deionized water to make it a solution.

Of the obtained solution, a part thereof was sampled and after diluting with deionized water, and when mass spectrometry by ESI-MS was carried out under the conditions of Analytical condition 3 of Example 3, then the molecular weight of 23984.8 was observed, whereby the objective DEL compound "SS-DEL" having the single-stranded DNA was identified.

### <Synthesis of photoreactive cross linker-modified primer>

The photoreactive cross linker-modified primer "PXL-Pr" of the sequence shown in 26 was synthesized by the following procedure. Incidentally, in the sequence notations in Table 26, "(X)" means a group represented by the following formula (12) 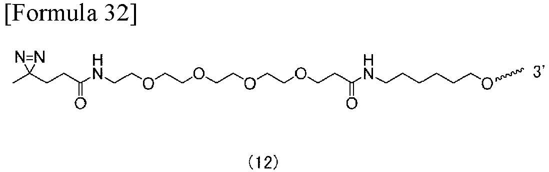 , and other notations are the same as in Table 2.

**[Table 26]**

| | No. | Seq. | Expected MW. | Observed MW. (deconvolution) |
|---|---|---|---|---|
| PXL-Pr | 119 | XTTGACTCCCAAATCGATGTG | 6628.6 | 6627.6 |

To a PCR tube were added a solution (200 µL, 1 mM) of L-Pr (synthesized in the same manner as in Example 1, the sequence is shown in Table 27) in sodium borate buffer (150 mM, pH 9.4) cooled to 10°C. To a tube were added 40 equivalents of N-Fmoc-15-amino-4,7,10,13-tetraoxaoctadecanoic acid (20 µL, 0.4 M N,N-dimethylacetamide solution), subsequently 40 equivalents of 4-(4,6-dimethoxy[1.3.5]triazin-2-yl)-4-methylmorpholinium chloride hydrate (DMTMM) (16 µL, 0.5 M aqueous solution), and the formed mixture was shaken at 10°C for 5 hours. Incidentally, the sequence notations in Table 27 are the same as in Table 8.

**[Table 27]**

| | No. | Seq. |
|---|---|---|
| L-Pr | 120 | (amino-C6-L)TTGACTCCCAAATCGATGTG |

The reaction liquid was treated with 23.6 µL of 5 M aqueous sodium chloride solution and 778.8 µL of cooled (-20°C) ethanol, and allowed to stand at -78°C overnight. After centrifugation, the supernatant was removed and the obtained pellets were air-dried. To the pellets was added 180 µL of deionized water to make it a solution, and then, 20 µL of piperidine was added thereto and the mixture was shaken at 10°C for 3 hours.

The obtained solution was treated with 20 µL of 5 M aqueous sodium chloride solution and 660 µL of cooled (-20°C) ethanol, and allowed to stand at -78°C for 30 minutes. After centrifugation, the supernatant was removed, and to the obtained pellets was added 200 µL of deionized water to make it a 1 mM solution.

To 100 µL of the solution obtained as mentioned above were added 75 µL of triethylamine hydrochloride buffer (500 mM, pH 10), subsequently 50 equivalents of sodium 1-((3-(3-methyl-3H-diazirin-3-yl)propanoyl)oxy)-2,5-dioxopyrrolidine-3-sulfonate (Sulfo-SDA) (25 µL, 200 mM aqueous solution), and the mixture was shaken at 37°C for 2 hours.

The obtained solution was treated with 20 µL of 5 M aqueous sodium chloride solution and 660 µL of cooled (-20°C) ethanol, and allowed to stand at -78°C for 30 minutes. After centrifugation, the supernatant was removed, and to the obtained pellets were added 100 µL of deionized water, subsequently 75 µL of triethylamine hydrochloride buffer (500 mM, pH 10) and 50 equivalents of Sulfo-SDA (25 µL, 200 mM aqueous solution), and the mixture was shaken at 37°C for 1 hour and 20 minutes. Further, 50 equivalents of Sulfo-SDA (25 µL, 200 mM aqueous solution) was added thereto, and the mixture was shaken at 37°C for 40 minutes.

The obtained solution was treated with 22.5 µL of 5 M aqueous sodium chloride solution and 743 µL of cooled (-20°C) ethanol, and allowed to stand at -78°C overnight. After centrifugation, the supernatant was removed, to the obtained pellets was added 100 µL of deionized water, subsequently 75 µL of triethylamine hydrochloride buffer (500 mM, pH 10), subsequently 50 equivalents of Sulfo-SDA (25 µL, 200 mM aqueous solution) was added thereto, and the mixture was shaken at 37°C for 3 hours.

The obtained solution was treated with 20 µL of 5 M aqueous sodium chloride solution and 660 µL of cooled (-20°C) ethanol, and allowed to stand at -78°C overnight. After centrifugation, the supernatant was removed and the obtained pellets were air-dried. The pellets were dissolved in 50 mM triethyl ammonium acetate buffer (pH 7.5), and purified by reverse phase HPLC using Phenomenex Gemini C18 column. Using a dual mobile phase gradient profile, the target product was eluted using 50 mM triethyl ammonium acetate buffer (pH7.5) and acetonitrile/water (100:1, v/v). Fractions containing the target product were collected, mixed and concentrated. The obtained solution was desalted with an Amicon (Registered trademark) Ultra Centrifugal filter (3 kD cutoff) and ethanol precipitation was carried out, and then, 100 µL of deionized water was added to the pellets to make it a solution.

Of the obtained solution, a part thereof was sampled and after diluting with deionized water, and mass spectrometry by ESI-MS was carried out under the conditions of Analytical condition 3 of Example 3, then the objective product of the photoreactive cross linker-modified primer "PXL-Pr" was identified (the expected molecular weight and the observed molecular weight of the compound are shown in Table 26).

### <Synthesis of photoreactive cross linker-modified double-stranded DEL>

Using SS-DEL and PXL-Pr obtained as mentioned above, a primer elongation reaction was carried out by the following procedure to synthesize the photoreactive cross linker-modified double-stranded DEL "PXL-DS-DEL" having the sequence shown in Table 28. Incidentally, the sequence notations in Table 28 are the same as in Table 26, and it means that PXL-DS-DEL is formed by a double strand of the oligonucleotide chains of SEQ ID NO:122 and SEQ ID NO:119.

**[Table 28]**

| | No. | Seq. | Expected MW. | Observed MW. (deconvolution) |
|---|---|---|---|---|
| PXL-DS-DEL | 121 | XTTG ACTCCCAAATCG ATG TGG TCAG G AAG CAAG G CAG TG G C AAG TG ACCG AG TATTCAG ACAAG CTTCACCTG C | 23404.4 | 23400.4 |
| | 118 | G C AG G TG AAG C TTG TC TG AATAC TC G G TC AC TTG C C AC TG C C T TG C TTC C TG AC C AC ATC G ATTTG G G AG TC AA (dSpacer)(dSpacer) (AO P-Am inoC 7)(dS pacer)(dS pacer)(p) | 23990.5 | 23987.8 |

To a PCR tube were added 50 µL of 8 µM "SS-DEL" aqueous solution; 0.673 µL of 594 µM "PXL-Pr" aqueous solution; 80 µL of 10 × NEBuffer (merchandise mark) 2 (available from New England BioLabs, Catalog number: B7002S) and 645 µL of deionized water. To the solution were added 8 µL of DNA Polymerase I, Large (Klenow) Fragment (available from New England BioLabs, Catalog number: M0210) and 16 µL of Deoxynucleotide (dNTP) Solution Mix (available from New England BioLabs, Catalog number: N0447), and the obtained solution was incubated at 25°C for 90 minutes.

The obtained solution was desalted with an Amicon (Registered trademark) Ultra Centrifugal filter (3 kD cutoff). To the obtained supernatant was added 17 µL of deionized water, thereafter, the solution was treated with 6 µL of 5 M aqueous sodium chloride solution and 198 µL of cooled (-20°C) ethanol, and allowed to stand at -78°C for 60 minutes. After centrifugation, the supernatant was removed and the obtained pellets were air-dried. To the pellets was added 40 µL of deionized water to make it a solution.

Of the obtained solution, a part thereof was sampled and after diluting with deionized water, mass spectrometry by ESI-MS was carried out under the conditions of Analytical condition 3 of Example 3, then the objective product of the photoreactive cross linker-modified double-stranded DEL "PXL-DS-DEL" was identified (the expected molecular weight and the observed molecular weight of the compound are shown in Table 28).

In addition, of the obtained reaction solution, a part thereof was sampled and analysis by polyacrylamide gel electrophoresis was carried out under the conditions mentioned below. From the results shown in Fig. 21, it was confirmed that PXL-DS-DEL was formed with high yield by an elongation reaction of the primer. Incidentally, the samples of each Lane of Fig. 21 are as follows.
Lane 1: 20 bp DNA Ladder (manufactured by Lonza, Lonza 20 bp DNA Ladder, Catalog number: 50330)
Lane 2: DS-BIO-DEL
Lane 3: SS-DEL
Lane 4: Sample (PXL-DS-DEL) after subjecting to primer elongation reaction of SS-DEL

Polyacrylamide gel electrophoresis:
Gel: SuperSep (merchandise mark) DNA 15% TBE gel (available from FUJIFILM Wako Pure Chemical Corporation, Catalog number: 190-15481)
Loading Buffer: 6× Loading Buffer (available from Takara Bio Inc., Catalog number: 9156)
Temperature: room temperature
Voltage: 200V
Electrophoresis time: 50 min
Dyeing reagent: SYBER (merchandise mark) GreenII Nucleic Acid Gel Stain (available from Takara Bio Inc., Catalog number: 5770A)

### INDUSTTRIAL APPLICAPABILITY

In the present invention, a nucleic acid compound containing a selectively cleavable site can be utilized. Further, in the present invention, a DNA-encoded library containing a selectively cleavable site, a composition for synthesizing the same and a method of using the same are provided, so that the production of a DNA-encoded library having higher convenience than the conventional becomes possible.

## Claims

1. A compound which is a compound represented by the formula (I) 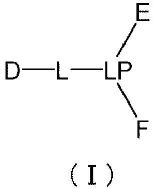 wherein
E and F are each independently
an oligomer constituted by nucleotides or nucleic acid analogues,
provided that E and F contain base sequences, which are complementary to each other and form a duplex oligonucleotide,
LP is a loop site,
L is a linker and
D is a reactive functional group and
has at least one selectively cleavable site at any of at least one site of E, F and LP.

2. The composition using for preparation of a head piece of a compound library, wherein the composition contains the compound according to Claim 1.

3. A composition using for preparation of a head piece of a DNA-encoded library, which contains the compound according to Claim 1.

4. A compound used as a head piece of a compound library, which is a compound represented by the formula (I) 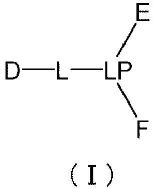 wherein
E and F are each independently
an oligomer constituted by nucleotides or nucleic acid analogues,
provided that E and F contain base sequences, which are complementary to each other and form a duplex oligonucleotide,
LP is a loop site,
L is a linker and
D is a reactive functional group and
has at least one selectively cleavable site at any of at least one site of E, F and LP.

5. A compound used as a head piece of a DNA-encoded library, which is a compound represented by the formula (I) 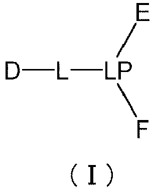 wherein
E and F are each independently
an oligomer constituted by nucleotides or nucleic acid analogues,
provided that E and F contain base sequences, which are complementary to each other and form a duplex oligonucleotide,
LP is a loop site,
L is a linker and
D is a reactive functional group and
has at least one selectively cleavable site at any of at least one site of E, F and LP.

6. A head piece of a compound library, which is a compound represented by the formula (I) 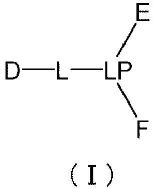 wherein
E and F are each independently
an oligomer constituted by nucleotides or nucleic acid analogues,
provided that E and F contain base sequences, which are complementary to each other and form a duplex oligonucleotide,
LP is a loop site,
L is a linker and
D is a reactive functional group and
has at least one selectively cleavable site at any of at least one site of E, F and LP.

7. A head piece of a DNA-encoded library, which is a compound represented by the formula (I) 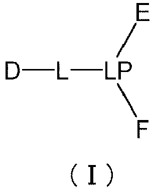 wherein
E and F are each independently
an oligomer constituted by nucleotides or nucleic acid analogues,
provided that E and F contain base sequences, which are complementary to each other and form a duplex oligonucleotide,
LP is a loop site,
L is a linker and
D is a reactive functional group and
has at least one selectively cleavable site at any of at least one site of E, F and LP.

8. A compound represented by the formula (II) 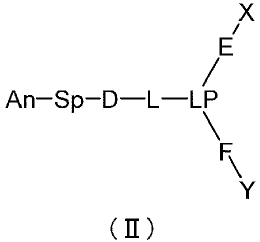 wherein
X and Y are oligonucleotide chains,
E and F are each independently
an oligomer constituted by nucleotides or nucleic acid analogues,
provided that E and F contain base sequences, which are complementary to each other and form a duplex oligonucleotide,
LP is a loop site,
L is a linker and
D is a divalent group derived from a reactive functional group,
Sp is a bonding or a bifunctional spacer and
An is a partial structure constituted by at least one building block,
X and Y have a sequence capable of forming a duplex at least a part thereof,
X binds to E at the 5' terminal end,
Y binds to F at the 3' terminal end and
has at least one selectively cleavable site at any of at least one site of E, F and LP.

9. The compound according to Claim 8, which is represented by the formula (III)
An-Sp-C-Bn (III)
wherein
An and Sp represent the same meanings as defined in Claim 8,
Bn represents the double-stranded oligonucletide tag formed by an oligonucleotide chain X and an oligonucleotide chain Y,
C is represented by the formula (I) 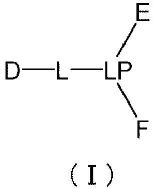
wherein E, LP, L, D and F represent the same meanings as defined in Claim 8, provided that D directly binds to An or binds via a bifunctional spacer and E and F each bind to corresponding terminal side of the double-stranded oligonucletide tag Bn.

10. The compound according to Claim 8 or 9, wherein An is the same as defined in Claim 8 and is a partial structure constructed by n building blocks α1 to αn, where n is an integer of 1 to 10,
Bn is the double-stranded oligonucletide tag formed by an oligonucleotide chain X and an oligonucleotide chain Y and is a partial structure containing an oligonucleotide which contains a base sequence capable of identifying the structure of An.

11. The compound according to any one of Claims 1, 4, 5 and 8 to 10, wherein LP is a loop site represented by (LP1)p-LS-(LP2)q,
LS is a partial structure selected from a compound group described in the following (A) to (C),
(A) a nucleotide
(B) a nucleic acid analogue
(C) a C1 to 14 trivalent group which may have a substituent(s)
LP1 is each a partial structure selected independently or differently with a number of p from a compound group described in the following (1) and (2),
(1) a nucleotide
(2) a nucleic acid analogue
LP2 is each a partial structure selected independently or differently with a number of q from a compound group described in the following (1) and (2),
(1) a nucleotide
(2) a nucleic acid analogue
and a total number of p and q is 0 to 40.

12. The compound according to Claim 11, wherein a total number of p and q is 2 to 20.

13. The compound according to Claim 11, wherein a total number of p and q is 2 to 10.

14. The compound according to Claim 11, wherein a total number of p and q is 2 to 7.

15. The compound according to Claim 11, wherein a total number of p and q is 0.

16. The compound according to any one of Claims 11 to 15, wherein LP1, LP2 and LS are each a structure independently or differently selected from the following structures:
(A) a nucleotide
or
(B) a nucleic acid analogue which requires the following (B11) to (B15)
(B11) it has phosphoric acid or a corresponding site and a hydroxyl group or its corresponding site,
(B12) it is constituted by carbon, hydrogen, oxygen, nitrogen, phosphorus or sulfur,
(B13) a molecular weight is from 142 to 1,500,
(B14) a number of atoms between residues is 3 to 30 and
(B15) a bonding mode of the atoms between the residues is either all single bonds or containing one to two double bonds and the remaining are single bonds.

17. The compound according to any one of Claims 11 to 16, wherein LP1, LP2 and LS are each a structure independently or differently selected from the following structures:
(A) a nucleotide
or
(B) a nucleic acid analogue which requires the following (B21) to (B25)
(B21) it has phosphoric acid and a hydroxyl group,
(B22) it is constituted by carbon, hydrogen, oxygen, nitrogen or phosphorus,
(B23) a molecular weight is from 142 to 1,000,
(B24) a number of atoms between residues is 3 to 15 and
(B25) a bonding mode of the atoms between the residues is all single bonds.

18. The compound according to any one of Claims 11 to 17, wherein LP1, LP2 and LS are each a structure independently or differently selected from the following structures:
(A) a nucleotide
or
(B) a nucleic acid analogue which requires the following (B31) to (B35)
(B31) it has phosphoric acid and a hydroxyl group,
(B32) it is constituted by carbon, hydrogen, oxygen, nitrogen or phosphorus,
(B33) a molecular weight is from 142 to 700,
(B34) a number of atoms between residues is 4 to 7 and
(B35) a bonding mode of the atoms between the residues is all single bonds.

19. The compound according to any one of Claims 11 to 18, wherein LP1 and LP2 are each any of the following:
(B41) d-Spacer,
(B5) a polyalkylene glycol phosphoric acid ester.

20. The compound according to any one of Claims 11 to 19, wherein LP1 and LP2 are each diethylene glycol phosphoric acid ester or triethylene glycol phosphoric acid ester.

21. The compound according to any one of Claims 11 to 20, wherein LP1 and LP2 are each triethylene glycol phosphoric acid ester.

22. The compound according to any one of Claims 11 to 19, wherein LP1 and LP2 are each d-Spacer.

23. The compound according to any one of Claims 11 to 18, wherein LP1 and LP2 are each a nucleotide.

24. The compound according to any one of Claims 11 to 23, wherein LS is any of the formula (a) to the formula (g): 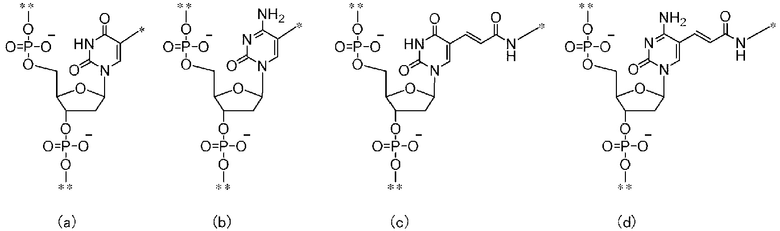 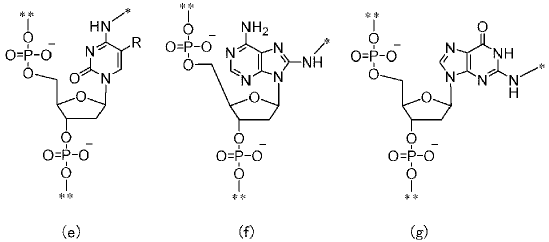 wherein * means a binding site with the linker, ** means a binding site with LP1 or LP2 and R is a hydrogen atom or a methyl group.

25. The compound according to any one of Claims 11 to 23, wherein LS is the formula (h): 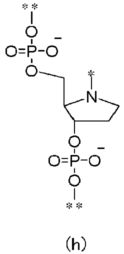 wherein * means a binding site with the linker and ** means a binding site with LP1 or LP2.

26. The compound according to any one of Claims 11 to 23, wherein LS is a polyalkylene glycol phosphoric acid ester.

27. The compound according to any one of Claims 11 to 23, wherein LS is any of the formula (i) to the formula (k): 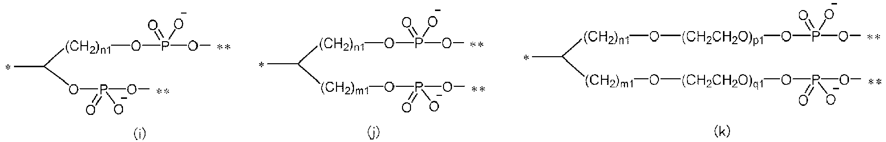 wherein n1, m1, p1 and q1 are each independently an integer of 1 to 20, * means a binding site with the linker and ** means a binding site with LP1 or LP2.

28. The compound according to any one of Claims 11 to 23, wherein LS is the formula (l): 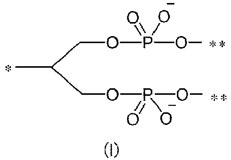 wherein * means a binding site with the linker and ** means a binding site with LP1 or LP2.

29. The compound according to any one of Claims 11 to 23, wherein LS is any of (B42), (B43) or (B44):
(B42) Amino C6 dT
(B43) mdC(TEG-Amino)
(B44) Uni-Link (trademark registration) Amino Modifier.

30. The compound according to any one of Claims 11 to 23, wherein LS is a nucleotide.

31. The compound according to any one of Claims 11 to 15 and 19 to 23, wherein LS is (C) a C1 to 14 trivalent group which may have a substituent(s) and (C) is either of the following structures:
(1) a C1 to 10 aliphatic hydrocarbon which may have a substituent(s) and may be replaced with 1 to 3 hetero atoms,
(2) a C6 to 14 aromatic hydrocarbon which may have a substituent(s),
(3) a C2 to 9 aromatic heterocyclic ring which may have a substituent(s), or
(4) a C2 to 9 non-aromatic heterocyclic ring which may have a substituent(s).

32. The compound according to any one of Claims 11 to 15 and 19 to 23, wherein LS is (C) a C1 to 14 trivalent group which may have a substituent(s) and (C) is either of the following structures:
(1) a C1 to 6 aliphatic hydrocarbon which may have a substituent(s),
(2) a C6 to 10 aromatic hydrocarbon which may have a substituent(s), or
(3) a C2 to 5 aromatic heterocyclic ring which may have a substituent(s).

33. The compound according to any one of Claims 11 to 15 and 19 to 23, wherein LS is (C) a C1 to 14 trivalent group which may have a substituent(s) and (C) is either of the following structures:
(1) a C1 to 6 aliphatic hydrocarbon,
(2) benzene, or
(3) a C2 to 5 nitrogen-containing aromatic heterocyclic ring
here, the (1) to (3) are unsubstituted, or may be substituted by 1 to 3 substituents independently or differently selected from a substituent group ST1, the substituent group ST1 is a group constituted by a C1 to 6 alkyl group, a C1 to 6 alkoxy group, a fluorine atom and a chlorine atom, provided that when the substituent group ST1 is substituted with the aliphatic hydrocarbon, an alkyl group is not selected from the substituent group ST1.

34. The compound according to any one of Claims 11 to 15 and 19 to 23, wherein LS is (C) a C1 to 14 trivalent group which may have a substituent(s) and (C) is either of the following structures:
(1) a C1 to 6 alkyl group and
(2) benzene which is unsubstituted or substituted by one or two C1 to 3 alkyl group(s) or C1 to 3 alkoxy group(s).

35. The compound according to any one of Claims 11 to 15 and 19 to 23, wherein LSis (C) a C1 to 14 trivalent group which may have a substituent(s) and (C) is the following structure:
(1) a C1 to 6 alkyl group.

36. The compound according to any one of Claims 1, 4, 5 and 8 to 35, wherein E and F are each independently an oligomer constituted by nucleotides or nucleic acid analogues and
a chain length of E and F is each 3 to 40.

37. The compound according to any one of Claims 1, 4, 5 and 8 to 36, wherein E and F are each independently an oligomer constituted by nucleotides or nucleic acid analogues,
a chain length of E and F is each 4 to 30.

38. The compound according to any one of Claims 1, 4, 5 and 8 to 37, wherein E and F are each independently an oligomer constituted by nucleotides or nucleic acid analogues,
a chain length of E and F is each 6 to 25.

39. The compound according to any one of Claims 1, 4, 5 and 8 to 38, wherein E and F are each independently an oligomer constituted by nucleotides or nucleic acid analogues,
E and F contain base sequences, which are complementary to each other and form a duplex oligonucleotide, and
the duplex oligonucleotide of E and F is a sticky end.

40. The compound according to Claim 39, wherein a protruded portion of the sticky end has a length of 2 bases or more.

41. The compound according to any one of Claims 1, 4, 5 and 8 to 38, wherein E and
F are each independently an oligomer constituted by nucleotides or nucleic acid analogues,
E and F contain base sequences, which are complementary to each other and form a duplex oligonucleotide, and
the duplex oligonucleotide of E and F is a blunt end.

42. The compound according to any one of Claims 1, 4, 5 and 8 to 41, wherein chain lengths of the base sequences, which are complementary to each other contained in E and F are each 3 bases or more.

43. The compound according to any one of Claims 1, 4, 5 and 8 to 42, wherein chain lengths of the base sequences, which are complementary to each other contained in E and F are each 4 bases or more.

44. The compound according to any one of Claims 1, 4, 5 and 8 to 43, wherein chain lengths of the base sequences, which are complementary to each other contained in E and F are each 6 bases or more.

45. The compound according to any one of Claims 1, 4, 5 and 8 to 44, wherein E and F are each independently an oligomer constituted by a nucleotide.

46. The compound according to any one of Claims 1, 4, 5 and 8 to 45, wherein the nucleotide is a ribonucleotide or a deoxyribonucleotide.

47. The compound according to any one of Claims 1, 4, 5 and 8 to 46, wherein the nucleotide is a deoxyribonucleotide.

48. The compound according to any one of Claims 1, 4, 5 and 8 to 47, wherein the nucleotide is deoxyadenosine, deoxyguanosine, thymidine or deoxycytidine.

49. The compound according to any one of Claims 1, 4, 5 and 8 to 44, wherein E and F are each independently an oligomer constituted by nucleic acid analogues.

50. The compound according to any one of Claims 1, 4, 5 and 8 to 49, wherein L is
(1) a C1 to 20 aliphatic hydrocarbon which may have a substituent(s) and may be replaced with 1 to 3 hetero atoms,
or
(2) a C6 to 14 aromatic hydrocarbon which may have a substituent(s).

51. The compound according to any one of Claims 1, 4, 5 and 8 to 50, wherein L is a C1 to 6 aliphatic hydrocarbon which may have a substituent(s), a C1 to 6 aliphatic hydrocarbon which may be replaced with one or two oxygen atoms or a C6 to 10 aromatic hydrocarbon which may have a substituent(s) .

52. The compound according to any one of Claims 1, 4, 5 and 8 to 51, wherein L is a C1 to 6 aliphatic hydrocarbon substitutable with the substituent group ST1 or benzene substitutable with the substituent group ST1, here, the substituent group ST1 is a group constituted by a C1 to 6 alkyl group, a C1 to 6 alkoxy group, a fluorine atom and a chlorine atom, provided that when the substituent group ST1 is substituted with the aliphatic hydrocarbon, an alkyl group is not selected from the substituent group ST1.

53. The compound according to any one of Claims 1, 4, 5 and 8 to 52, wherein L is a C1 to 6 alkyl group or a benzene which is unsubstituted or substituted by one or two C1 to 3 alkyl group(s) or C1 to 3 alkoxy group(s).

54. The compound according to any one of Claims 1, 4, 5 and 8 to 53, wherein L is a C1 to 6 alkyl group.

55. The compound according to any one of Claims 1, 4, 5 and 8 to 54, wherein the reactive functional group of D is a reactive functional group which can constitute a C-C, amino, ether, carbonyl, amide, ester, urea, sulfide, disulfide, sulfoxide, sulfonamide or sulfonyl bond.

56. The compound according to any one of Claims 1, 4, 5 and 8 to 55, wherein the reactive functional group of D is a C1 hydrocarbon having a leaving group, an amino group, a hydroxyl group, a precursor of a carbonyl group, a thiol group or an aldehyde group.

57. The compound according to any one of Claims 1, 4, 5 and 8 to 56, wherein the reactive functional group of D is a C1 hydrocarbon having a halogen atom(s), a C1 hydrocarbon having a sulfonic acid-based leaving group, an amino group, a hydroxyl group, a carboxyl group, a halogenated carboxyl group, a thiol group or an aldehyde group.

58. The compound according to any one of Claims 1, 4, 5 and 8 to 57, wherein the reactive functional group of D is -CH₂Cl, -CH₂Br, -CH₂OSO₂CH₃, -CH₂OSO₂CF₃, an amino group, a hydroxyl group or a carboxy group.

59. The compound according to any one of Claims 1, 4, 5 and 8 to 58, wherein the reactive functional group of D is a primary amino group.

60. The compound according to any one of Claims 1, 4, 5 and 8 to 59, wherein the selectively cleavable site is deoxyribonucleoside which is neither of deoxyadenosine, deoxyguanosine, thymidine nor deoxycytidine.

61. The compound according to any one of Claims 1, 4, 5 and 8 to 60, wherein the selectively cleavable site is deoxyuridine, bromodeoxyuridine, deoxyinosine, 8-hydroxydeoxyguanosine, 3-methyl-2'-deoxyadenosine, N6-etheno-2'-deoxyadenosine, 7-methyl-2'-deoxyguanosine, 2'-deoxyxanthosine or 5,6-dihydroxy-5,6 dihydro-deoxythymidine.

62. The compound according to any one of Claims 1, 4, 5 and 8 to 61, wherein the selectively cleavable site is deoxyuridine or deoxyinosine.

63. The compound according to any one of Claims 1, 4, 5 and 8 to 62, wherein the selectively cleavable site is deoxyuridine.

64. The compound according to any one of Claims 1, 4, 5 and 8 to 62, wherein the selectively cleavable site is deoxyinosine.

65. The compound according to any one of Claims 1, 4, 5 and 8 to 59, wherein the selectively cleavable site is a phosphodiester bond at the second in a 3' direction from deoxyinosine.

66. The compound according to any one of Claims 1, 4, 5 and 8 to 59, wherein the selectively cleavable site is ribonucleoside.

67. The compound according to any one of Claims 1, 4, 5 and 8 to 66, wherein the selectively cleavable site is 1.

68. The compound according to any one of Claims 1, 4, 5 and 8 to 66, wherein at least one cleavable site is contained in E or (LP1)p and at least one cleavable site is contained in F or (LP2)q.

69. The compound according to Claim 68, wherein the cleavable site contained in E or (LP1)p and the cleavable site contained in F or (LP2)q can be cleaved under different conditions.

70. The compound according to any one of Claims 8 to 69, wherein An is a partial structure constructed by n building blocks α1 to αn, where n is an integer of 1 to 10.

71. The compound according to any one of Claims 8 to 70, wherein An is a low molecular weight organic compound.

72. The compound according to any one of Claims 8 to 71, wherein the building block of An is a compound having a molecular weight of 500 or less.

73. The compound according to any one of Claims 8 to 72, wherein the building block of An is a compound having a molecular weight of 300 or less.

74. The compound according to any one of Claims 8 to 73, wherein the building block of An is a compound having a molecular weight of 150 or less.

75. The compound according to any one of Claims 8 to 74, wherein An is an organic compound constituted by an element selected alone or differently from the element group consisting of H, B, C, N, O, Si, P, S, F, Cl, Br and I.

76. The compound according to any one of Claims 8 to 75, wherein An is a low molecular weight organic compound having a substituent selected alone or differently from a substituent group consisting of an aryl group, a non-aromatic cyclyl group, a heteroaryl group and a non-aromatic heterocyclyl group.

77. The compound according to any one of Claims 8 to 76, wherein An has a molecular weight of 5,000 or less.

78. The compound according to any one of Claims 8 to 77, wherein An has a molecular weight of 800 or less.

79. The compound according to any one of Claims 8 to 78, wherein An has a molecular weight of 500 or less.

80. The compound according to any one of Claims 8 to 70, wherein An is a polypeptide.

81. The compound according to any one of Claims 8 to 80, wherein Sp is a bond.

82. The compound according to any one of Claims 8 to 80, wherein
Sp is a bifunctional spacer,
the bifunctional spacer is SpD-SpL-SpX,
SpD is a divalent group derived from a reactive group capable of constituting a C-C, amino, ether, carbonyl, amide, ester, urea, sulfide, disulfide, sulfoxide, sulfonamide or sulfonyl bond,
SpL is polyalkylene glycol, polyethylene, a C1 to 20 aliphatic hydrocarbon which may be optionally replaced with a hetero atom(s), peptide, oligonucleotide or a combination thereof,
SpX is a divalent group derived from a reactive group which forms an amino, carbonyl, amide, ester, urea or sulfonamide bond.

83. The compound according to any one of Claims 8 to 80, wherein
Sp is a bifunctional spacer,
the bifunctional spacer is SpD-SpL-SpX,
SpD is a divalent group derived from a primary amino group,
SpL is polyethylene glycol or polyethylene and
SpX is a divalent group derived from a carboxy group.

84. The compound according to any one of Claims 8 to 83, wherein the oligonucleotide chain X and the oligonucleotide chain Y are sequences capable of forming a duplex.

85. The compound according to any one of Claims 8 to 84, wherein the oligonucleotide chain X and the oligonucleotide chain Y contain a complementary base sequence.

86. The compound according to any one of Claims 8 to 85, wherein the oligonucleotide chain X and the oligonucleotide chain Y are each having a length of 1 to 200 bases.

87. The compound according to any one of Claims 8 to 86, wherein the oligonucleotide chain X and the oligonucleotide chain Y are each having a length of 3 to 150 bases.

88. The compound according to any one of Claims 8 to 87, wherein the oligonucleotide chain X and the oligonucleotide chain Y are each having a length of 30 to 150 bases.

89. The compound according to any one of Claims 8 to 88, wherein the oligonucleotide chain X and the oligonucleotide chain Y have a blunt end.

90. The compound according to any one of Claims 8 to 88, wherein the oligonucleotide chain X and the oligonucleotide chain Y have a sticky end.

91. The compound according to Claim 90, wherein a protruded portion of the sticky end has a length of 1 to 30 bases.

92. The compound according to Claim 90 or 91, wherein a protruded portion of the sticky end has a length of 2 to 5 bases.

93. The compound according to any one of Claims 90 to 92, wherein the oligonucleotide chain X and the oligonucleotide chain Y have a sticky end and a specific molecular recognition sequence is further bonded to the sticky end.

94. The compound according to any one of Claims 8 to 93, wherein a functional molecule is bound to any one of X and Y.

95. The compound according to any one of Claims 8 to 93, wherein biotin is bound to any one of X and Y.

96. A compound library which contains a compound(s) described in any one of Claims 1, 4, 5 and 8 to 95.

97. A DNA-encoded library which contains a compound(s) described in any one of Claims 1, 4, 5 and 8 to 95.

98. The library according to Claim 96 or 97, which is constituted by 1,000 or more different compounds.

99. A method which is a method for producing a compound An-Sp-C-Bn,
An is a partial structure constructed by n building blocks α1 to αn and n is an integer of 2 to 10,
Sp is a bond or a bifunctional spacer,
C is a hairpin type head piece having at least one "selectively cleavable site" and
Bn is a partial structure containing an oligonucleotide which contains a base sequence capable of identifying the structure of An,
which comprises subjecting to C the following steps of;
(a) binding α1-Sp, or binding Sp and α1 and
(b) binding an oligonucletide tag which contains a base sequence capable of identifying a structure of α1,
to obtain a compound A1-Sp-C-B1,
then, subjecting to A(m-1)-Sp-C-B(m-1), where m is an integer of 2 to n,
the following steps (c) and (d) by repeating until m from 2 to n in ascending order;
(c) binding αn to the A portion and
(d) binding an oligonucletide tag which contains a base sequence capable of identifying a structure of αn to the B portion
to obtain a compound Am-Sp-C-Bm,
where the steps (a) and (b) and the steps (c) and (d) can be carried out in an optional order.

100. A method which is a method for producing An-Sp-C-Bn which is a compound according to any one of Claims 9 to 95,
An is a partial structure constructed by n building blocks α1 to αn and n is an integer of 2 to 10,
Sp is a bonding or a bifunctional spacer and
C is a hairpin type head piece having at least one "selectively cleavable site" and
Bn is a partial structure containing an oligonucleotide which contains a base sequence capable of identifying the structure of An,
which comprises subjecting to C the following steps of;
(a) binding α1-Sp, or binding Sp and α1 and
(b) binding an oligonucletide tag which contains a base sequence capable of identifying a structure of α1,
to obtain a compound A1-Sp-C-B1,
then, subjecting to A(m-1)-Sp-C-B(m-1), where m is an integer of 2 to n,
the following steps (c) and (d) by repeating until m from 2 to n in ascending order;
(c) binding αn to the A portion and
(d) binding an oligonucletide tag which contains a base sequence capable of identifying a structure of αn to the B portion
to obtain a compound Am-Sp-C-Bm,
where the steps (a) and (b) and the steps (c) and (d) can be carried out in an optional order.

101. A method which is a method for producing An-Sp-C-Bn, where An, Sp, C and Bn represent the same meanings as defined above, which is a compound according to any one of Claims 9 to 95,
which comprises subjecting to C the following steps of;
(a) binding α1-Sp, or binding Sp and α1 and
(b) binding an oligonucletide tag which contains a base sequence capable of identifying a structure of α1,
to obtain a compound A1-Sp-C-B1,
then, subjecting to A(m-1)-Sp-C-B(m-1), where m is an integer of 2 to n,
the following steps (c) and (d) by repeating until m from 2 to n in ascending order;
(c) binding αn to the A portion and
(d) binding an oligonucletide tag which contains a base sequence capable of identifying a structure of αn to the B portion
to obtain a compound Am-Sp-C-Bm,
where the steps (a) and (b) and the steps (c) and (d) can be carried out in an optional order.

102. A method which is a method for evaluating a compound library containing at least one compound represented by the formula (III)
An-Sp-C-Bn (III)
wherein
An is a partial structure constructed by n building blocks α1 to αn and n is an integer of 1 to 10,
Sp is a bonding or a bifunctional spacer and
C is a hairpin type head piece having at least one "selectively cleavable site" and
Bn is a partial structure containing an oligonucleotide which contains a base sequence capable of identifying the structure of An,
which is constituted by the following steps of:
(1) by contacting the compound library with a biological target under conditions suitable for binding at least one library molecule of the compound library to the target,
(2) removing the library molecule that does not bind to the target and selecting a library molecule that have affinity to the biological target,
(3) cleaving cleavable sites selectively,
(4) identifying sequences of oligonucleotides constituting Bn and
(5) using the sequences determined in (4) to identify the structure of one or more compounds that bind to the biological target.

103. A method which is a method for evaluating a compound library containing at least one compound according to any of Claims 8 to 95 and represented by the formula (III)
An-Sp-C-Bn (III)
wherein
An is a partial structure constructed by n building blocks α1 to αn and n is an integer of 1 to 10,
Sp is a bonding or a bifunctional spacer and
C is a hairpin type head piece having at least one "selectively cleavable site" and
Bn is a partial structure containing an oligonucleotide which contains a base sequence capable of identifying the structure of An,
which is constituted by the following steps:
(1) by contacting the compound library with a biological target under conditions suitable for binding at least one library molecule of the compound library to the target,
(2) removing the library molecule that does not bind to the target and selecting a library molecule that have affinity to the biological target,
(3) cleaving cleavable sites selectively,
(4) identifying sequences of oligonucleotides constituting Bn and
(5) using the sequences determined in (4) to identify the structure of one or more compounds that bind to the biological target.

104. The method according to Claim 102 or 103, which includes a step of amplifying an oligonucleotide constituting Bn between the steps (3) and (4).

105. The method according to any one of Claims 102 to 104, wherein the step of selectively cutting cleavable site is a step of selectively cutting cleavable site by an enzyme.

106. The method according to any one of Claims 102 to 104, wherein the step of selectively cutting cleavable site is a step of selectively cutting cleavable site by a combination of an enzyme and change in chemical conditions.

107. The method according to Claim 105 or 106, wherein the enzyme is at least one selected from glycosylase and nuclease.

108. The method according to Claim 107, wherein the enzyme is uracil DNA glycosylase.

109. The method according to Claim 107, wherein the enzyme is endonuclease VIII.

110. The method according to Claim 107, wherein the enzyme is a combination of uracil DNA glycosylase and endonuclease VIII.

111. The method according to Claim 107, wherein the enzyme is alkyl adenine DNA glycosylase.

112. The method according to Claim 107, wherein the enzyme is endonuclease V.

113. The method according to any one of Claims 106 to 112, wherein the change in chemical conditions is heating at 50 to 100°C in a solution containing water.

114. The method according to any one of Claims 106 to 113, wherein the change in chemical conditions is heating at 80 to 95°Cin a solution containing water.

115. The method according to any one of Claims 106 to 114, wherein the change in chemical conditions is a basic condition of pH 8 to 13.

116. The method according to any one of Claims 106 to 115, wherein the change in chemical conditions is a basic condition of pH 8 to 11.

117. The method according to any one of Claims 106 to 116, wherein the change in chemical conditions is a basic condition of pH 9 to 10.

118. The method according to any one of Claims 102 to 117, wherein a cleavable site is provided near the terminal of the DNA tag, if necessary, the site is cleaved to form a new sticky end and a specific molecule identification sequence is ligated to the sticky terminal to identify sequences of oligonucleotides constituting Bn.

119. The method according to Claim 118, wherein the cleavable site provided near the terminal of the DNA tag and the cleavable site contained in C are cleaved under different conditions.

120. A method of utilizing as a double-stranded nucleic acid which comprises using a nucleic acid that binds to a compound having a cleavable site and a hairpin structure and cleaving a cleavable site.

121. The method according to Claim 120, wherein a nucleic acid that is chemically stable than a double-stranded nucleic acid and binds to a compound having a cleavable site and a hairpin structure is used and utilized as a double-stranded nucleic acid by cleaving the cleavable site.

122. The method according to Claim 120 or 121, wherein a nucleic acid that binds to a compound having a cleavable site and a hairpin structure is used and after subjecting to chemical structure conversion to the compound, it is utilized as a double-stranded nucleic acid by cleaving the cleavable site.

123. The method according to any one of Claims 120 to 122, wherein a nucleic acid that binds to a compound having a cleavable site and a hairpin structure is used and after further subjecting to chemical structure conversion to the nucleic acid, it is utilized as a double-stranded nucleic acid by cleaving the cleavable site.

124. The method according to any one of Claims 120 to 123, wherein a nucleic acid that binds to a compound having a cleavable site and a hairpin structure is used and after further subjecting to nucleic acid elongation reaction to the nucleic acid, it is utilized as a double-stranded nucleic acid by cleaving the cleavable site.

125. The method according to any one of Claims 120 to 124, which is made capable of utilizing as a double-stranded nucleic acid by cleaving the cleavable site using a nucleic acid that binds to a compound having a cleavable site and a hairpin structure, to carry out a PCR reaction.

126. The method according to any one of Claims 120 to 125, which is used for evaluation of functionality of a compound.

127. The method according to any one of Claims 120 to 126, which is used for evaluation of biological activity of a compound.

128. The method according to any one of Claims 120 to 127, which is used for DEL.

129. The method according to any one of Claims 120 to 124, which is used for production of DEL.

130. A method for converting into DEL having a single-stranded DNA which comprises cleaving a cleavable site to a DEL compound synthesized by using a nucleic acid that binds to a compound having a cleavable site and a hairpin structure.

131. A method for forming a double strand with a cross linker-modified DNA which comprises cleaving a cleavable site of a DEL compound synthesized by using a nucleic acid that binds to a compound having a cleavable site and a hairpin structure to convert it into DEL having a single-stranded DNA.

132. A method for synthesizing a cross linker-modified double-stranded DEL compound which comprises cleaving a cleavable site of a DEL compound synthesized by using a nucleic acid that binds to a compound having a cleavable site and a hairpin structure, adding a cross linker-modified primer and elongating the added primer.
